(19) Europäisches Patentamt
European Patent Office
Office européen des brevets

(11) **EP 4 194 855 A1**

(12) **EUROPEAN PATENT APPLICATION**

(43) Date of publication:
**14.06.2023 Bulletin 2023/24**

(21) Application number: **22209155.5**

(22) Date of filing: **09.10.2020**

(51) International Patent Classification (IPC):
*G01N 33/543* (2006.01) *C12Q 1/6806* (2018.01)

(52) Cooperative Patent Classification (CPC):
(C-Sets available)
**G01N 33/543; C12Q 1/6816;** G01N 2458/10

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2019 US 201962914296 P**

(62) Document number(s) of the earlier application(s) in accordance with Art. 76 EPC:
**20800449.9 / 4 022 309**

(71) Applicant: **10X Genomics, Inc.**
**Pleasanton, CA 94588-3260 (US)**

(72) Inventors:
• **MCDERMOTT, Geoffrey**
**Pleasanton, CA 94588-3260 (US)**
• **TAYLOR, Sarah**
**Pleasanton, CA 94588-3260 (US)**

• **BOUTET, Stephane Claude**
**Pleasanton, CA 94588-3260 (US)**
• **MCDONNELL, Wyatt James**
**Pleasanton, CA 94588-3260 (US)**
• **STUBBINGTON, Michael John Terry**
**Pleasanton, CA 94588-3260 (US)**

(74) Representative: **J A Kemp LLP**
**80 Turnmill Street**
**London EC1M 5QU (GB)**

Remarks:
•This application was filed on 23.11.2022 as a divisional application to the application mentioned under INID code 62.
•Claims filed after the date of filing of the application / after the date of receipt of the divisional application (Rule 68(4) EPC).

(54) **METHODS FOR ANALYTE DETECTION AND ANALYSIS**

(57) Provided herein are methods and systems for measuring secreted cytokines or other analytes from single cells. The methods disclosed herein include the use of analyte-specific and/or barcoded binding agents (e.g., antibodies) and beads in partitions (e.g., droplets or wells) to measure such analytes on a single cell basis. Further described herein are methods comprising the use of hydrogel-encapsulated cells (e.g., cell beads) to measure secreted and/or cellular analytes from single cells.

*FIG. 13*

**(Cont. next page)**

(52) Cooperative Patent Classification (CPC): (Cont.)

C-Sets
**C12Q 1/6816, C12Q 2563/179, C12Q 2565/514,
C12Q 2565/629**

**Description**

**CROSS-REFERENCE TO RELATED APPLICATION**

This application claims priority to U.S. Provisional Patent Application Serial No. 62/914,296, filed October 11, 2019, which application is herein incorporated by reference in its entirety for all purposes.

**BACKGROUND**

Nucleic-acid based barcoding technology can be used to measure proteins by using antibodies conjugated to a DNA sequence that contains a primer for amplification/sequencing and a unique oligonucleotide that acts as an antibody barcode. It is a useful tool for detection and quantification of proteins that remain bound to the surface of a cell. However, there is a need for approaches that allow analysis of proteins and molecules secreted by a cell.

**SUMMARY**

Provided herein are methods and compositions for analyzing a secreted analyte, a soluble analyte, and/or an extracellular analyte, e.g., from a cell of interest.

Measuring secreted proteins and molecules is essential to fully understand functional heterogeneity and decipher the underlying mechanisms of cellular interactions and communication. Secreted proteins have previously been measured with single cell resolution using assays such as the "Cytokine Secretion Assay." For example, the secreted protein of interest can be isolated using an antibody-antibody complex that coats the cell and can "catch" the molecules. The cell can then be detected by another fluorochrome-labelled antibody and subsequently analyzed using fluorescent-activated cell sorting (FACS). The FACS method is broadly similar to the enzyme-linked immunosorbent assay (ELISA) antibody format, except that the encapsulated cells remain intact. The Cytokine Secretion Assay can also be directly combined with measuring cell surface proteins for immunophenotyping and/or peptide-MHC tetramer staining for functional characterization of antigen-specific T cells. However, the same limitations associated with measuring surface proteins using FACS also apply to secreted proteins: limited ability to multiplex analytes beyond tens of proteins and inability to simultaneously measure nuclei acids from the same cell. Thus, improved methods and systems for multiplexing secreted analytes with large sample size and for simultaneous measurement of secreted analysts, mRNAs, cell surface proteins, paired $\alpha\beta$ T-cell receptor sequences, and antigen binding specificity, etc. are needed. The present disclosure provides methods and systems for such multiplexing and simultaneous measurement of various analytes of interest from a single cell.

In an aspect, the present disclosure provides methods of processing a secreted molecule from a cell, the method comprises (a) coupling the secreted molecule to a capture agent (e.g., a first polypeptide) coupled to a surface of the cell to form a first conjugate; and (b) coupling a reporter agent (e.g., a second polypeptide) to the secreted molecule to form a second conjugate, wherein the reporter agent comprises a nucleic acid reporter molecule comprising a first barcode sequence (e.g., a reporter sequence comprising a reporter barcode sequence).

In some embodiments, prior to (a), the method further comprises incubating the cell with the capture agent under a sufficient condition such that the capture agent couples to the surface of the cell. In some embodiments, prior to (b), the method further comprises stimulating the cell to induce secretion of the secreted molecule.

In some embodiments, the cell is stimulated with an antigen. In some embodiments, the antigen is a Pattern recognition receptor (PRR) ligand. The PRR ligands may be based on or derived from a Pathogen Associated Molecular Pattern (PAMP) (also known as a Microbe Associated Molecular Pattern (MAMP) and can comprise one or more elements of a PAMP-based macromolecule (e.g., nucleic acid or amino acid). In some embodiments, the PRR ligand is a Toll-like receptors (TLR) ligand, a NOD-like receptor (NLRs) ligand, a RIG-I-like receptor (RLR) ligand, Absent-in-melanoma (AIM)-like receptor (ALR) ligand, a C-type lectin receptor (CLR) ligand, or a cytosolic dsDNA sensor (CDS) ligand. In some embodiments, the antigen is, comprises, or is derived from a viral protein (e.g., F protein from respiratory syncytial virus (RSV), or a glycoprotein from vesicular stomatitis virus (VSV)), a lipopolypeptide or lipopeptide (e.g., a triacyl lipopeptide, a diacyl lipopolypeptide), a peptidoglycan, phosphatidylserine, zymosan, flagellin, hemozoin, a lipopolysaccharide (LPS), a double-stranded DNA (dsDNA), a double-stranded RNA (dsRNA), a synthetic dsRNA, or CpG oligodeoxynucleotides (CpG ODN). In some embodiments, the synthetic dsRNA is polyinosinic-polycytidylic acid (poly I:C) or polyadenylic-polyuridylic acid (poly(A:U)).

In some embodiments, the method further comprises providing a plurality of antigens to the cell at a concentration that is sufficient to induce secretion of the secreted molecule. In some embodiments, the plurality of antigens is provided to the cell for a length of time that is sufficient to induce the secretion of the secreted molecule. In some embodiments, an antigen of the plurality of antigens is coupled to a major histocompatibility complex (MHC) molecule. In some embodiments, the MHC molecule is an MHC multimer. In some embodiments, the MHC multimer comprises a dextran polymer. In some embodiments, the MHC molecule is present in an antigen presenting cell.

In some embodiments, the method further comprises contacting the cell with one or more co-stimulatory molecules. In some embodiments, the one or more co-stimulatory molecules comprises one or more antibodies. In some embodiments, the one or more antibodies are anti-CD3 or anti-CD28 antibodies. In some embodiments, the one or more co-stimulatory molecules comprises one or more cytokines.

In some embodiments, the one or more cytokines comprises an interleukin. In some embodiments, the one or more co-stimulatory molecules are present on an antigen presenting cell. In some embodiments, the method further comprises co-partitioning a plurality of nucleic acid barcode molecules and the cell (e.g., a labelled cell) comprising the first and the second conjugates (e.g., a complex coupled to a surface of the cell and comprising (i) a capture agent, (ii) a secreted analyte, and (iii) a reporter agent) into a partition, wherein each nucleic acid molecule of the plurality of nucleic acid molecules comprises a second barcode sequence, and wherein the second barcode sequence is different from the first barcode sequence. In some embodiments, the plurality of nucleic acid barcode molecules is attached to a support (e.g., a bead). In some embodiments, the plurality of nucleic acid barcode molecules is releasably attached to said support (e.g., bead). In some embodiments, the plurality of nucleic acid barcode molecules is releasably attached to the support (e.g., bead) via a labile bond. In some embodiments, the plurality of nucleic acid barcode molecules is releasable from the support (e.g., bead) upon application of a stimulus. In some embodiments, the stimulus is a thermal stimulus, chemical stimulus, biological stimulus, or a photo-stimulus. In some embodiments, the chemical stimulus is a reducing agent. In some embodiments, the labile bond is a disulfide bond.

In some embodiments, the method further comprises releasing nucleic acid barcode molecules of the plurality of nucleic acid barcode molecules from the support, such as a bead. In some embodiments, the bead is a gel bead. In some embodiments, the bead is a degradable gel bead. In some embodiments, the gel bead is degradable upon application of a stimulus. In some embodiments, the stimulus is a thermal stimulus, chemical stimulus, biological stimulus, or a photo-stimulus. In some embodiments, the chemical stimulus is a reducing agent. In some embodiments, the partition is a droplet or a well.

In some embodiments, the method further comprises lysing the cell. In some embodiments, the method further comprises releasing nucleic acids from a cell. In one embodiment the nucleic acids comprise a messenger ribonucleic acid (mRNA) molecule from the cell. In some embodiments, the method further comprises using a nucleic acid barcode molecule of the plurality of nucleic acid barcode molecules and the reporter molecule to generate a first barcoded nucleic acid molecule comprising a sequence corresponding to the first barcode sequence and a sequence corresponding to the second barcode sequence. In some embodiments, the nucleic acid barcode molecule comprises a sequence complementary to a sequence in the reporter molecule.

In some embodiments, the method further comprises hybridizing the nucleic acid barcode molecule to the reporter molecule and performing a nucleic acid reaction to generate the first barcoded nucleic acid molecule. In some embodiments, the nucleic acid reaction is a ligation reaction or a nucleic acid extension reaction. In some embodiments, the method further comprises using nucleic acid molecules from the cell (e.g., the mRNA molecule(s)) and a nucleic acid barcode molecule of the plurality of nucleic acid barcode molecules to generate a second barcoded nucleic acid molecule comprising a sequence corresponding to the mRNA molecule and a sequence corresponding to the second barcode sequence.

In some embodiments, the nucleic acid barcode molecule comprises a sequence complementary to a sequence in the mRNA molecule. In some embodiments, the method further comprises hybridizing the nucleic acid barcode molecule to the mRNA molecule or a cDNA thereof and performing a nucleic acid reaction to generate the barcoded nucleic acid molecule. In some embodiments, the nucleic acid reaction is a reverse transcription reaction, a ligation reaction, or a nucleic acid extension reaction. In some embodiments, the method further comprises amplifying the reporter molecule and/or a cDNA molecule derived from the mRNA molecule.

In some embodiments, the amplification comprises a polymerase chain reaction (PCR). In some embodiments, wherein the amplification is isothermal. In some embodiments, the method further comprises sorting the nucleic acid molecule and/or cDNA molecule according to their sizes. In some embodiments, the method further comprises sequencing the first barcoded nucleic acid molecule or a derivative thereof and/or the second barcoded nucleic acid molecule or a derivative thereof.

In some embodiments, the method further comprises performing one or more nucleic acid reactions to add one or more functional sequences to the first barcoded nucleic acid molecule and/or the second barcoded nucleic acid molecule. In some embodiments, the one or more functional sequences are a primer sequence, a sequencing primer sequence, or a sequence configured to attach to a flow cell of a sequencer. In some embodiments, the reporter molecule comprises one or more functional sequences selected from the group consisting of a primer sequence, a sequencing primer sequence, a sequence configured to attach to a flow cell of a sequencer, and a unique molecular index (UMI). In some embodiments, the plurality of nucleic acid barcode molecules each comprise one or more functional sequences selected from the group consisting of a primer sequence, a sequencing primer sequence, a partial sequencing primer sequence, a sequence configured to attach to a flow cell of a sequencer, and a unique molecular index (UMI).

In some embodiments, the method further comprises identifying the first barcode sequence and the second barcode sequence and associating the secreted molecule and/or the mRNA molecule with the cell. In some embodiments, the secreted molecule is a cytokine. In some embodiments, the capture agent (e.g., the first polypeptide) is coupled to the cell via a cell surface protein. In some embodiments, the capture agent (e.g., the first polypeptide) comprises a first antibody or antibody fragment and a second antibody of antibody fragment, wherein the first antibody or antibody fragment and a second antibody of antibody fragment are associated. In some embodiments, the first antibody or antibody fragment is capable of coupling to the secreted molecule. In some embodiments, the second antibody of antibody fragment is capable of coupling to the surface of the cell. In some embodiments, the reporter agent (e.g., the second polypeptide) comprises or is an antibody or antibody fragment.

In some embodiments, the method further comprises contacting the cell with a plurality of capture agent molecules and, prior to (b), removing capture agent molecules not bound to the surface of the cell. In some embodiments, the method further comprises contacting the cell with a plurality of reporter agent molecules (e.g., second polypeptide molecules) and, subsequent to (b), removing reporter agent molecules not bound to the secreted molecule.

In another aspect, the present disclose provides methods of processing a molecule from a cell, the methods comprise (a) generating a cell bead, wherein the cell bead comprises a cell encapsulated by a polymer matrix, wherein the polymer matrix comprises a plurality of capture agents; and (b) coupling a molecule secreted from the cell to a first capture agent of the plurality of capture agents to form a first conjugate.

In some embodiments, the polymer matrix is a hydrogel matrix. In some embodiments, the polymer matrix comprises collagen, laminin, and/or fibronectin. In some embodiments, the plurality of the capture agents (e.g., first polypeptides) are coupled to a polymer backbone of the polymer matrix. In some embodiments, prior to (b), the method further comprises stimulating the cell bead containing the cell to induce secretion of the molecule from the cell. In some embodiments, the cell bead is stimulated with an antigen. In some embodiments, the antigen is a Pattern recognition receptor (PRR) ligand. In some embodiments, the PRR ligand is a Toll-like receptors (TLR) ligand, a NOD-like receptor (NLRs) ligand, a RIG-I-like receptor (RLR) ligand, a C-type lectin receptor (CLR) ligand, or a cytosolic dsDNA sensor (CDS) ligand. In some embodiments, the antigen is a lipopolysaccharide (LPS), a double-stranded DNA (dsDNA), a double-stranded RNA (dsRNA), a synthetic dsRNA, or CpG oligodeoxynucleotides (CpG ODN). In some embodiments, the synthetic dsRNA is polyinosinic-polycytidylic acid (poly I:C) or polyadenylic-polyuridylic acid (poly(A:U).

In some embodiments, the method further comprises providing a plurality of antigens to the cell bead at a concentration that is sufficient to induce secretion of the secreted molecule. In some embodiments, the plurality of antigens is provided to the cell bead for a length of time that is sufficient to induce the secretion of the secreted molecule. In some embodiments, an antigen of the plurality of antigens is coupled to a major histocompatibility complex (MHC) molecule. In some embodiments, the MHC molecule is an MHC multimer. In some embodiments, the MHC multimer comprises a dextran polymer. In some embodiments, the MHC molecule is present in an antigen presenting cell.

In some embodiments, the method further comprises contacting the cell bead with one or more co-stimulatory molecules. In some embodiments, the one or more co-stimulatory molecules comprises one or more antibodies. In some embodiments, the one or more antibodies are anti-CD3 or anti-CD28 antibodies. In some embodiments, the one or more co-stimulatory molecules comprises one or more cytokines. In some embodiments, the one or more cytokines comprises an interleukin. In some embodiments, the one or more co-stimulatory molecules are present on an antigen presenting cell.

In some embodiments, the method further comprises coupling a reporter agent (e.g., second polypeptide) to the molecule secreted from the cell to form a second conjugate, wherein the reporter agent comprises a nucleic acid reporter molecule comprising a first barcode sequence (e.g., reporter barcode sequence). In some embodiments, the method further comprises removing unbound reporter agent molecules from the cell bead.

In some embodiments, subsequent to (b), the method further comprises (c) partially digesting the cell bead with an enzyme, thereby generating a partially digested cell bead. In some embodiments, the enzyme is collagenase or dispase. In some embodiments, the method further comprises contacting a plurality of reporter agents comprising a barcode to the partially digested cell bead, under a condition so that a reporter agent from the plurality of reporter agents couples to the molecule secreted from the cell. In some embodiments, the method further comprises co-partitioning a plurality of nucleic acid barcode molecules and the cell bead comprising the first and the second conjugate into a partition, wherein each nucleic acid barcode molecule of the plurality of nucleic acid barcode molecules comprises a second barcode sequence, and wherein the second barcode sequence is different from the first barcode sequence.

In some embodiments, the plurality of nucleic acid barcode molecules is attached to a bead. In some embodiments, the plurality of nucleic acid barcode molecules is releasably attached to said bead. In some embodiments, the plurality of nucleic acid barcode molecules is releasably attached to the bead via a labile bond. In some embodiments, the plurality of nucleic acid barcode molecules is releasable from the bead upon application of a stimulus. In some embodiments, the stimulus is a thermal stimulus, chemical stimulus, biological stimulus, or a photo-stimulus. In some embodiments, the chemical stimulus is a reducing agent. In some embodiments, the labile bond is a disulfide bond.

In some embodiments, the method further comprises releasing nucleic acid barcode molecules of the plurality of nucleic acid barcode molecules. In some embodiments, wherein the bead is a gel bead. In some embodiments, the bead is a degradable gel bead. In some embodiments, the gel bead is degradable upon application of a stimulus. In some embodiments, the stimulus is a thermal stimulus, chemical stimulus, biological stimulus, or a photo-stimulus. In some embodiments, the chemical stimulus is a reducing agent. In some embodiments, the partition is a droplet or a well. In some embodiments, the method further comprises degrading the cell bead.

In some embodiments, the method further comprises lysing the cell. In some embodiments, the method further comprises releasing a messenger ribonucleic acid (mRNA) molecule from the cell. In some embodiments, the method further comprises using a nucleic acid barcode molecule of the plurality of nucleic acid barcode molecules and the reporter molecule to generate a first barcoded nucleic acid molecule comprising a sequence corresponding to the first barcode sequence and a sequence corresponding to the second barcode sequence.

In some embodiments, the nucleic acid barcode molecule comprises a sequence complementary to a sequence in the reporter molecule. In some embodiments, the method further comprises hybridizing the nucleic acid barcode molecule to the reporter molecule and performing a nucleic acid reaction to generate the first barcoded nucleic acid molecule. In some embodiments, the method further comprises the nucleic acid reaction is a ligation reaction or a nucleic acid extension reaction. In some embodiments, the method further comprises using the mRNA molecule and a nucleic acid barcode molecule of the plurality of nucleic acid barcode molecules to generate a second barcoded nucleic acid molecule comprising a sequence corresponding to the mRNA molecule and a sequence corresponding to the second barcode sequence.

In some embodiments, the nucleic acid barcode molecule comprises a sequence complementary to a sequence in the mRNA molecule. In some embodiments, the method further comprises hybridizing the nucleic acid barcode molecule to the mRNA molecule or a cDNA thereof and performing a nucleic acid reaction to generate the barcoded nucleic acid molecule. In some embodiments, the nucleic acid reaction is a reverse transcription reaction, a ligation reaction, or a nucleic acid extension reaction. In some embodiments, the method further comprises amplifying the reporter molecule and/or a cDNA molecule derived from the mRNA molecule.

In some embodiments, the amplification comprises a polymerase chain reaction (PCR). In some embodiments, the amplification is isothermal. In some embodiments, the method further comprises sorting the nucleic acid molecule and/or cDNA molecule according to their sizes. In some embodiments, the method further comprises sequencing the first barcoded nucleic acid molecule or a derivative thereof and/or the second barcoded nucleic acid molecule or a derivative thereof. In some embodiments, the method further comprises performing one or more nucleic acid reactions to add one or more functional sequences to the first barcoded nucleic acid molecule and/or the second barcoded nucleic acid molecule.

In some embodiments, the one or more functional sequences are a primer sequence, a sequencing primer sequence, or a sequence configured to attach to a flow cell of a sequencer. In some embodiments, the reporter molecule comprises one or more functional sequences selected from the group consisting of a primer sequence, a sequencing primer sequence, a sequence configured to attach to a flow cell of a sequencer, and a unique molecular index (UMI). In some embodiments, the plurality of nucleic acid barcode molecules each comprise one or more functional sequences selected from the group consisting of a primer sequence, a sequencing primer sequence, a partial sequencing primer sequence, a sequence configured to attach to a flow cell of a sequencer, and a unique molecular index (UMI).

In some embodiments, the method further comprises identifying the first barcode sequence and the second barcode sequence and associating the secreted molecule and/or the mRNA molecule with the cell. In some embodiments, the secreted molecule is a cytokine. In some embodiments, the second polypeptide is an antibody or antibody fragment. In some embodiments, the MHC molecule comprises a nucleic acid molecule comprising a third barcode sequence. In some embodiments, the third barcode sequence is different from the first barcode sequence or the second barcode sequence.

In certain embodiments, methods of processing a secreted molecule from a cell comprise (a) coupling the secreted molecule to a capture agent (e.g., a first polypeptide or a first binding agent comprising a first polypeptide) coupled to a surface of the cell to form a first conjugate; and (b) coupling a reporter agent (e.g., a second polypeptide or a second binding agent comprising a second polypeptide) to the secreted molecule to form a second conjugate, wherein the reporter agent comprises a nucleic acid molecule comprising a first barcode sequence. Further, the methods comprise prior to (a), incubating the cell with the capture agent such that the capture agent couples to the surface of the cell. Moreover, the methods comprise prior to (a), providing a plurality of antigens to the cell at a concentration that is sufficient to induce secretion of the secreted molecule. In addition, the methods comprise co-partitioning a support, such as a bead (e.g., a gel bead such as an emulsion bead), and the cell coupled to the second conjugate into a partition, wherein the bead is coupled to a plurality of nucleic acid molecules comprising a second barcode sequence, wherein the second barcode sequence is different from the first barcode sequence. The methods also comprise lysing the cells to release intracellular content such as cellular mRNA molecules, reverse transcribing the mRNA molecules thereby generating corresponding cDNA molecules that comprise the second barcode sequence that the beads (e.g., emulsion beads) may be comprised of, and attaching the second barcode sequence (e.g., partition-specific barcode sequence) to the nucleic acid molecules comprising the first, analyte-specific barcode sequence. The nucleic acid molecules (e.g., cDNAs, barcode sequences, etc.) may be amplified, and sorted by size, followed by sequencing the sorted nucleic acid molecules. Sequencing may identify secreted analytes (e.g., cytokines), mRNA molecules, and other cell-specific analytes and enable association of these cellular analytes with their corresponding cell or cell type (e.g., an immune cell such as a T cell) for, e.g., immune phenotyping etc. The present disclosure provides methods that do not only allow to obtain this information on a single cell basis, but also allows the analysis of secreted analytes (e.g., cytokines or other secreted proteins, see e.g., operation **1803**) in addition and/or simultaneous to the analysis of cellular nucleic acid molecules (e.g., mRNAs for analyzing T cell receptor (TCR) sequences, see e.g., operations **1804** and **1805**), cell surface proteins (e.g., T cell receptors, B cell receptors, etc., see e.g., operation **1801**), antigen specificity (see e.g., operation **1802**) of a single cell, etc. Exemplary methods and compositions for single cell analysis of analytes are disclosed in WO2019/157529, incorporated herein by reference in its entirety for all purposes. In addition, the methods allow for multiplexing or otherwise increasing throughput of samples for analysis through the use of labelling agents, which comprise nucleic acid reporter molecules and are capable of binding to or otherwise coupling to one or more cells or cell features, as further described herein.

Further, in certain embodiments, the methods of processing a secreted molecule from a cell comprise (a) generating a cell bead, wherein the cell bead comprises a cell encapsulated by a polymer matrix (e.g., a hydrogel matrix), wherein the polymer matrix is coupled to a capture agent (e.g., a first polypeptide or a first binding agent comprising a first polypeptide), wherein the capture agent is specific for the secreted molecule; and (b) coupling the secreted molecule to the capture agent to form a first conjugate. The methods further comprise, prior to (b), providing a plurality of antigens to the cell bead at a concentration that is sufficient to induce secretion of the secreted molecule. Subsequent to forming the first conjugate, a reporter agent (e.g., a second polypeptide or a second binding agent comprising a second polypeptide) may be coupled to the secreted molecule bound to the capture agent (e.g., the first polypeptide) to form a second conjugate, wherein the reporter agent comprises a nucleic acid molecule comprising a first barcode sequence (e.g., reporter barcode sequence), thereby barcoding the secreted molecule. In addition, the methods further comprise co-partitioning the cell bead coupled to the second conjugate into a partition, wherein the partition further comprises a bead (e.g., a gel bead such as an emulsion bead), and wherein the bead comprises plurality of nucleic acid molecules comprising a second barcode sequence, wherein second the barcode sequence is different from the first barcode sequence. The polymer matrix is dissolved and the cells subsequently lysed to release intracellular content such as cellular mRNA molecules. The mRNA molecules are reverse transcribed thereby generating corresponding cDNA molecules that comprise the second barcode sequence (e.g., partition-specific barcode sequence). The nucleic acid molecules comprising the first, analyte-specific barcode sequence are also barcoded with the second barcode sequence. The nucleic acids (e.g., cDNAs, barcode sequences, etc.) may be amplified, and sorted by size, followed by sequencing the sorted nucleic acid molecules. Sequencing may identify secreted analytes (e.g., cytokines), mRNA molecules, and other cell-specific analytes and enable association of these cellular analytes with their corresponding cell or cell type (e.g., an immune cell such as a T cell) for, e.g., immune phenotyping etc. The present disclosure provides methods that do not only allow to obtain this information on a single cell basis, but also allows the analysis of secreted analytes (e.g., cytokines or other secreted proteins, see e.g., operation **1803**) in addition and/or simultaneous to the analysis of cellular nucleic acid molecules (e.g., mRNAs for analyzing T cell receptor (TCR) sequences, see e.g., operations **1804** and **1805**), cell surface proteins (e.g., T cell receptors, B cell receptors, etc., see e.g., operation **1801**), antigen specificity (see e.g., operation **1802**) of a single cell, etc. In addition, the methods allow for multiplexing or otherwise increasing throughput of samples for analysis through the use of labelling agents, which comprise nucleic acid reporter molecules and are capable of binding to or otherwise coupling to one or more cells or cell features, as further described herein.

In some embodiments, provided herein is a method of single cell analysis, comprising: a) contacting a cell with a reporter agent comprising a reporter nucleic acid molecule to provide a labelled cell, wherein the reporter nucleic acid molecule comprises a reporter sequence corresponding to the reporter agent, wherein the labelled cell comprises a complex coupled to a surface of the cell, and wherein the complex comprises (i) a capture agent, (ii) a secreted analyte, and (iii) the reporter agent; b) partitioning the labelled cell in a partition, wherein the partition comprises a plurality of barcode nucleic acid molecules which comprise a plurality of partition barcode sequences; and c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

In any of the preceding embodiments, the secreted analyte can comprise a secreted protein. In any of the preceding embodiments, the capture agent can be configured to couple to a cell surface molecule. In any of the preceding embodiments, said cell surface molecule can comprise a cell surface protein. In any of the preceding embodiments, the capture agent can be configured to couple to the secreted analyte. In any of the preceding embodiments, said capture agent can be configured to couple to both the cell surface protein and said secreted analyte. In any of the preceding embodiments, said capture agent can be a first protein binding agent. In any of the preceding embodiments, the reporter agent can be configured to couple to the secreted analyte. In any of the preceding embodiments, said reporter agent can be a second protein binding agent. In any of the preceding embodiments, said reporter nucleic acid molecule can further comprise a sequence configured to couple to the barcode nucleic acid molecule.

In any of the preceding embodiments, said labelled cell can further comprise a second reporter agent comprising a second reporter nucleic acid molecule. In any of the preceding embodiments, said second reporter agent can be configured to couple to a second cell surface protein of the cell. In any of the preceding embodiments, said second reporter nucleic acid molecule can comprise a second reporter sequence corresponding to the second reporter agent. In any of the preceding embodiments, the partition can further comprise a second barcode nucleic acid molecule of a plurality of second barcode nucleic acid molecules. In any of the preceding embodiments, said second reporter nucleic acid molecule can comprise a second sequence configured to couple to the second barcode nucleic acid molecule. In any of the preceding embodiments, the method can further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

In any of the preceding embodiments, said labelled cell can further comprise a plurality of nucleic acid analytes, and a nucleic acid analyte of said plurality of nucleic acid analytes can comprise a nucleic acid analyte sequence. In any of the preceding embodiments, the partition can further comprise a plurality of third barcode nucleic acid molecules. In any of the preceding embodiments, a third barcode nucleic acid molecule of said plurality of third barcode nucleic acid molecules can comprise a third sequence configured to couple to the nucleic acid analyte sequence. In any of the preceding embodiments, the method can further comprise generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

In any of the preceding embodiments, said partition can comprise a support that comprises the plurality of barcode nucleic acid molecules. In any of the preceding embodiments, said support can comprise a bead. In any of the preceding embodiments, said bead can comprise a gel bead. In any of the preceding embodiments, said plurality of barcode nucleic acid molecules can be releasably attached to the support. In any of the preceding embodiments, the partition can be from a plurality of partitions. In any of the preceding embodiments, the partition can comprise a droplet or a microwell.

In some embodiments, provided herein is a method of single cell analysis, comprising: a) contacting a cell bead with a reporter agent comprising a reporter nucleic acid molecule to provide a labelled cell bead, wherein the cell bead comprises a cell in a matrix, wherein the reporter nucleic acid molecule comprises a reporter sequence corresponding to the reporter agent, wherein the labelled cell bead comprises a complex in or on the cell bead, and wherein the complex comprises (i) a capture agent, (ii) a secreted analyte, and (iii) the reporter agent; b) partitioning the labelled cell bead in a partition, wherein the partition comprises a plurality of barcode nucleic acid molecules which comprise a plurality of partition barcode sequences; and c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

In any of the preceding embodiments, said secreted analyte can comprise a secreted protein. In any of the preceding embodiments, the capture agent can be configured to couple to the matrix of the cell bead. In any of the preceding embodiments, said matrix can be a degradable polymer matrix. In any of the preceding embodiments, the capture agent can be configured to couple to the secreted analyte. In any of the preceding embodiments, said capture agent can be configured to couple to both the matrix and said secreted analyte. In any of the preceding embodiments, said capture agent can be a first protein binding agent. In any of the preceding embodiments, the reporter agent can be configured to couple to the secreted analyte. In any of the preceding embodiments, said reporter agent can be a second protein binding agent. In any of the preceding embodiments, said reporter nucleic acid molecule can further comprise a sequence configured to couple to the barcode nucleic acid molecule.

In any of the preceding embodiments, said cell or said labelled cell bead can further comprise a second reporter agent comprising a second reporter nucleic acid molecule. In any of the preceding embodiments, said second reporter agent can be configured to couple to a cell surface protein of the cell. In any of the preceding embodiments, said second reporter nucleic acid molecule can comprise a second reporter sequence corresponding to the second reporter agent. In any of the preceding embodiments, the partition can further comprise a second barcode nucleic acid molecule of a plurality of second barcode nucleic acid molecules. In any of the preceding embodiments, said second reporter nucleic acid molecule can comprise a second sequence configured to couple to the second barcode nucleic acid molecule. In any of the preceding embodiments, the method can further comprise generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

In any of the preceding embodiments, said cell can further comprise a plurality of nucleic acid analytes, wherein a nucleic acid analyte of said plurality of nucleic acid analytes can comprise a nucleic acid analyte sequence. In any of the preceding embodiments, the partition can further comprise a plurality of third barcode nucleic acid molecules. In any of the preceding embodiments, a third barcode nucleic acid molecule of said plurality of third barcode nucleic acid molecules can comprise a third sequence configured to couple to the nucleic acid analyte sequence. In any of the preceding embodiments, the method can further comprise generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

In any of the preceding embodiments, said partition can comprise a support that comprises the plurality of barcode nucleic acid molecules. In any of the preceding embodiments, said support can comprise a bead. In any of the preceding embodiments, said bead comprises a gel bead. In any of the preceding embodiments, said plurality of barcode nucleic acid molecules can be releasably attached to the support. In any of the preceding embodiments, the partition can be from a plurality of partitions. In any of the preceding embodiments, the partition can comprise a droplet or a microwell.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 shows an example of a microfluidic channel structure for partitioning individual biological particles.

FIG. 2 shows an example of a microfluidic channel structure for delivering barcode carrying beads to droplets.

FIG. 3 shows an example of a microfluidic channel structure for co-partitioning biological particles and reagents.

FIG. 4 shows an example of a microfluidic channel structure for the controlled partitioning of beads into discrete droplets.

FIG. 5 shows an example of a microfluidic channel structure for increased droplet generation throughput.

FIG. 6 shows another example of a microfluidic channel structure for increased droplet generation throughput.

FIG. 7A shows a cross-section view of another example of a microfluidic channel structure with a geometric feature for controlled partitioning.

FIG. 7B shows a perspective view of the channel structure of FIG. 7A.

FIG. 8 illustrates an example of a barcode carrying bead.

FIG. 9 illustrates another example of a barcode carrying bead.

FIG. 10 schematically illustrates an exemplary microwell array.

FIG. 11 schematically illustrates an exemplary workflow for processing nucleic acid molecules.

FIG. 12A shows an example of culturing cells to allow or induce secretion of endogenous molecules.

FIG. 12B shows an example of a first binding agent (e.g., capture agent) coupling to a cell surface and a secreted analyte.

FIG. 13 shows an example of a cell coupling to a first binding agent (e.g., capture agent) comprising polypeptides (e.g., antibodies); and each capture agent coupling to secreted analytes (e.g., cytokines), which are then coupled to a second binding agent (e.g., reporter agent) comprising a polypeptide (e.g., an antibody) and a nucleic acid molecule comprising a first barcode sequence (e.g., reporter barcode sequence).

FIGS. 14A-14C show examples of methods disclosed herein.

FIG. 15A shows an example of forming a cell bead.

FIG. 15B shows an example of culturing cells within cell beads to allow or induce secretion of endogenous molecules.

FIG. 15C shows an example of a first binding agent (e.g., capture agent) comprising an analyte specific antibody which is bound to or coupled to a polymer backbone.

FIG. 15D shows an example of a hydrogel matrix (e.g., a cell bead) coupled to a binding agent (e.g., reporter agent) comprising a polypeptide (e.g., an antibody) and a nucleic acid molecule comprising a first barcode sequence (e.g., reporter barcode sequence), where the binding agent is coupled to secreted analytes (e.g., cytokines).

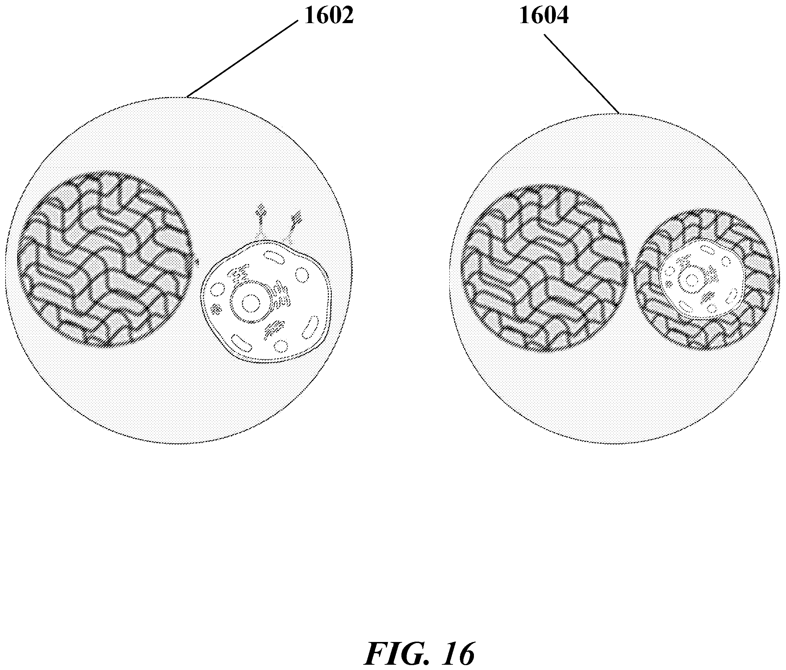

FIG. 16 shows an example of a partitioned cell 1602 and partitioned cell bead 1604.

FIG. 17A shows an example of a barcoded bead that may be used in a partition such as a droplet to couple to a barcode (e.g., a partition-specific barcode) one or more analytes (e.g., secreted analytes such as cytokines, mRNAs, etc) of a single cell, thereby associating said one or more analytes with the single cell.

FIG. 17B shows an illustration of the conversion of barcoded analytes into sequencing libraries.

FIG. 18 shows an example of simultaneous measurement of secreted analysts, mRNAs, cell surface proteins, paired αβ T-cell receptor sequences, and antigen binding specificity.

FIG. 19 schematically illustrates examples of labelling agents.

FIG. 20 depicts an example of a barcode carrying bead.

FIGS. 21A-21C schematically depict an example workflow for processing nucleic acid molecules.

FIG. 22 shows a computer system that is programmed or otherwise configured to implement methods provided herein.

## DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The term "real time," as used herein, can refer to a response time of less than about 1 second, a tenth of a second, a hundredth of a second, a millisecond, or less. The response time may be greater than 1 second. In some instances, real time can refer to simultaneous or substantially simultaneous processing, detection or identification.

The term "subject," as used herein, generally refers to an animal, such as a mammal (e.g., human) or avian (e.g., bird), or other organism, such as a plant. For example, the subject can be a vertebrate, a mammal, a rodent (e.g., a mouse), a primate, a simian or a human. Animals may include, but are not limited to, farm animals, sport animals, and pets. A subject can be a healthy or asymptomatic individual, an individual that has or is suspected of having a disease (e.g., cancer) or a pre-disposition to the disease, and/or an individual that is in need of therapy or suspected of needing therapy. A subject can be a patient. A subject can be a microorganism or microbe (e.g., bacteria, fungi, archaea, viruses).

The term "genome," as used herein, generally refers to genomic information from a subject, which may be, for example, at least a portion or an entirety of a subject's hereditary information. A genome can be encoded either in DNA or in RNA. A genome can comprise coding regions (e.g., that code for proteins) as well as non-coding regions. A genome can include the sequence of all chromosomes together in an organism. For example, the human genome ordinarily has a total of 46 chromosomes. The sequence of all of these together may constitute a human genome.

The terms "adaptor(s)", "adapter(s)" and "tag(s)" may be used synonymously. An adaptor or tag can be coupled to a polynucleotide sequence to be "tagged" by any approach, including ligation, hybridization, or other approaches.

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina®, Pacific Biosciences (PacBio®), Oxford Nanopore®, or Life Technologies (Ion Torrent®). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

As used herein, the term "barcoded nucleic acid molecule" generally refers to a nucleic acid molecule that results from, for example, the processing of a nucleic acid barcode molecule with a nucleic acid sequence (e.g., nucleic acid sequence complementary to a nucleic acid primer sequence encompassed by the nucleic acid barcode molecule). The nucleic acid sequence may be a targeted sequence or a non-targeted sequence. The nucleic acid barcode molecule may be coupled to or attached to the nucleic acid molecule comprising the nucleic acid sequence. For example, in the methods and systems described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). The processing of the nucleic acid molecule comprising the nucleic acid sequence, the nucleic acid barcode molecule, or both, can include a nucleic acid reaction, such as, in non-limiting examples, reverse transcription, nucleic acid extension, ligation, etc. The nucleic acid reaction may be performed prior to, during, or following barcoding of the nucleic acid sequence to generate the barcoded nucleic acid molecule. For example, the nucleic acid molecule comprising the nucleic acid sequence may be subjected to reverse transcription and then be attached to the nucleic acid barcode molecule to generate the barcoded nucleic acid molecule, or the nucleic acid molecule comprising the nucleic acid sequence may be attached to the nucleic acid barcode molecule and subjected to a nucleic acid reaction (e.g., extension, ligation) to generate the barcoded nucleic acid molecule. A barcoded nucleic acid molecule may serve as a template, such as a template polynucleotide, that can be further processed (e.g., amplified) and sequenced to obtain the target nucleic acid sequence. For example, in the methods and systems described herein, a barcoded nucleic acid molecule may be further processed (e.g., amplified) and sequenced to obtain the nucleic acid sequence of the nucleic acid molecule (e.g., mRNA).

The term "sample," as used herein, generally refers to a biological sample of a subject. The biological sample may comprise any number of macromolecules, for example, cellular macromolecules. The sample may be a cell sample. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears.

The term "biological particle," as used herein, generally refers to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or may include a matrix (e.g., a gel or polymer matrix) comprising a cell or one or more constituents from a cell (e.g., cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix.

The terms "coupled," "linked," "conjugated," "associated," "attached," "connected" or "fused," as used herein, may be used interchangeably herein and generally refer to one molecule (e.g., polypeptide, receptor, analyte, etc.) being attached or connected (e.g., chemically bound) to another molecule (e.g., polypeptide, receptor, analyte, etc.).

The term "binding agent," as used herein generally refers to a molecule capable of binding to one or more other molecules (e.g., analytes, receptors, other binding agents, etc.) and that comprises one or more portions. In some cases, a binding agent comprises at least one, at least two, at least three, or at least four portions. Each portion may comprise a polypeptide. The polypeptide of a specific portion may be capable of binding one or more molecules. For example, a polypeptide of a specific portion may bind to a molecule located on a surface of a cell, such as a cell surface protein or receptor (e.g., a CD surface marker such as CD45). A polypeptide of another portion of a binding agent may bind a molecule that may be secreted from a cell (e.g., T cell, B-cell, dendritic cell, etc.). The one or more portions of a binding agent may be directly or indirectly linked to, conjugated to, or fused to one another. For example, a first portion of a binding agent may be directly or indirectly linked to, conjugated to, or fused to a second portion of the binding agent. Moreover, the terms "binding agent," "polypeptide," and "antibody" may be used interchangeably herein.

The term "cell bead," as used herein, generally refers to a hydrogel, polymeric, or crosslinked material that comprises (e.g., encapsulates, contains, etc.) a biological particle (e.g., a cell, a nucleus, a fixed cell, a cross-linked cell), a virus, components of or macromolecular constituents of or derived from a cell or virus. For example, a cell bead may comprise a virus and/or a cell. In some cases, a cell bead comprises a single cell. In some cases, a cell bead may comprise multiple cells adhered together. A cell bead may include any type of cell, including without limitation prokaryotic cells, eukaryotic cells, bacterial, fungal, plant, mammalian, or other animal cell types, mycoplasmas, normal tissue cells, tumor cells, immune cells, e.g., a T-cell (e.g., CD4 T-cell, CD4 T-cell that comprises a dormant copy of human immunodeficiency virus (HIV)), a B cell, or a dendritic cell, a fixed cell, a cross-linked cell, a rare cell from a population of cells, or any other cell type, whether derived from single cell or multicellular organisms. Furthermore, a cell bead may comprise a live cell, such as, for example, a cell may be capable of being cultured. Moreover, in some examples, a cell bead may comprise a derivative of a cell, such as one or more components of the cell (e.g., an organelle, a cell protein, a cellular nucleic acid, genomic nucleic acid, messenger ribonucleic acid, a ribosome, a cellular enzyme, etc.). In some examples, a cell bead may comprise material obtained from a biological tissue, such as, for example, obtained from a subject. In some cases, cells, viruses or macromolecular constituents thereof are encapsulated within a cell bead. Encapsulation can be within a polymer or gel matrix that forms a structural component of the cell bead. In some cases, a cell bead is generated by fixing a cell in a fixation medium or by cross-linking elements of the cell, such as the cell membrane, the cell cytoskeleton, etc.

The term "macromolecular constituent," as used herein, generally refers to a macromolecule contained within or from a biological particle. The macromolecular constituent may comprise a nucleic acid. In some cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The terms "antigen binding fragment," "epitope binding fragment," or "antibody fragment," as used herein, may be used interchangeably and generally refer to a portion of a complete antibody (e.g., comprising each domain of the light and heavy chains respectively) capable of binding the same epitope/antigen as the complete antibody, albeit not necessarily to the same extent. Although multiple types of epitope binding fragments are possible, an epitope binding fragment typically comprises at least one pair of heavy and light chain variable regions (VH and VL, respectively) held together (e.g., by disulfide bonds) to preserve the antigen binding site and does not contain all or a portion of the Fc region. Epitope binding fragments of an antibody can be obtained from a given antibody by any suitable technique (e.g., recombinant DNA technology or enzymatic or chemical cleavage of a complete antibody), and typically can be screened for specificity in the same manner in which complete antibodies are screened. In some embodiments, an epitope binding fragment comprises an F(ab')$_2$ fragment, Fab' fragment, Fab fragment, Fd fragment, or Fv fragment. In some embodiments, the term "antibody" includes antibody-derived polypeptides, such as single chain variable fragments (scFv), diabodies or other multimeric scFvs, heavy chain antibodies, single domain antibodies, or other polypeptides comprising a sufficient portion of an antibody (e.g., one or more complementarity determining regions (CDRs)) to confer specific antigen binding ability to the polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

The term "analyte," as used herein, generally refers to a species of interest for detection. An analyte may be biological analyte, such as a nucleic acid molecule or protein. An analyte may be an atom or molecule. An analyte be a subunit of a larger unit, such as, e.g., a given sequence of a polynucleotide sequence or a sequence as part of a larger sequence. An analyte of the present disclosure includes a secreted analyte, a soluble analyte, and/or an extracellular analyte.

The present disclosure provides methods and systems for single cell analyte detection and measurement comprising nucleotide barcoded polypeptides that may be coupled to secreted analytes from single cells. The methods and systems described herein may comprise performing next generation sequencing for measuring and analyzing analyte molecules (e.g., cytokines, mRNAs, etc.) that may be secreted from and/or present in a single cell. The herein disclosed methods and systems may increase the number of different molecules or analytes that can be measured from a single cell compared to other cell analysis methods. Moreover, the disclosed methods and systems may allow simultaneous measurement of a plurality of cellular molecules (e.g., secreted and/or intracellular molecules), such as secreted analytes (e.g., cytokines), mRNAs, cell surface proteins, paired $\alpha\beta$ T-cell receptor sequences, and antigen binding specificity, etc.

**Barcoding Analytes from a Single Cell**

Provided herein are methods and systems for processing one or more analytes (e.g., secreted molecules or cellular nucleic acids) from a cell (e.g., an immune cell such as a T cell, B cell, or dendritic cell). The methods and systems described herein may comprise coupling (e.g., covalently or non-covalently binding or linking) a barcoded polypeptide (e.g., a polypeptide coupled to nucleic acid molecule comprising a barcode sequence) to an analyte secreted from a single cell (e.g., an immune cell such as a T cell, B cell, or dendritic cell).

In some embodiments, a method of processing a secreted molecule from a cell may comprise: (i) coupling the secreted molecule to a capture agent (e.g., a first polypeptide) coupled to a surface of the cell to form a first conjugate; and (ii) coupling a reporter agent (e.g., second polypeptide) to the secreted molecule to form a second conjugate, wherein the reporter agent (e.g., second polypeptide) comprises a nucleic acid molecule comprising a first barcode sequence (e.g., reporter barcode sequence). The methods disclosed herein may further comprise, prior to (i), incubating the cell with the capture agent such that the capture agent couples to the surface of the cell, e.g., via binding to a cell surface receptor such as CD45. The cell may be stimulated to induce secretion of the secreted molecule. Secretion of a molecule (e.g., a cytokine, hormone, or growth factor) may be induced by using one or more stimulatory (or co-stimulatory) molecules. Stimulatory molecules or antigens that may be used with the methods described herein include, but are not limited to, a Pattern recognition receptor (PRR) ligand, wherein the PRR ligand may be a Toll-like receptor (TLR) ligand, a NOD-like receptor (NLRs) ligand, a RIG-I-like receptor (RLR) ligand, a C-type lectin receptor (CLR) ligand, or a cytosolic dsDNA sensor (CDS) ligand. Further, secretion may be stimulated using a lipopolysaccharide (LPS), a double-stranded DNA (dsDNA), a double-stranded RNA (dsRNA), a synthetic dsRNA, or CpG oligodeoxynucleotides (CpG ODN), wherein the synthetic dsRNA may be polyinosinic-polycytidylic acid (poly I:C) or polyadenylic-polyuridylic acid (poly(A:U)).

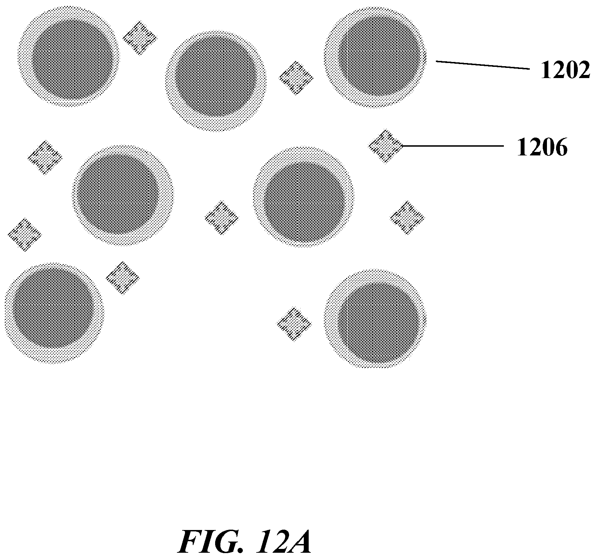

Cell samples comprising one or more cells (e.g., immune cells such as a T cells, B cells, and/or dendritic cells) that may be analyzed using the herein disclosed methods and systems may be obtained by various means. For example, a cell sample may be isolated from a biological fluid, such as plasma or may be isolated from a specific tissue or organ from a subject. In certain embodiments, cells **1202** (e.g., immune cells such as T cells, B cells, dendritic cells, etc.) along with their secreted analytes **1206** may be provided as shown in **FIG. 12A**; **1206** may also be a stimulatory molecule such as a molecule comprising an antigen.

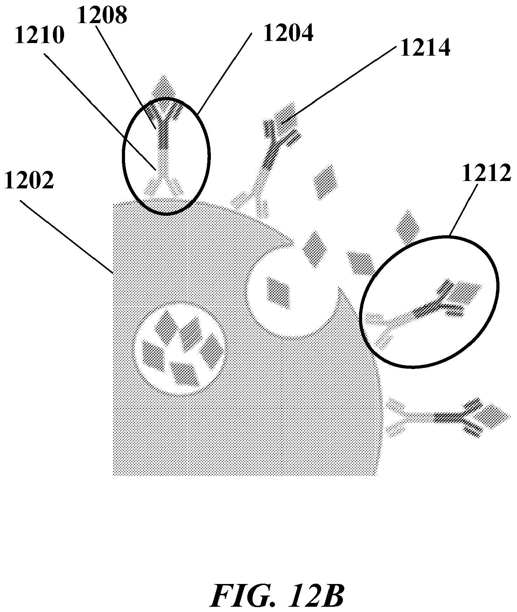

In some embodiments, the methods, kits, and systems disclosed herein comprise use of binding agents (e.g., a capture and reporter agent comprising a first and a second polypeptide, respectively) capable of binding the secreted analyte. For example, as shown in **FIG. 12B,** cells **1202** (e.g., T cells, B-cells, dendritic cells, etc.) are contacted with a capture agent **1204.**

Generally, as disclosed herein, a capture agent may comprise one or more portions. In some cases, a capture agent comprises at least one, at least two, at least three, or at least four or more portions. Each portion may comprise a polypeptide. The polypeptide of a specific portion may be capable of binding one or more molecules. For example, a polypeptide of a specific portion may bind to a molecule located on a surface of a cell, such as a cell surface protein or receptor (e.g., a CD surface marker such as CD45). A polypeptide of another portion of a capture agent may bind a molecule that may be secreted from a cell (e.g., T cell, B-cell, dendritic cell, etc.). The one or more portions of a capture agent may be directly or indirectly linked to, conjugated to, or fused to one another. For example, a first portion of a capture agent may be directly or indirectly linked to, conjugated to, or fused to a second portion of the capture agent. In some cases, the first portion (e.g., **1208** in **FIG. 12B**) is bound (e.g., chemically such as covalently or non-covalently) or linked to the second portion (e.g., **1210** in **FIG. 12B**). In some cases, the first portion and the second portion are connected via a linker (e.g., an amino acid linker). In some cases, the first portion and the second portion are connected via one or more domains or polypeptide chains. A capture agent as described herein may comprise one or more immunoglobulin (or antibody) molecules (e.g., IgA, IgE, IgG, or IgM molecules) or any fragments (e.g., epitope-binding fragments) or derivatives thereof, and in any suitable combination. For example, a capture agent may be a bispecific antibody. A bispecific antibody may comprise a first portion comprising a capture agent (e.g., a first polypeptide) capable of binding a cell surface molecule (e.g., a CD receptor), and a second portion comprising a second polypeptide capable of binding one or more molecules of a cell (e.g., one or more molecules secreted from the cell, one or more soluble molecules from the cell, and/or one or more extracellular molecules of the cell, including molecules that are in exosomes or extracellular vesicles, e.g., molecules on the surface of an exosome or microvesicle). In some cases, the capture agent (e.g., the first polypeptide) of a first portion of a capture agent may comprise a first immunoglobulin molecule (e.g., IgA, IgE, IgG, or IgM), or fragment(s) or derivative(s) thereof (e.g., a Fab, F(ab')$_2$, bispecific Fab$_2$, scFv, diabodies, etc.), and the second polypeptide of a second portion of a binding agent may comprise a second immunoglobulin molecule (e.g., IgA, IgE, IgG, or IgM), or fragment(s) or derivative(s) thereof (e.g., a Fab, F(ab')$_2$, bispecific Fab$_2$, scFv, diabodies, etc.)

The first polypeptide (e.g., the first immunoglobulin molecule) of a capture agent may be capable of binding a molecule on the surface of a cell, and the second polypeptide (e.g., the second immunoglobulin molecule) may be capable of binding one or more molecules secreted from the cell.

The one or more portion of a capture agent comprising one or more polypeptides, respectively, may be associated, e.g., bound to, linked to, or coupled to one another. For example, a first portion of a capture agent comprising a first polypeptide may be linked to a second portion of a capture agent comprising a second polypeptide via a linker. The linker may be a flexible linker, allowing efficient binding of the first and second polypeptide to their respective binding partners. The linker may be an amino acid linker such as a glycine-rich linker. A linker may be a cleavable or a non-cleavable linker.

In some embodiments, a capture agent comprises a polypeptide **1204**. The polypeptide **1204** may be a bispecific antibody (or another antibody construct such as a trispecific antibody) comprising a first portion **1208** capable of binding a molecule (e.g., a cytokine, a hormone, or a growth factor) secreted from the cell (e.g., an immune cell such as a T cell, a B cell, or a dendritic cell), and a second portion **1210** capable of binding a cell surface molecule such as a cell surface receptor (e.g., CD45). Cells (e.g., T cells) may be incubated with the capture agent comprising polypeptide **1204** in a solution for a certain amount of time under a reasonable and sufficient condition to allow coupling of polypeptide **1204** to the cell surface (e.g., via the cell surface-binding portion **1210** of polypeptide **1204**). In certain embodiments, the polypeptide **1204** is a bispecific antibody comprising a first portion **1208** and a second portion **1210**. In some embodiments, the first portion **1208** is an antibody or an epitope binding fragment thereof that is capable of coupling to secreted analytes (e.g., cytokines) from a single cell (e.g., an immune cell). Exemplary cytokines that may be analyzed using the methods, kits, and systems described herein include, but are not limited to tumor necrosis factor superfamily members (such as TNFα, CD27, CD30, CD40, etc.), interferons (such as IFN-α, IFN-β, IFN-γ, etc.), transforming growth factors (such as TGF-β, etc.), interleukins (such as IL-1, IL-2, IL-4, IL-6, IL-10, IL-13, IL-17RA, IL-17RB, IL-17RC, IL17RD, IL-17RE, IL-22, etc), colony-stimulating factors (such as M-CSF, GM-CSF, etc.), and chemokines (such as CC chemokines, CXC chemokines, etc.). In some embodiments, the first portion **1208** comprises an antibody or an epitope binding fragment thereof that has a binding affinity for one or more molecules, such as for one or more different types of cytokines, e.g., the first portion **1208** may bind one or more molecules (e.g., one or more cytokines) that come into its vicinity. In other embodiments, the first portion **1208** of the polypeptide **1204** comprises an antibody that has a binding affinity for one or more members of one or more particular families of cytokines, such as the TNF or interleukin family, e.g., the portion **1208** may selectively bind to one or more TNF family members or one or more interleukin family members. The first portion **1208** may be selected such that it binds to a particular secreted analyte of interest from the cell or cells (e.g., immune cells). In other embodiments, the first portion **1208** comprises a plurality of antibodies or fragments thereof (e.g., an epitope binding fragment) each may be selective for a certain analyte such as a cytokine. For example, the first portion **1208** may comprise two antibodies or fragments thereof (e.g., epitope binding fragments) that each have a binding affinity for a different analyte. The first portion **1208** may comprise one antibody, two antibodies, three antibodies, four antibodies, five antibodies, six antibodies, seven antibodies, or eight antibodies or fragments thereof, wherein each antibody or fragments thereof has a binding affinity for a different analyte. In other cases, the one or more antibodies and/or antibody fragments (e.g., epitope binding fragments) have a binding affinity for the same analyte (e.g., the same cytokine).

Further, in certain embodiments, the second portion **1210** comprises an antibody or an epitope binding fragment thereof. The second portion **1210** may be configured such that it targets, binds, or otherwise associates with one or more cell surface proteins, such as receptor tyrosine kinases (RTKs), a G-protein-coupled receptors (GPCRs), cluster of differentiation (CD) proteins, (such as CD45, etc.), etc., or any combination thereof. In some embodiments, the second portion **1210** is configured to bind (e.g., covalently or non-covalently bind) to the cell surface and allow the first portion **1208** to bind (e.g., covalently or non-covalently) to one or more secreted analytes as described herein. A capture agent (or a polypeptide or portion thereof) may bind to a cell surface in a variety of ways. For example, the second portion **1210** of polypeptide **1204** may be attached to the cell surface using any suitable approach, such as attachment through cysteine residues or through unnatural amino acids having reactive functionality. In some cases, the second portion **1210** is attached to the surface via its carbohydrate groups (e.g., glycosylation sites), for example, on the constant region (e.g., Fc) of the antibody, or via lipid-lipid, or lipid-protein interactions. Moreover, in some embodiments, the second portion **1210** comprises two antibodies or epitope binding fragments thereof, each having a binding affinity to a different cell surface molecule, respectively. In other embodiments, the second portion **1210** comprises three, four, five, six, seven, eight or more antibodies or epitope binding fragments thereof, each selectively targeting a specific cell surface molecule (e.g., the same or different cell surface molecules).

In some embodiments, a first portion of a capture agent (e.g., those comprising polypeptide **1204**) may be linked to or conjugated to (e.g., chemically linked or conjugated to) a second portion. Alternatively, a first portion of a capture agent may be fused (e.g., recombinantly fused) to a second portion. Thus, in some embodiments, polypeptide **1204** is a fusion protein comprising the first portion **1208** and the second portion **1210.** The fusion polypeptide **1204** may be generated by recombinant DNA technology, e.g., translation of fusion genes (one or more genes encodes for the first portion **1208** and the other one or more genes encodes for the second portion **1210**) results in a fusion polypeptide **1204.** In certain embodiments, one or more linker (e.g., amino acid linker such as polypeptide linker) link the first portion **1208** to the second portion **1210** to, e.g., ensure proper folding of the first and second portion and/or to ensure effective cell surface and/or analyte binding. Furthermore, polypeptide linker may allow important domain interactions, reinforce stability, and reduce steric hindrance. In other embodiments, the first portion protein **1208** and the second portion protein **1210** are linked or conjugated post-translationally. For example, a polypeptide of a first portion of a capture agent (e.g., portion **1208**) may be conjugated to a second portion (e.g., portion **1210**) using any suitable bioconjugation strategy including but not limited to activated esters (e.g., NHS esters), cycloaddition reactions, Staudinger ligation, click chemistry etc. In yet other embodiments, the first portion protein **1208** and the second portion **1210** can be connected end-to-end via linkage of N or C termini between the first portion **1208** and the second portion **1210**, providing a flexible bridge structure for proper folding and reduced steric hindrance.

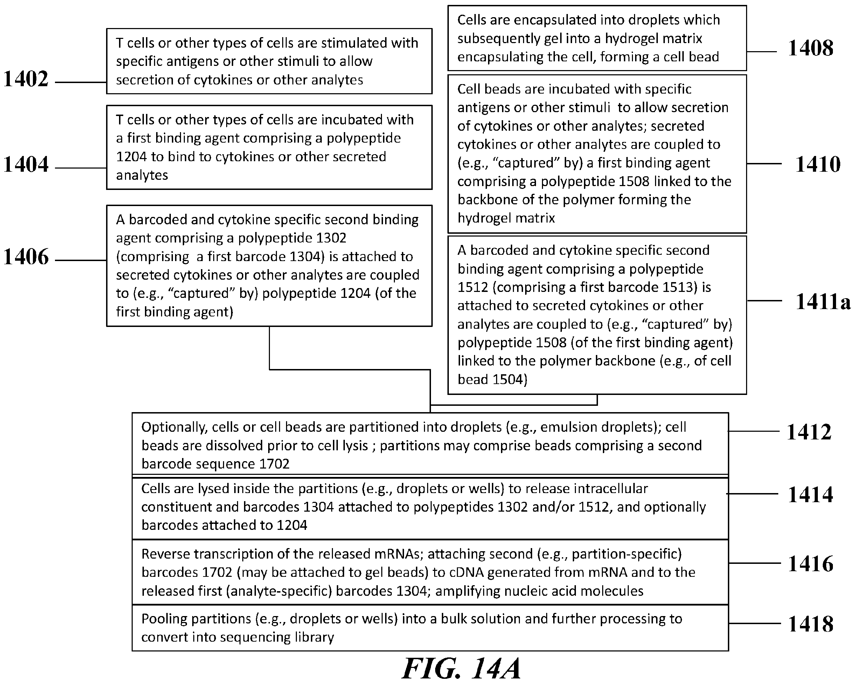

**FIG. 14A** illustrates an exemplary workflow for detecting secreted analytes from a single cell. Generally, the methods and systems described herein may comprise stimulating a cell (e.g., an immune cell) to induce secretion of one or more analytes (e.g., secreted molecules) from the cell. Stimulating a cell (e.g., an immune cell such as a T cell, B cell, or dendritic cell) to induce secretion of one or more analytes (e.g., secreted molecules such as cytokines or antibodies) may include subjecting (e.g., contacting) the cell to specific molecules (e.g., stimulatory or co-stimulatory molecules). Specific molecules that may be used to induce secretion of an analyte from the cell may include antigens such as Pattern recognition receptor (PRR) ligands (e.g., Toll-like receptors (TLR) ligands, NOD-like receptor (NLRs) ligands, RIG-I-like receptor (RLR) ligands, C-type lectin receptor (CLR) ligands, cytosolic dsDNA sensor (CDS) ligands, etc.), lipopolysaccharide (LPS), double-stranded DNA (dsDNA), double-stranded RNA (dsRNA), a synthetic dsRNA (e.g., polyinosinic-polycytidylic acid (poly I:C) or polyadenylic-polyuridylic acid (poly(A:U)), CpG oligodeoxynucleotides (CpG ODN), or any combination thereof. The methods described herein may include providing a plurality of one or more different stimulatory molecules to the cell at a concentration that is sufficient to induce secretion of the one or more secreted molecule (e.g., cytokines).

Referring to operation **1402**, immune cells (e.g., T cells) or other cell types of interest are incubated with a suitable concentration of antigens (or other stimulatory molecules) to induce secretion of cytokines or other analytes. Any suitable concentration of stimulatory molecules (e.g., antigens) sufficient to induce cytokine secretion from the cells (e.g., T cells) may be used herein. In some cases, a stimulatory or co-stimulatory molecule (e.g., an antigen) is coupled to a major histocompatibility complex (MHC) molecule. In some cases, the MHC molecule is an MHC multimer (e.g., a monomer, dimer, trimer, tetramer, pentamer, etc.) resulting in a multimeric (e.g., a monomeric, dimeric, trimeric, tetrameric, etc.) MHC-antigen complex (e.g., MHC-peptide complex if the antigen is a peptide). The MHC multimer may be linked to a cell or a polymer. The cell may be an antigen-presenting cell (APC). The polymer may be a dextran polymer (e.g., a dextramer). An MHC multimer (e.g., tetramer or dextramer) or an APC may comprise a plurality of MHC complexes. The MHC molecules/multimers may comprise one or more stimulatory molecules, such as antigenic peptides, thereby forming peptide-MHC complexes. Thus, MHC molecules (e.g., multimers) and/or APCs may be used to present stimulatory and/or co-stimulatory molecules (e.g., stimulatory peptides via MHC-peptide complexes) to the cell (e.g., an immune cell) to induce secretion of the one or more analytes. Moreover, cell or other non-cell constructs may be used to present stimulatory molecules to a cell to induce secretion of one or more analytes. In some cases, an antigen-presenting cell (APC) may comprise a plurality of MHC molecules (e.g., MHC multimers), and thus an APC may be used to induce analyte secretion of a cell (e.g., an immune cell). A co-stimulator molecule as described herein may be an antibody (e.g., an anti-CD3 or an anti-CD28 antibody) or a cytokine (e.g., an interleukin). An APC or an MHC multimer (such as a tetramer or dextramer), for example, may comprise a plurality of co-stimulatory molecules and thus may be used to induce analyte secretion from a cell. In some cases, the stimulator or co-stimulatory molecules, the MHC multimer, the APC, and/or the MHC molecules as described herein may comprise a nucleic acid molecule comprising a barcode sequence. In some cases, methods disclosed herein comprise antigens being part of an antigen-MHC complex (e.g., an antigen-MHC tetramer) comprising the antigen (e.g., a peptide or polypeptide) and an MHC molecule (e.g., an MHC multimer such as a tetramer). The MHC molecule may comprise a nucleic acid molecule comprising a barcode sequence that identifies the peptide(s) present in an MHC molecule or multimer. See, e.g., U.S. Pat. 10,011,872, which is incorporated by reference in its entirety, for exemplary molecules and methods for analyzing cells and immune receptors using barcoded MHC labelling agents. The MHC coupled barcode sequence as used and described herein may be different than the first barcode sequence (e.g., attached to a capture agent and/or reporter agent) and the second barcode sequence (e.g., cell or partition-specific barcode, such as a barcode attached to a bead, e.g., a gel bead).

The methods and systems described herein may comprise use of one or more barcoded components or molecules (e.g., capture agents or portions thereof) to analyze a biological sample (e.g., a population of immune cells) on a single cell basis. In some embodiments, the methods described herein comprise use a secondary binding agent (e.g., reporter agent) that is capable of binding an analyte secreted from a cell (e.g., a cytokine). The reporter agent may bind to the analyte while the analyte is bound to the capture agent that is bound to the cell surface (see e.g., polypeptide (primary binding agent) **1204** in **FIG. 12B**). The reporter agent may be an antibody or fragment (e.g., an epitope-binding fragment) or derivative thereof. The reporter agent may be an antibody capable of binding the analyte bound to the capture agent. The reporter agent may comprise a barcode (e.g., reporter barcode sequence), e.g., an oligonucleotide sequence comprising a barcode sequence, and may barcode the analyte. Thus, an analyte (e.g., a secreted molecule) may be barcoded while bound to the reporter agent that in turn may be bound to the cell that secreted said analyte. Barcodes as described herein may be used to associate one or more analytes (e.g., secreted molecules or cellular nucleotide sequences such as mRNAs) with a cell and/or a partition (e.g., droplet or well) upon analyzing the barcodes using e.g., sequencing reads generated using an Illumina sequencer.

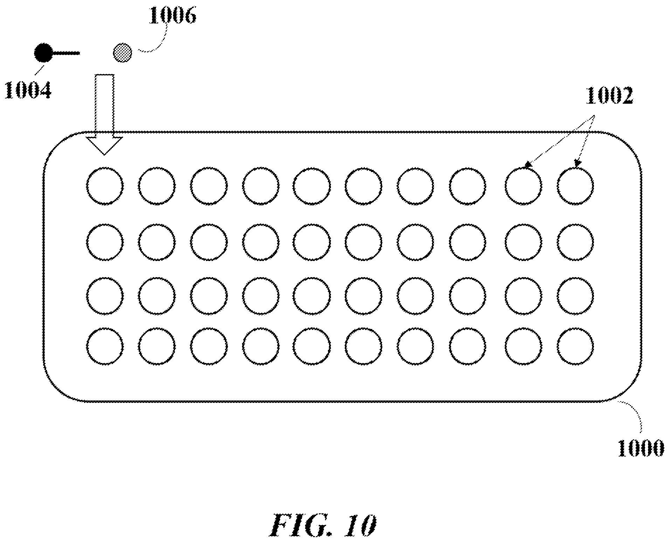

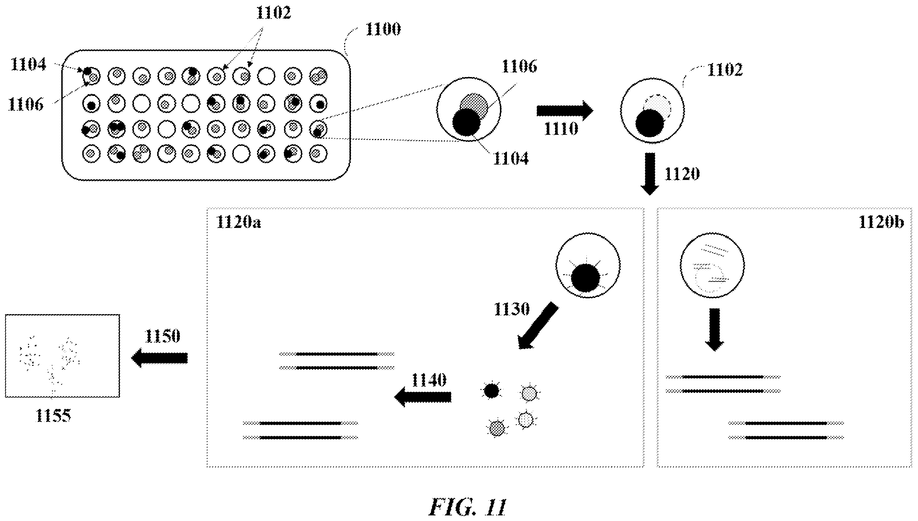

The herein described methods may comprise use of multiple barcodes to analyze multiple analytes and cellular molecules such as cytokines and/or mRNA molecules. A barcode may be a nucleic acid sequence (barcode sequence). A first barcode may be different from a second barcode. For example, the nucleic acid sequence of a first barcode sequence may be different from that of a second barcode sequence. As described herein, nucleic acid molecules comprising a barcode sequence may be coupled to other molecules, polymer, or particles. For example, nucleic acid molecules comprising a barcode sequence may be coupled to MHC molecules (e.g., tetrameric MHC-peptide complexes comprising a barcode sequence), secondary binding agents (e.g., an antibody coupled to a barcode sequence), polymers (e.g., dextramers or polymers capable of forming hydrogels), and/or beads (e.g., beads in emulsion droplets, or in wells of a microwell array, e.g., as shown in **FIG. 10** and **FIG. 11**). Use of one or more barcodes may allow measurement and analysis of one or more analytes of a cell and may allow associating the one or more analytes with the respective cell. This may be particularly advantageous when measuring and analyzing multiple analytes (e.g., secreted molecules and/or mRNAs) from a single cell or from a plurality of cells such as one or more cell populations (e.g., immune cells). In such cases, a first barcode may be used to measure a first analyte of a cell (e.g., a secreted molecule such as a cytokine), and a second barcode may be used to measure a second analyte of the cell (e.g., an mRNA molecule), and so forth. In these instances, a partition or cell specific barcode (e.g., attached to a bead, such as a gel bead) may be utilized to link the first barcode and the second barcode to attribute one or more analytes to a single cell. The analysis of an immune cell (e.g., a T cell, B cell, or dendritic cell), for example, may comprise measuring one or more signaling molecules (e.g., cytokines) that may be secreted upon stimulation of the cell (e.g., by using a stimulatory molecule), and one or more mRNA molecules that may be released from the cell upon cell lysis for analyzing, e.g., immune cell receptor gene segments (e.g., a V(D)J sequence of a T cell receptor (TCR)). See, e.g., U.S. Pat. Pub. 2018/0105808, which is incorporated by reference in its entirety, for exemplary molecules and methods for analyzing V(D)J sequences of single cells using nucleic acid barcode molecules.

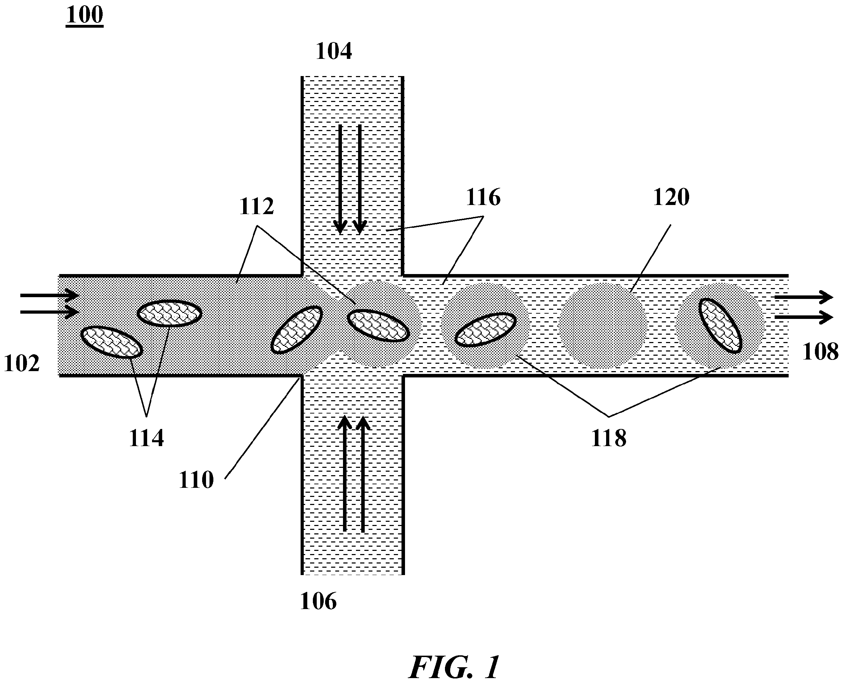

As described herein, analyzing and measuring analyte(s) of a cell (e.g., an immune cell) may be performed in a partition such as a droplet or a well (e.g., gel bead-in emulsions). Thus, a barcode such as nucleic acid barcode sequences may be specific for the partition (e.g., a droplet or a well) in which the cell may be encapsulated in, allowing the association of cellular analytes (e.g., secreted analytes and cellular nucleic acid molecules) with that cell. A partition (e.g., a droplet or a well) may be generated using methods described herein (see e.g., **FIG. 1 - FIG. 7, FIG. 10,** and **FIG. 11**).

Further, referring to operation **1404** (**FIG. 14A**), cells (e.g., T cells) or other cell types of interest are incubated with the capture agent. Incubation with the capture agent may be performed at a suitable concentration of the capture agent (e.g., comprising polypeptide **1204**) for a certain amount of time. Any suitable concentration of the polypeptide **1204** that can induce sufficient binding of the second portion **1210** to the cell surface is contemplated herein. Further, the incubation process in operation **1404** is conducted for a sufficient amount of time to allow the binding of the first portion proteins **1208** to secreted cytokines or any other analytes of interest and the binding of the second portion proteins **1210** to the cell surface. In certain embodiments, operation **1404** can be performed prior to operation **1402**. Incubating cells (e.g., immune cells) with antigens such as stimulatory and/or co-stimulatory molecules and/or cells (e.g., APCs) or polymers (e.g., dextramers) presenting the same may be configured to minimize decoupling or dissociation of the polypeptides **1204** from the cell surface.

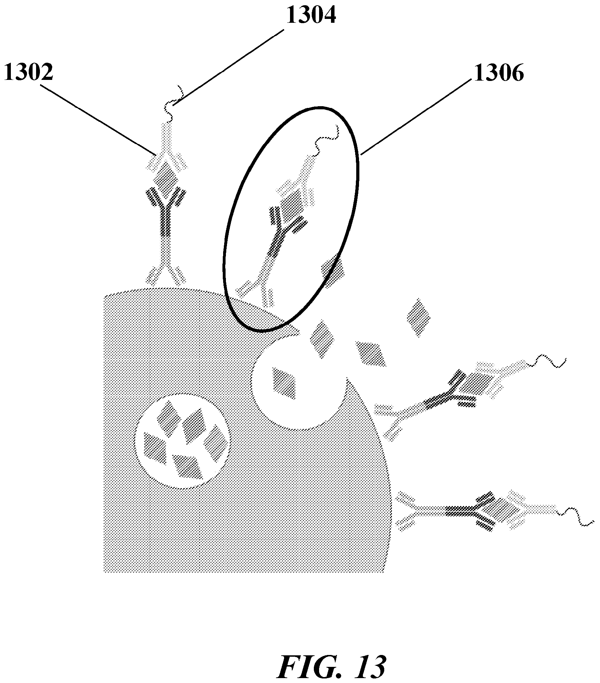

Referring to operation **1406**, after secreted cytokines or other analytes bind to the first portion protein **1208** to form a first conjugate **1212**, the cells (e.g., T cells) or other types of cells are further incubated with a solution comprising a plurality of polypeptides (e.g., reporter agents) **1302** (**FIG. 13**). Each reporter agent **1302** is coupled to a nucleic acid molecule **1304** comprising a barcode sequence (e.g., reporter barcode sequence). In certain embodiments, the reporter agent **1302** is a protein. In certain embodiments the reporter agent is an antibody or an epitope-binding fragment thereof that selectively binds to at least one cytokine. In other embodiments, the antibody selectively binds to other secreted analytes of interest. Further, in some embodiments, the reporter agent comprises a fluorophore, chromophore, heavy metal, or any combinations thereof in addition to the barcode **1304**. Examples of fluorophore that can be utilized here are fluorescein isothiocyanate (FITC), phycoerythrin (PE), allophycocyanin (APC), ALexa Four, DyLight, and etc.

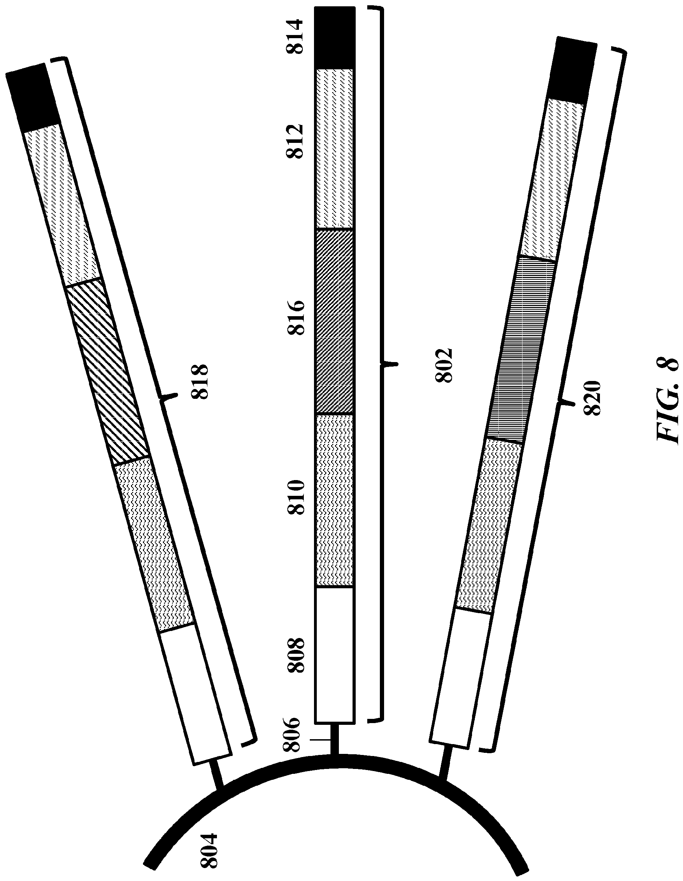

The reporter agent along with the barcode **1304** binds to cytokines that are captured by the capture agent **1204** to form a second conjugate **1306**. The barcodes attached to, e.g., MHC multimers (MHC barcode) utilized to stimulate cytokine secretion may be different from barcodes **1304** attached to the cytokine specific antibodies **1302**. In an exemplary embodiment, the barcodes, including barcodes **1304**, are DNA oligonucleotides. Accordingly, in some instances, attaching a cell-specific barcode (e.g., in a partition, such as a droplet-see **FIG. 8**) to molecules containing the cytokine-barcode (e.g., **1304**) and, where present, the MHC barcode allows the association of both the cytokine molecule and the protein-MHC complex with the same single cell.

In addition, e.g., during operation **1406**, additional barcoded molecules (e.g., antibodies or antibody fragments) that are selective towards certain cell surface proteins (cell surface protein specific molecules) may be added as well to analyze the presence and/or amount of cell surface protein. As a result, cell surface protein(s) of interest may be interrogated by binding these barcoded antibodies to the cell surface and subjecting them to further analysis using the barcoding reactions described herein (e.g., partition-based barcoding of cell surface protein specific molecules bound to single cells). See, e.g., U.S. Pat. 10,011,872, which is incorporated by reference in its entirety, for exemplary molecules and methods for analyzing protein molecules using barcoded labelling agents. The barcodes attached to the cell surface protein specific molecules (cell surface protein barcode) may be used to identify the cell surface protein specific molecules and, in some instances, are different from, e.g., the MHC multimer barcodes and the cytokine barcodes (e.g., **1304**). In certain embodiments, the cell surface protein barcodes are DNA oligonucleotides. In some instances, the nucleic acid molecules attached to the cell surface specific binding molecules may comprise one or more functional sequences in addition to the cell surface barcode sequence. For example, the nucleic acid molecule(s) attached to the cell surface binding molecules may comprise one or more of a unique molecular identifier (UMI), a primer sequence or primer binding sequence (e.g., a sequencing primer sequence (or partial sequencing primer sequence) such as an R1 and/or R2 sequence), a sequence configured to attach to the flow cell of a sequencer (e.g., P5 and/or P7), or sequence complementary to a sequence on a nucleic acid barcode molecule (e.g., attached to a bead, such as those described in **FIG. 8**). Accordingly, barcoded molecules (e.g., comprising a cell-surface specific and cell specific barcode) generated from molecules attached to a cell surface biding molecule (or a derivative thereof) may also comprise these functional sequences.

## Barcoding Analytes from a Single Cell using Cell Beads

Provided herein are methods and systems for processing one or more analytes (e.g., secreted molecules or cellular nucleic acids) from a cell (e.g., an immune cell such as a T cell, B cell, or dendritic cell). The methods and systems described herein may comprise coupling (e.g., covalently or non-covalently binding or linking) a barcoded polypeptide (e.g., a polypeptide coupled to nucleic acid molecule comprising a barcode sequence) to an analyte secreted from a single cell (e.g., an immune cell such as a T cell, B cell, or dendritic cell).

In some embodiments, a method of processing a secreted molecule from a cell may comprise: (a) generating a cell bead, wherein the cell bead comprises a cell encapsulated by a polymer matrix, wherein the polymer matrix comprises a plurality of capture agents (e.g., first polypeptides); and (b) coupling a molecule secreted from the cell to a capture agent of the plurality of capture agents to form a first conjugate.

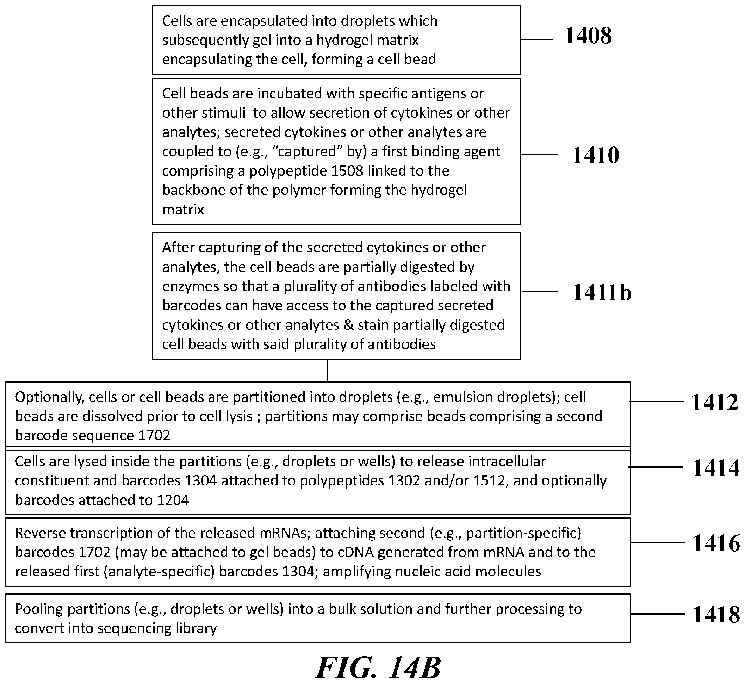

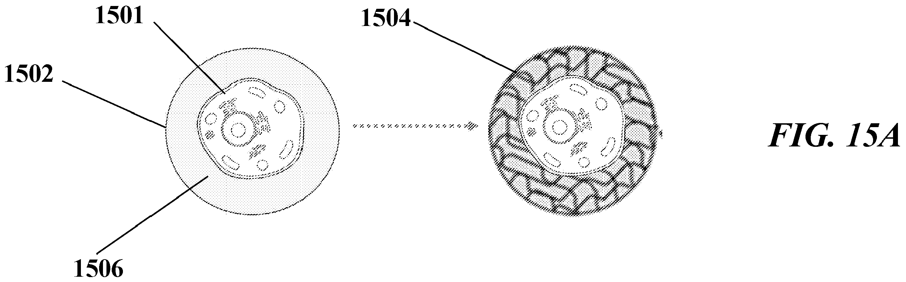

An exemplary secreted analyte barcoding process utilizing cell beads is described in **FIGS. 14A and 14B** (see, e.g., operation **1408**, **1410**, **1411a**, **1411b** etc.). In certain embodiments, a microfluidic channel structure (e.g., **FIGS. 1-7**) can be used to encapsulate a cell into a droplet **1502** as shown in **FIG. 15A**. See, e.g., U.S. Pat. Pub. 2018/0216162 (now U.S. Pat. 10,428,326) and U.S. Pat. Pub. 2019/0100632 (now U.S. Pat. 10,590,244), which are incorporated by reference in their entirety, for exemplary cell bead generation systems and methods. In certain embodiments, droplet **1502** comprises cell **1501** (e.g., a single cell) that and an aqueous solution comprising polymer and/or crosslink precursors **1506**. To encapsulate a cell into a droplet, in some embodiments, a microfluidic channel structure comprises a first aqueous fluid that includes suspended biological particles (e.g., cells or nuclei) may be transported along a first channel segment and brought into contact with a second fluid that is immiscible with the first aqueous fluid to create discrete droplets of the first aqueous fluid. In some instances, the first aqueous fluid includes other reagents as described elsewhere herein, including polymer and/or crosslink precursors for cell bead generation. In other instances, a second aqueous fluid containing other reagents, including polymer and/or crosslink precursors, is brought into contact with the first aqueous fluid prior to or concurrent with droplet generation. In still other instances, two discrete droplets containing a first aqueous fluid (e.g., comprising a cell) and a second aqueous fluid (e.g., comprising reagents, such as polymer and/or crosslink precursors) can be merged into a coalesced droplet. In some instances, a discrete droplet generated includes at most, a single biological particle (such as a single cell or single nucleus, see, e.g., droplet **1502**).

Further, the droplet **1502** encapsulating, e.g., cell **1501**, can be subjected to suitable conditions such that the polymer and/or crosslink precursors **1506** can be polymerized/crosslinked to generate extra-cellular matrix **1504** (**FIG. 15A**). Cell beads may be of uniform size or heterogeneous size. In some cases, the diameter of a cell bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a cell bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m

In some embodiments, the bead may comprise covalent or ionic bonds between polymeric precursors (e.g., monomers, oligomers, linear polymers), oligonucleotides, primers, and other entities. In some cases, the covalent bonds comprise carbon-carbon bonds or thioether bonds.

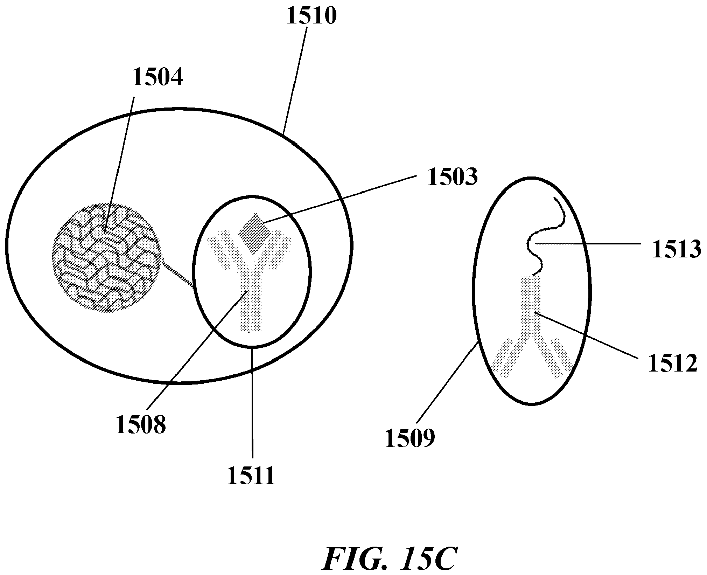

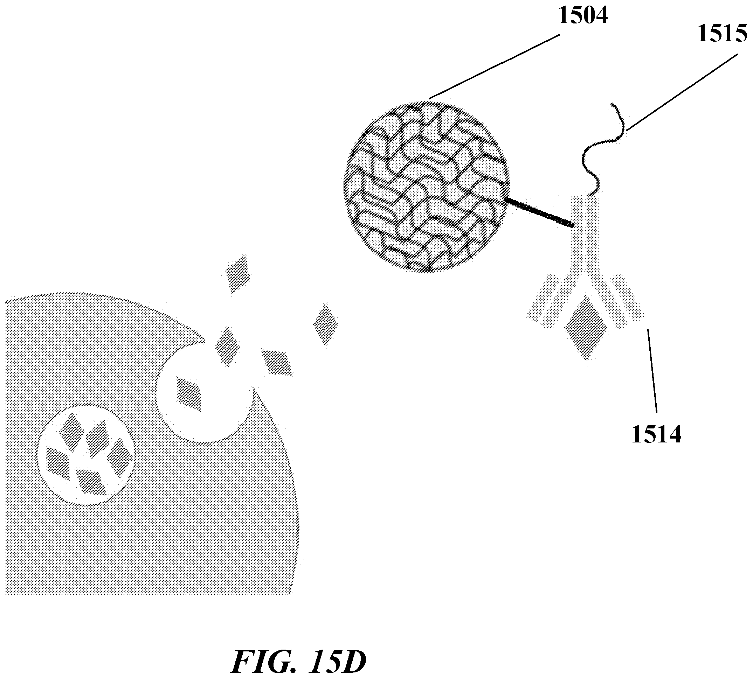

In some cases, a bead may comprise an acrydite moiety or click chemistry moiety, which in certain aspects may be used to attach one or more binding agents (e.g., capture agents comprising analyte specific antibodies **1508** (**FIG. 15C**)). See, e.g., U.S. Pat. Pub. 2019/0100632 (now U.S. Pat. 10,590,244), which is incorporated by reference in its entirety, for exemplary cell bead polymers and functionalization strategies. In some instances, a chemical conjugation method is utilized to attach molecules (e.g., capture agent **1508**) to the cell bead hydrogel matrix. In some instances, an antibody may comprise a chemical modification (e.g., click chemistry precursor such as an alkene/alkyne) that is configured to react with a chemical modification (e.g., click chemistry precursor such as an azide/alkyne) present in the polymer matrix or polymer precursors of a cell bead. In certain embodiments, the capture agents comprising the analyte specific antibody **1508** is coupled to the polymer backbone of the polymer matrix of cell beads **1504** as shown in **FIG. 15C.** Subsequent to binding or coupling of an analyte (e.g., a secreted cytokine **1503**) to the first capture agent **1508** to form a conjugate **1511**, a second binding agent (e.g., reporter agent) **1509** comprising an analyte specific antibody **1512** and a nucleic acid molecule comprising a barcode sequence (e.g., reporter barcode sequence) **1513**. In some embodiments, e.g., as shown in **FIG. 15D**, a hydrogel matrix **1504** (e.g., a cell bead) is coupled to a binding agent (e.g., reporter agent) comprising a polypeptide **1514** (e.g., an antibody) and a nucleic acid molecule **1515** comprising a first barcode sequence (e.g., reporter barcode sequence), where the binding agent is coupled to secreted analytes (e.g., cytokines or antibodies). In some embodiments, upon secretion, a secreted analyte is captured by an analyte-specific binding agent (e.g., reporter agent) coupled to the cell bead, while other secreted analytes not specifically captured by the binding agent is removed, e.g., washed away, from the cell bead. Thus, in some embodiments, the analyte-specific binding agent serves both as a capture agent and as a reporter agent for the secreted analyte.

In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as an oligonucleotide (e.g., barcode sequence, barcoded oligonucleotide, primer, or other oligonucleotides). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment is reversible, such that when the disulfide bond is broken (e.g., in the presence of a reducing agent), the attached species is released from the bead. In other cases, an acrydite moiety comprises a reactive hydroxyl group that may be used for attachment.

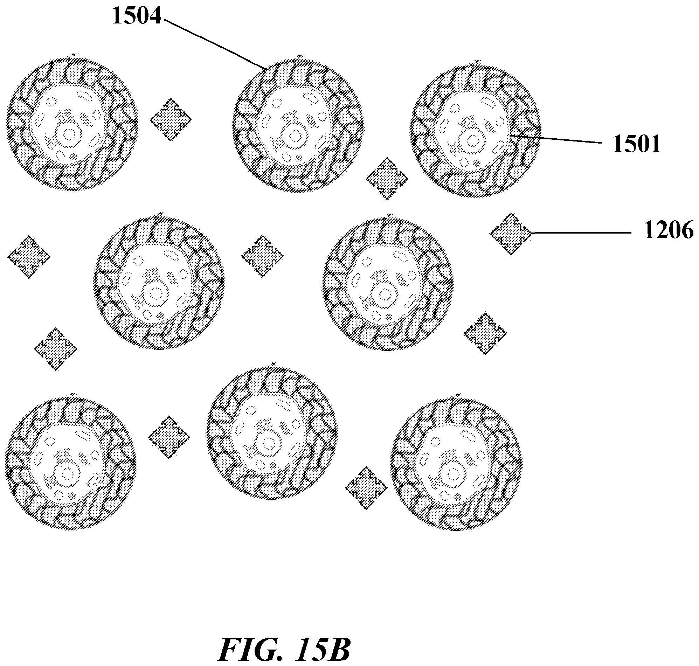

In some embodiments, after the formation of cell beads comprising cytokine-specific capture agents (e.g., antibodies) (e.g., complex **1511**), stimulatory agents (such as antigens) are used to stimulate the cells (e.g., T cells) to secrete cytokines or other analytes of interest as illustrated in operation **1410** (**FIGS. 14A and 14B**) and **FIG. 15B**. In certain embodiments, the antigens are coupled to barcoded peptide-major histocompatibility complex (MHC) molecules (e.g., MHC multimers such as tetramers or dextramers). Any suitable concentrations of antigen can be used to allow sufficient secretion of cytokines from the cell beads. Moreover, sufficient incubation time is employed to ensure cytokine secretion from the cell beads. After cytokines or other analytes of interest are secreted from the cells, a plurality of capture and/or reporter agents (e.g., analyte/cytokine specific antibodies) may couple to (e.g., covalently or non-covalently bind to) the secreted cytokines or other analytes. As a result, the capture agent may form a conjugate with a "captured" or bound cytokine or analyte.

Subsequently, e.g., in operation **1411a** (**FIG. 14A**), a reporter agent that, in some instances, comprises an analyte (e.g., cytokine) specific antibody comprising a nucleic acid molecule comprising a first barcode sequence (e.g., reporter barcode sequence) may couple to (e.g., covalently or non-covalently bind to) the secreted cytokine or other analyte bound to the capture agent. Further processing (e.g., attaching a cell and/or partition barcode and sequencing) of the reporter agent and/or nucleic acid molecule may allow for identification of the presence and/or amount of the secreted analyte.

Furthermore, as illustrated in operation **1411b,** after operation **1410,** the extra-cellular matrix (ECM) **1420** of the cell beads can be partially digested by enzymes. In some cases, the enzyme may be collagenase, which assists in breaking the peptide bonds in collagen. In other cases, the enzyme may be dispase, which is a protease which cleaves fibronectin, collagen IV, and to a lesser extent collagen I. In other cases, a suitable enzyme that can partially digest the ECM **1420** may be used here. Additionally, the ECM may comprise collagen, laminin, fibronectin, etc.

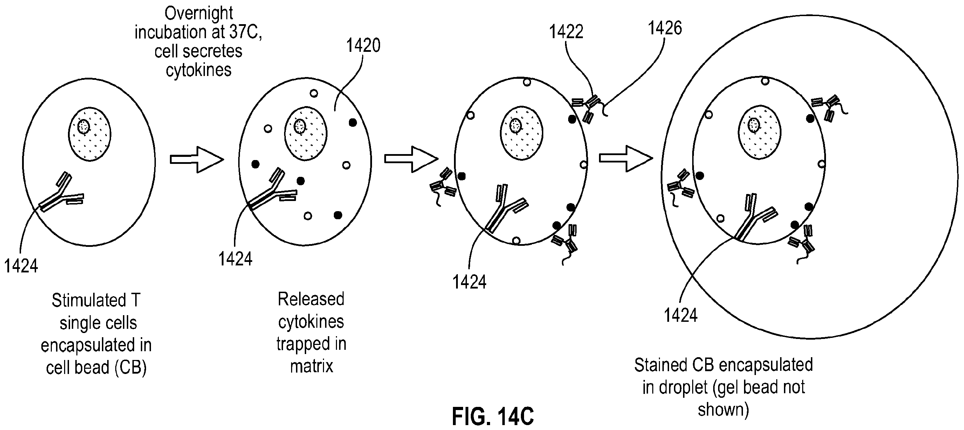

Partially digested ECM allows another analyte (e.g., cytokine) specific reporter agent (e.g., antibody) **1422** (**FIG**. **14C**) to bind to analytes captured by antibodies (e.g., capture agents) **1424**. As discussed above, a capture agent (e.g., an analyte specific antibody) **1424** is coupled to the polymer backbone of the ECM **1504** as shown in **FIG. 15C**. The analyte specific antibody (e.g., reporter agent) further comprises a third barcode sequence **1426** (**FIG. 14C**). In addition, in operation **1411b,** the partially digested cell beads are incubated with a plurality of antibodies, each comprising a barcode sequence. Excess antibodies may be washed away. After washing away excess antibodies, the cell beads stained with antibodies may be partitioned into emulsion droplets as described above in options **1412**, **1414**, **1416**, and **1418**.

**Analysis and Measurement of Barcoded Analytes**

The methods and systems disclosed herein may comprise measuring and/or analyzing one or more analytes of a cell. The analytes may be secreted molecules (e.g., molecules released/secreted from the cell upon stimulation of the cell) and/or other molecules such as nucleic acid molecules, surface proteins, and surface receptors of a cell. As described herein, an analyte of the one or more analytes may be coupled (e.g., directly or indirectly) to a first barcode (e.g., a nucleic acid molecule comprising a first barcode sequence). This may, for example, occur (i) on the surface of a cell, e.g., if the analyte is a surface receptor; (ii) after secretion of the analyte from the cell (e.g., if the analyte is a cytokine or another secreted molecule); or (iii) after lysis of the cell, e.g., when the analyte is a nucleic acid molecule (e.g., an mRNA molecule) of the cell.

In some embodiments, secretion of an analyte or molecule of interest (e.g., a cytokine) from a cell and coupling (e.g., binding such as covalent or non-covalent binding) of the analyte to one or more binding agents (e.g., a capture agent and a reporter agent), is followed by co-partitioning the cell into a partition (see e.g., **FIGS. 14A and 14B**). In other cases, co-partitioning occurs prior to secretion of an analyte from the cell and coupling of the analyte to the one or more binding agents. In some embodiments, at least some of the partitions may comprise at most one cell. In some cases, a partition comprises at least one cell. A partition as described herein may include a droplet or a well (e.g., in a microwell array). A droplet may be an emulsion droplet. A droplet (e.g., an emulsion droplet) may be formed by bringing a first phase in contact with a second phase that is immiscible with the first phase. In other cases, the partition may be a well as part of a plurality of wells. In yet other cases, the partition may be a chamber as part of a plurality of chambers. Partitions may be fluidically isolated from one another or physically isolated from one another.

Thus, in some instances, a partition (e.g., a droplet or a well) may comprise a cell, a capture agent and a reporter agent, wherein the capture agent and the reporter agent may be coupled to an analyte, and coupled directly and/or indirectly to the cell (see e.g., **FIG. 12B, FIG. 13,** and **FIG. 14C**). In some cases, the reporter agent comprises (e.g., is coupled or linked to) a first, analyte-specific barcode (e.g., reporter barcode sequence). A first barcode may comprise a nucleic acid molecule comprising a first barcode sequence.

A partition (e.g., a droplet or a well) may further comprise a plurality of nucleic acid molecules, wherein each nucleic acid molecule of the plurality of nucleic acid molecules comprises a second barcode sequence (e.g., cell or partition-specific barcode), and wherein the second barcode sequence is different from the first, analyte-specific barcode sequence. The plurality of nucleic acid molecules comprising the second barcode sequence may be coupled to (e.g., covalently or non-covalently bound or linked to) a particle, a polymer, or macromolecular structure. In some cases, the plurality of nucleic acid molecules comprising the second barcode sequence is coupled to (e.g., covalently or non-covalently linked to) a bead. The bead may be a gel bead as described elsewhere herein.

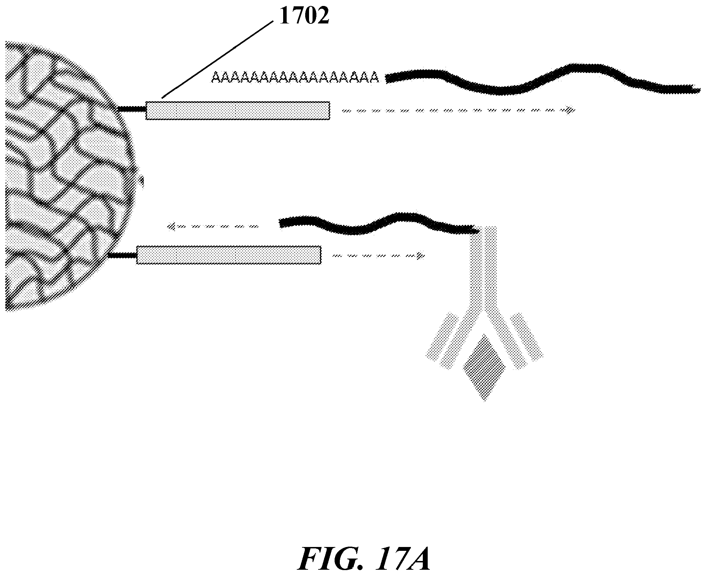

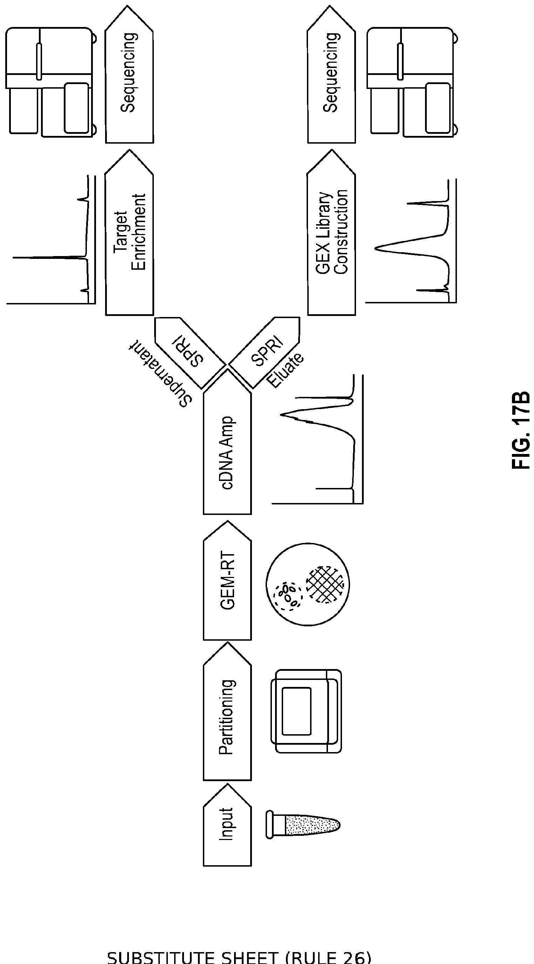

In some embodiments, the plurality of nucleic acid molecules comprising the second barcode sequence is coupled to (e.g., covalently or non-covalently bound or linked to) a bead (e.g., a gel bead). A nucleic acid molecule comprising the second barcode sequence may be coupled to the bead using any suitable coupling strategy such as bioconjugation reactions (e.g., Staudinger ligation or streptavidin-biotin coupling) or click chemistry. A bead comprising the plurality of nucleic acid molecules comprising the second barcode sequence may be co-partitioned into a partition, such as a droplet. Thus, a partition (e.g., a droplet or a well) as described herein may comprise a cell (e.g., comprising a capture and/or reporter agent and an analyte-specific barcode) and a bead comprising a partition/cell-specific barcode, see, e.g., **FIG. 8**). The cell may be stimulated (e.g., using a stimulatory molecule such as an antigen) to secrete an analyte, wherein the analyte, upon secretion from the cell, may be coupled to the capture and reporter agents (e.g., antibodies or bispecific antibodies). The first barcode sequence (analyte specific barcode coupled to the reporter agent) may be attached or otherwise processed to add the second barcode sequence (cell specific barcode, which in some instances is attached to a bead), thereby associating the secreted analyte with the cell (and in some instances, the partition). In some cases, the cell is lysed inside the partition (e.g., the droplet or well) and thus additional analytes (e.g., nucleic acid molecules) of the cell may be barcoded (e.g., with the second barcode sequence). Thus, the second cell-specific barcode sequence may associate the analyte(s) with the cell. In some cases, mRNA molecules that are released from the cell after cell lysis are reverse transcribed using the nucleic acid molecules comprising the second, cell specific barcode sequence, thereby attaching the second barcode to the reversed transcribed nucleic acid molecules (e.g., cDNA, see e.g., **FIG. 17A**). In addition, the second barcode may be attached to the nucleic acid molecule comprising the first, analyte-specific barcode sequence (e.g., the reporter agent bound to a secreted analyte, see e.g., **FIGS. 13, 14C** and **17A**). The nucleic acid molecules including the barcoded cDNA molecules derived from the cellular mRNA molecules and the nucleic acid molecules comprising the first and the second barcode sequence (e.g., those derived from the reporter agent bound to the secreted analyte) may be processed by one or more nucleic acid reactions (either in partition or in bulk) as described elsewhere herein, with one analytical workflow embodiment outlined in **FIG. 17B**.

The present disclosure provides methods that comprise use of macromolecules (e.g., polymers) capable of forming a matrix such as a polymer and/or crosslinked matrix. In some cases, a polymer precursors are polymerized to form a polymer gel matrix. In some embodiments, the polymer gel may be a hydrogel thus forming a hydrogel matrix. The polymer molecules used to form the hydrogel matrix may comprise a plurality of capture agents. The plurality of capture agents may be coupled to or linked to (e.g., covalently or non-covalently bound or linked to the backbone of the polymer. See, e.g., U.S. Pat. Pub. 2019/0100632 (now U.S. Pat. 10,590,244), which is incorporated by reference in its entirety, for exemplary cell bead functionalization strategies. A first capture agent of the plurality of capture agents may comprise a polypeptide capable of binding a specific molecule (e.g., a secreted molecule, such as a cytokine). The capture agent comprising the polypeptide capable of binding the specific molecule may be an antibody or an antigen-binding fragment or derivative thereof. The specific molecule that the capture agent may be capable of binding may be an analyte of a cell (e.g., an immune cell). The analyte may be a molecule that may be secreted by the cell, e.g., upon stimulation with one or more stimulatory molecules or one or more APCs.

A plurality of cells (e.g., immune cells such as a T cell, B cell, and/or dendritic cells) may be co-partitioned (e.g., encapsulated into droplets) such that a partition (e.g., a droplet or a well) may comprise a cell of the plurality of cells. The partition may further comprise components capable of forming a polymeric or crosslinked matrix such as a hydrogel matrix inside the partition, resulting in the formation of a cell bead. The gel matrix (e.g., hydrogel matrix) and thus the cell bead may be formed using a reversible gel (e.g., hydrogel). The gel matrix may be a hydrogel matrix and the backbones of the polymer molecules forming the hydrogel matrix may comprise a plurality of capture agents comprising a polypeptide capable of binding a specific molecule (e.g., a cytokine).

The methods disclosed herein may comprise stimulating the cell (e.g., immune cell) forming the cell bead such that the cell secretes one or more analytes. The analytes secreted by the cell may be coupled (e.g., covalently or non-covalently bound or linked) to a first capture agent of a plurality of capture agents (see e.g., **1508**) attached (e.g., coupled or linked) to the backbones of the polymer molecules forming the cell bead hydrogel matrix, e.g., **1510**. See, e.g., U.S. Pat. Pub. 2019/0100632 (now U.S. Pat. 10,590,244), which is incorporated by reference in its entirety, for exemplary cell bead functionalization strategies. Stimulation of the cell to induce secretion may be performed for a certain period of time to allow the secreted analyte molecules to couple to the capture agents. A second binding agent, e.g., reporter agent, (see e.g., **1302**) may be added to the cell bead, wherein the reporter agent may comprise (i) a moiety such as a second polypeptide (e.g., an antibody) capable of binding an analyte molecule (e.g., a secreted cytokine) that is bound to the capture agent; and (ii) a nucleic acid molecule comprising a first, analyte specific barcode sequence, thereby tagging the analyte molecules (e.g., a cytokine) bound to the capture agent. In some cases, any reporter agent molecules that are not bound to an analyte (e.g., a secreted cytokine) may be removed from the cell beads (e.g., by performing a washing step to remove unbound secondary binding agent molecules).

Referring to operation **1402,** immune cells (e.g., T cells) or other cell types of interest are incubated with a suitable concentration of antigens (or other stimulatory molecules) to induce secretion of cytokines or other analytes. Any suitable concentration of stimulatory molecules (e.g., antigens) sufficient to induce cytokine secretion from the cells (e.g., T cells) may be used herein. In some cases, a stimulatory or co-stimulatory molecule (e.g., an antigen) is coupled to a major histocompatibility complex (MHC) molecule. In some cases, the MHC molecule is an MHC multimer (e.g., a monomer, dimer, trimer, tetramer, pentamer, etc.) resulting in a multimeric (e.g., a monomeric, dimeric, trimeric, tetrameric, etc.) MHC-antigen complex (e.g., MHC-peptide complex if the antigen is a peptide). The MHC multimer may be linked to a cell or a polymer. The cell may be an antigen-presenting cell (APC). The polymer may be a dextran polymer (e.g., a dextramer). A dextramer or an APC may comprise a plurality of MHC complexes. The dextramer bound MHC complexes may comprise one or more stimulatory molecules such as antigen peptides, thereby forming MHC-peptide complexes. Thus, MHC molecules (e.g., MHC multimers) and/or APCs may be used to present stimulatory and/or co-stimulatory molecules (e.g., stimulatory peptides via MHC-peptide complexes) to the cell (e.g., an immune cell) to induce secretion of the one or more analytes. Moreover, cell or other non-cell constructs may be used to present stimulatory molecules to a cell to induce secretion of one or more analytes. In some cases, an antigen-presenting cell (APC) may comprise a plurality of MHC molecules (e.g., MHC multimers), and thus an APC may be used to induce analyte secretion of a cell (e.g., an immune cell). A co-stimulator molecule as described herein may be an antibody (e.g., an anti-CD3 or an anti-CD28 antibody) or a cytokine (e.g., an interleukin). An APC or MHC molecule, for example, may comprise a plurality of co-stimulatory molecules and thus may be used to induce analyte secretion from a cell. In some cases, the stimulator or co-stimulatory molecules, the MHC molecule, the APC, and/or the MHC molecules as described herein may be barcoded. In some cases, methods disclosed herein comprise antigens being part of an antigen-MHC complex (e.g., an antigen-MHC tetramer) comprising the antigen (e.g., a peptide or polypeptide) and an MHC molecule (e.g., an MHC multimer such as a tetramer). The MHC molecule may comprise a nucleic acid molecule comprising a third, peptide-specific barcode sequence. The third barcode sequence, as used and described herein, may be utilized to identify the respective peptide displayed by the MHC molecule. In some embodiments, the third, peptide-specific barcode sequence is different than the first, analyte-specific barcode sequence (e.g., reporter barcode sequence) and the second cell-specific (e.g., **FIG. 8**) barcode sequence. In some instances, the nucleic acid molecules attached to the MHC molecule/multimer may comprise one or more functional sequences in addition to the peptide-specific barcode sequence. For example, the nucleic acid molecules attached to the MHC molecule/multimer may comprise one or more of a unique molecular identifier (UMI), a primer sequence or primer binding sequence (e.g., a sequencing primer sequence (or partial sequencing primer sequence) such as an R1 and/or R2 sequence), a sequence configured to attach to the flow cell of a sequencer (e.g., P5 and/or P7), or sequence complementary to a sequence on a nucleic acid barcode molecule (e.g., attached to a bead, such as those described in **FIG. 8**). Accordingly, barcoded molecules (e.g., comprising a peptide specific and cell specific barcode) generated from molecules attached to an MHC molecule/multimer (or a derivative thereof) may also comprise these functional sequences.

The herein disclosed methods and systems may further comprise partitioning cell beads into a plurality of partitions with a plurality of nucleic acid molecule comprising a cell-specific barcode sequence (see, e.g., the barcode molecules described in **FIG. 8**). In some instances, the cell specific barcodes are attached to a bead, such a gel bead. The cellular barcodes may be releasably attached to the bead as described elsewhere herein. Cell beads and cellular barcodes (e.g., attached to a bead, such as a gel bead) may be partitioned in a droplet or a well. The droplet may be an emulsion droplet and may comprise a cell bead. The emulsion droplet may be formed or generated as described elsewhere herein, e.g., by contacting two phases (e.g., a first and a second phase) that are immiscible (e.g., an aqueous phase and an oil). The hydrogel matrix forming the cell bead may be dissolved, thus releasing the analyte conjugate comprising the analyte (e.g., a cytokine), the capture agent and the reporter agent comprising the first barcode. The cell bead may be dissolved using one or more stimuli such as change in pH, temperature, or ion concentration within the partition. In some instances, the cell is lysed, releasing cellular molecules such as nucleic acid molecules (e.g., mRNAs). The cell may be lysed prior to partitioning and barcoding of analyte or lysed in the partition. The cellular mRNA molecules may be reverse transcribed using nucleic acid molecules comprising a second barcode sequence, thereby attaching the second barcode to the reversed transcribed nucleic acid molecules (and e.g., associating the analytes with the cell). Alternatively, cellular mRNA molecules may be first reverse transcribed into cDNA (e.g., using a poly-T containing primer) and the second, cell-specific barcode sequence attached (e.g., to the 5' end of an mRNA/cDNA molecule) using, e.g., a template switching reaction as described elsewhere herein. See, e.g., U.S. Pat. Pub. 2018/0105808, which is incorporated by reference in its entirety, for exemplary molecules and methods for analyzing and barcoding mRNA of single cells using template switching reactions and template switching oligonucleotides. In some instances, cellular barcodes are released from, e.g., a bead (such as a gel bead) into the partition as described elsewhere herein (e.g., using a stimulus, such as a reducing agent). Similarly, the nucleic acid molecules comprising the first, analyte-specific barcode sequence (e.g., **1304**) can be utilized to generate a molecule comprising the first analyte specific barcode and the second, cell-specific barcode (see, e.g., **FIG. 17A**). The nucleic acid molecules (e.g., **1304**) attached to an analyte specific binding agent, e.g. reporter agent (e.g., **1302**), may comprise one or more functional sequences in addition to the analyte-specific barcode sequence. For example, the nucleic acid molecules (e.g., **1304**) attached to an analyte specific binding agent (e.g., **1302**) may comprise one or more of a unique molecular identifier (UMI), a primer sequence or primer binding sequence (e.g., a sequencing primer sequence (or partial sequencing primer sequence) such as an R1 and/or R2 sequence), a sequence configured to attach to the flow cell of a sequencer (e.g., P5 and/or P7), or sequence complementary to a sequence on a nucleic acid barcode molecule (e.g., attached to a bead, such as those described in **FIG. 8**). Accordingly, barcoded molecules (e.g., comprising a analyte specific and cell specific barcode) generated from, e.g., **1304** or a derivative thereof, may also comprise these functional sequences.

Upon completion of the one or more barcoding, reverse transcription, and/or nucleic acid processing steps (e.g., depending on how many different analytes of a cell are being barcoded), the contents of the partitions (e.g., droplets or wells) may be pooled and the nucleic acid molecules subjected to further bulk processing and sequencing. Thus, the presently described methods and systems allow the association of multiple analytes to a single cell, thereby enabling the measurement, analysis, and/or characterization of a plurality of cells at the single cell level. As described herein, the plurality of cells (e.g., one or more cell populations such as populations of immune cells) may be analyzed and characterized in an efficient and simultaneous manner. The methods disclosed herein not only allow analysis of cellular molecules after lysis of the cell, but also allow analysis of molecules that may be secreted by the cell (e.g., an immune cell such as a T cell, B cell, or dendritic cell) such as cytokines, hormones, or growth factors.

Referring to operation **1414** in **FIG. 14A**, in embodiments where intracellular analytes (e.g., mRNA) are processed in parallel to secreted analytes, the cells contained in a partition (e.g., a droplet or a well) **1602** and cell beads contained in a partition (e.g., a droplet or a well) **1604** (after cell beads are optionally dissolved e.g., by dissolving the polymer matrix) are contacted with lysis reagents in order to release the contents of cells or viruses associated with the cell bead. In some cases, the lysis agents can be contacted with a cell bead suspension in bulk after cell bead formation. Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), a surfactant based lysis solution (e.g., TritonX-100, Tween 20, sodium dodecyl sulfate (SDS)) for example, as well as other commercially available lysis enzymes. Electroporation, thermal, acoustic or mechanical cellular disruption may also be used in certain cases. In some cases, the cell bead matrix can be configured to give rise to a pore size that is sufficiently small to retain nucleic acid fragments of a particular size, following cellular disruption. In other instances, the cell bead matrix may be functionalized (e.g., covalently bound) with nucleic acid molecules (e.g., containing a poly-T sequence) configured to capture released analytes (e.g., mRNA, which optionally can be processed into cDNA prior to partitioning).

Other reagents can also be contacted with the cells, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated cell beads, the cell beads may be exposed to an appropriate stimulus to release the cell beads or their contents into, e.g., a partition. For example, in some cases, a chemical stimulus may be co-partitioned along with an encapsulated cell bead to allow for the degradation of the microcapsule and release of the cell or its contents into the larger partition. In some cases, this stimulus may be the same as the stimulus described elsewhere herein for release of oligonucleotides from their respective microcapsule (e.g., bead). In alternative aspects, this may be a different and non-overlapping stimulus, in order to allow an encapsulated cell bead release its contents into a partition at a different time from the release of oligonucleotides into the same partition.

After the releasing of cellular macromolecular constituent, in operation **1416**, the cellular mRNA molecules are subject to a reverse transcription reaction with other types of reverse transcription primers such that cDNA is generated from the mRNAs. In some cases, simultaneously, a partition-specific (e.g., cell-specific, such as those described in **FIG. 8**) barcode molecule **1702** as shown in **FIG. 17A** is attached during cDNA generation. In certain embodiments, the partition-specific (e.g., cell-specific) barcode sequence **1702** is a DNA oligonucleotide. The partition-specific (e.g., cell-specific) barcode sequences **1702** may be a second barcode sequence that is different from a first barcode sequence (e.g., analyte-specific barcode) attached to or coupled to a reporter agent used to barcode an analyte that is secreted from a cell (e.g., an immune cell such as a T cell).

In certain embodiments, the partition-specific (e.g., cell-specific) barcode molecules (e.g., oligonucleotides) are releasable from the beads upon the application of a particular stimulus to the beads, as described elsewhere herein. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

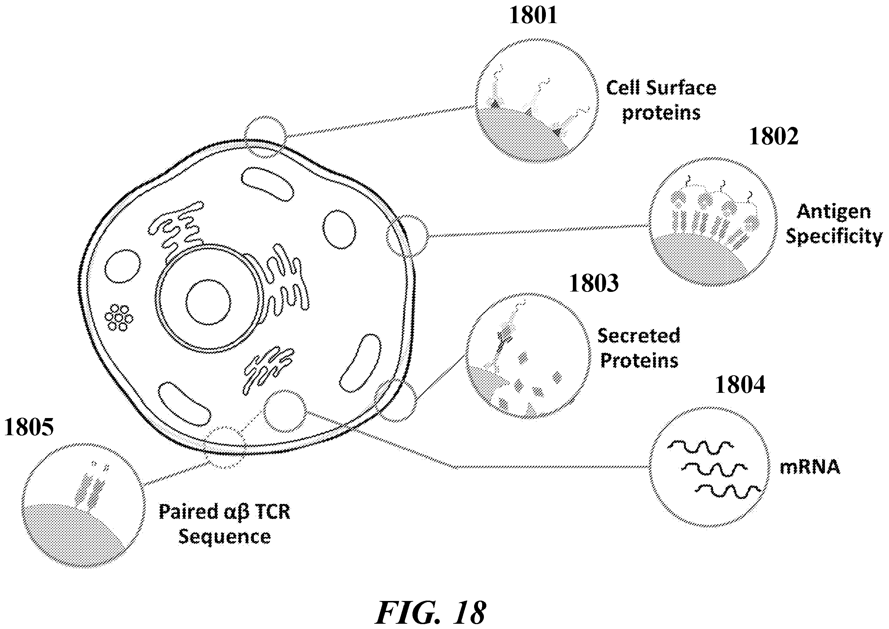

During operation **1414**, in some embodiments, barcode molecules (e.g., **1304**) attached to a second binding agent (e.g., reporter agent, an analyte specific polypeptide, such as an antibody) bound to an analyte (e.g., a cytokine) **1302** may be released (e.g., through a releasable linkage/labile bound as described elsewhere herein). Similarly, in some embodiments, barcodes attached to MHC multimers (see e.g., **1802** in **FIG. 18** and/or attached to cell surface protein specific antibodies (see e.g., **1801** in **FIG. 18** may also be released (e.g., through a releasable linkage/labile bound as described elsewhere herein). Partition-specific (e.g., dropletspecific or secondary) barcode sequences **1702** may be attached to any or all of these released barcode molecules (or derivatives thereof) in operation **1416**. These released barcodes are used as cell and/or partition-specific identifiers for RNA, DNA, proteins, and/or antigens that used to stimulate the cells in operations **1402** and **1410**. The assignment of unique barcodes specifically to an individual biological particle or groups of biological particles can attribute characteristics to individual biological particles or groups of biological particles. Unique identifiers, e.g., in the form of nucleic acid barcodes, are assigned or associated with individual biological particles or populations of biological particles, in order to tag or label the biological particle's macromolecular components (and as a result, its characteristics) with the unique identifiers. These unique identifiers, e.g., barcodes, can then be used to attribute the biological particle's components and characteristics to an individual biological particle or group of biological particles. Furthermore, as described elsewhere herein, in addition to cell and/or partition specific barcodes, unique molecular identifiers (UMIs) can also be added to cellular analytes (e.g., mRNA molecules) and reporter molecules (e.g., attached to binding agents, such as **1304** or reporter molecules attached to MHC molecules/multimers and/or antibodies, such as cell surface antibodies) to provide a unique identifier for quantitation of individual molecules.

In operation **1418**, barcoded partition contents are pooled into a bulk solution and further processed as described elsewhere herein to generate a sequencing library. Referring to **FIG. 18**, information regarding secreted analytes (e.g., cytokines), as well as other analytes, such as mRNAs, cell surface proteins, paired αβ T-cell receptor sequences, and antigen binding specificity can all be analyzed and attributed to the same cell using the cell-specific barcode (see, e.g., **FIG. 8**).

**Systems and methods for sample compartmentalization**

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion or a well. A partition may comprise one or more other partitions.

A partition may include one or more particles. A partition may include one or more types of particles. For example, a partition of the present disclosure may comprise one or more biological particles and/or macromolecular constituents thereof. A partition may comprise one or more beads. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead. A cell bead can be a biological particle and/or one or more of its macromolecular constituents encased inside of a gel or polymer matrix, such as via polymerization of a droplet containing the biological particle and precursors capable of being polymerized or gelled. Unique identifiers, such as barcodes, may be injected into the droplets previous to, subsequent to, or concurrently with droplet generation, such as via a microcapsule (e.g., bead), as described elsewhere herein. Microfluidic channel networks (e.g., on a chip) can be utilized to generate partitions as described herein. Alternative mechanisms may also be employed in the partitioning of individual biological particles, including porous membranes through which aqueous mixtures of cells are extruded into non-aqueous fluids.

The methods and systems of the present disclosure may comprise methods and systems for generating one or more partitions such as droplets. The droplets may comprise a plurality of droplets in an emulsion. In some examples, the droplets may comprise droplets in a colloid. In some cases, the emulsion may comprise a microemulsion or a nanoemulsion. In some examples, the droplets may be generated with aid of a microfluidic device and/or by subjecting a mixture of immiscible phases to agitation (e.g., in a container). In some cases, a combination of the mentioned methods may be used for droplet and/or emulsion formation.

Droplets can be formed by creating an emulsion by mixing and/or agitating immiscible phases. Mixing or agitation may comprise various agitation techniques, such as vortexing, pipetting, tube flicking, or other agitation techniques. In some cases, mixing or agitation may be performed without using a microfluidic device. In some examples, the droplets may be formed by exposing a mixture to ultrasound or sonication. Systems and methods for droplet and/or emulsion generation by agitation are described in International Application No. PCT/US2020/017785 and PCT/US2020/020486, which are entirely incorporated herein by reference for all purposes.

Microfluidic devices or platforms comprising microfluidic channel networks (e.g., on a chip) can be utilized to generate partitions such as droplets and/or emulsions as described herein. Methods and systems for generating partitions such as droplets, methods of encapsulating analyte carriers and/or analyte carriers in partitions, methods of increasing the throughput of droplet generation, and various geometries, architectures, and configurations of microfluidic devices and channels are described in U.S. Patent Publication Nos. 2019/0367997 and 2019/0064173, and International Application Nos. PCT/US2015/025197, PCT/US2020/017785 and PCT/US2020/020486, each of which is entirely incorporated herein by reference for all purposes.

In some examples, individual particles can be partitioned to discrete partitions by introducing a flowing stream of particles in an aqueous fluid into a flowing stream or reservoir of a non-aqueous fluid, such that droplets may be generated at the junction of the two streams/reservoir, such as at the junction of a microfluidic device provided elsewhere herein.

The methods of the present disclosure may comprise generating partitions and/or encapsulating particles, such as biological particles, in some cases, individual biological particles such as single cells. In some examples, reagents may be encapsulated and/or partitioned (e.g., co-partitioned with biological particles) in the partitions. Various mechanisms may be employed in the partitioning of individual particles. An example may comprise porous membranes through which aqueous mixtures of cells may be extruded into fluids (e.g., non-aqueous fluids).

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295, which is entirely incorporated herein by reference for all purposes. Emulsion systems for creating stable droplets in non-aqueous or oil continuous phases are described in, for example, U.S. Patent Application Publication No. 2010/0105112, which is entirely incorporated herein by reference for all purposes.

In the case of droplets in an emulsion, allocating individual particles to discrete partitions may in one non-limiting example be accomplished by introducing a flowing stream of particles in an aqueous fluid into a flowing stream of a non-aqueous fluid, such that droplets are generated at the junction of the two streams. Fluid properties (e.g., fluid flow rates, fluid viscosities, etc.), particle properties (e.g., volume fraction, particle size, particle concentration, etc.), microfluidic architectures (e.g.,

channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In some cases, partitions among a plurality of partitions may contain at most one biological particle (e.g., bead, DNA, cell or cellular material). In some embodiments, the various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

FIG. 1 shows an example of a microfluidic channel structure **100** for partitioning individual biological particles. The channel structure **100** can include channel segments **102**, **104**, **106** and **108** communicating at a channel junction **110**. In operation, a first aqueous fluid **112** that includes suspended biological particles (or cells) **114** may be transported along channel segment **102** into junction **110**, while a second fluid **116** that is immiscible with the aqueous fluid **112** is delivered to the junction **110** from each of channel segments **104** and **106** to create discrete droplets **118**, **120** of the first aqueous fluid **112** flowing into channel segment **108**, and flowing away from junction **110**. The channel segment **108** may be fluidically coupled to an outlet reservoir where the discrete droplets can be stored and/or harvested. A discrete droplet generated may include an individual biological particle **114** (such as droplets **118**). A discrete droplet generated may include more than one individual biological particle **114** (not shown in **FIG. 1**). A discrete droplet may contain no biological particle **114** (such as droplet **120**). Each discrete partition may maintain separation of its own contents (e.g., individual biological particle **114**) from the contents of other partitions.

The second fluid **116** can comprise an oil, such as a fluorinated oil, that includes a fluorosurfactant for stabilizing the resulting droplets, for example, inhibiting subsequent coalescence of the resulting droplets **118, 120**. Examples of particularly useful partitioning fluids and fluorosurfactants are described, for example, in U.S. Patent Application Publication No. 2010/0105112, which is entirely incorporated herein by reference for all purposes.

As will be appreciated, the channel segments of the microfluidic devices described herein may be coupled to any of a variety of different fluid sources or receiving components, including reservoirs, tubing, manifolds, or fluidic components of other systems. As will be appreciated, the microfluidic channel structure **100** may have other geometries and/or configurations. For example, a microfluidic channel structure can have more than one channel junction. For example, a microfluidic channel structure can have 2, 3, 4, or 5 channel segments each carrying particles (e.g., biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **118**, containing one or more biological particles **114**, and (2) unoccupied droplets **120**, not containing any biological particles **114**. Occupied droplets **118** may comprise singly occupied droplets (having one biological particle) and multiply occupied droplets (having more than one biological particle). As described elsewhere herein, in some cases, the majority of occupied partitions can include no more than one biological particle per occupied partition and some of the generated partitions can be unoccupied (of any biological particle). In some cases, though, some of the occupied partitions may include more than one biological particle. In some cases, the partitioning process may be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it may be desirable to minimize the creation of excessive numbers of empty partitions, such as to reduce costs and/or increase efficiency. While this minimization may be achieved by providing a sufficient number of biological particles (e.g., biological particles **114**) at the partitioning junction **110**, such as to ensure that at least one biological particle is encapsulated in a partition, the Poissonian distribution may expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the biological particles (e.g., in channel segment **102**), or other fluids directed into the partitioning junction (e.g., in channel segments **104, 106**) can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions. The above noted ranges of unoccupied partitions can be achieved while still providing any of the single occupancy rates described above. For example, in many cases, the use of the systems and methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

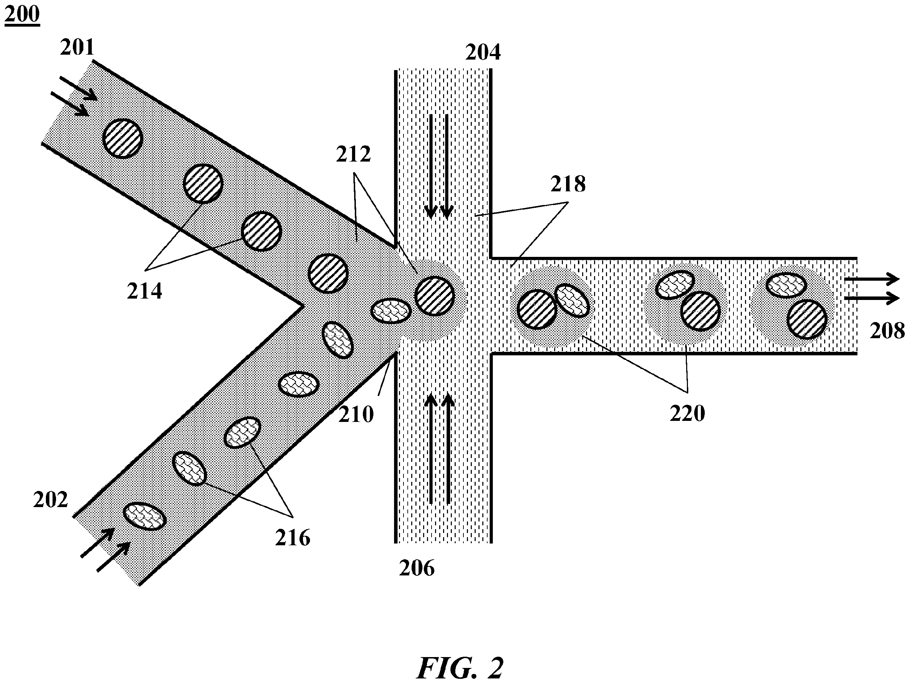

As will be appreciated, the above-described occupancy rates are also applicable to partitions that include both biological particles and additional reagents, including, but not limited to, microcapsules or beads (e.g., gel beads) carrying barcoded nucleic acid molecules (e.g., oligonucleotides) (described in relation to **FIG. 2**). The occupied partitions (e.g., at least about 10%, 20%, 30%, 40%, 50%, 60%, 70%, 80%, 90%, 95%, or 99% of the occupied partitions) can include both a microcapsule (e.g., bead) comprising barcoded nucleic acid molecules and a biological particle.

In another aspect, in addition to or as an alternative to droplet based partitioning, biological particles may be encapsulated within a microcapsule that comprises an outer shell, layer or porous matrix in which is entrained one or more individual biological particles or small groups of biological particles. The microcapsule may include other reagents. Encapsulation of biological particles may be performed by a variety of processes. Such processes may combine an aqueous fluid containing the biological particles with a polymeric precursor material that may be capable of being formed into a gel or other solid or semi-solid matrix upon application of a particular stimulus to the polymer precursor. Such stimuli can include, for example, thermal stimuli (e.g., either heating or cooling), photo-stimuli (e.g., through photocuring), chemical stimuli (e.g., through crosslinking, polymerization initiation of the precursor (e.g., through added initiators)), mechanical stimuli, or a combination thereof.

Preparation of microcapsules comprising biological particles may be performed by a variety of methods. For example, air knife droplet or aerosol generators may be used to dispense droplets of precursor fluids into gelling solutions in order to form microcapsules that include individual biological particles or small groups of biological particles. Likewise, membrane based encapsulation systems may be used to generate microcapsules comprising encapsulated biological particles as described herein. Microfluidic systems of the present disclosure, such as that shown in **FIG. 1**, may be readily used in encapsulating cells as described herein. In particular, and with reference to **FIG. 1**, the aqueous fluid **112** comprising (i) the biological particles **114** and (ii) the polymer precursor material (not shown) is flowed into channel junction **110**, where it is partitioned into droplets **118**, **120** through the flow of non-aqueous fluid **116**. In the case of encapsulation methods, non-aqueous fluid **116** may also include an initiator (not shown) to cause polymerization and/or crosslinking of the polymer precursor to form the microcapsule that includes the entrained biological particles. Examples of polymer precursor/initiator pairs include those described in U.S. Patent Application Publication No. 2014/0378345, which is entirely incorporated herein by reference for all purposes.

For example, in the case where the polymer precursor material comprises a linear polymer material, such as a linear polyacrylamide, PEG, or other linear polymeric material, the activation agent may comprise a cross-linking agent, or a chemical that activates a cross-linking agent within the formed droplets. Likewise, for polymer precursors that comprise polymerizable monomers, the activation agent may comprise a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) may be provided within the second fluid streams **116** in channel segments **104** and **106**, which can initiate the copolymerization of the acrylamide and BAC into a cross-linked polymer network, or hydrogel.

Upon contact of the second fluid stream **116** with the first fluid stream **112** at junction **110**, during formation of droplets, the TEMED may diffuse from the second fluid **116** into the aqueous fluid **112** comprising the linear polyacrylamide, which will activate the crosslinking of the polyacrylamide within the droplets **118**, **120**, resulting in the formation of gel (e.g., hydrogel) microcapsules, as solid or semi-solid beads or particles entraining the cells **114**. Although described in terms of polyacrylamide encapsulation, other 'activatable' encapsulation compositions may also be employed in the context of the methods and compositions described herein. For example, formation of alginate droplets followed by exposure to divalent metal ions (e.g., $Ca^{2+}$ ions), can be used as an encapsulation process using the described processes. Likewise, agarose droplets may also be transformed into capsules through temperature based gelling (e.g., upon cooling, etc.).

In some cases, encapsulated biological particles can be selectively releasable from the microcapsule, such as through passage of time or upon application of a particular stimulus, that degrades the microcapsule sufficiently to allow the biological particles (e.g., cell), or its other contents to be released from the microcapsule, such as into a partition (e.g., droplet). For example, in the case of the polyacrylamide polymer described above, degradation of the microcapsule may be accomplished through the introduction of an appropriate reducing agent, such as DTT or the like, to cleave disulfide bonds that cross-link the polymer matrix. See, for example, U.S.

Patent Application Publication No. 2014/0378345, which is entirely incorporated herein by reference for all purposes.

The biological particle can be subjected to other conditions sufficient to polymerize or gel the precursors. The conditions sufficient to polymerize or gel the precursors may comprise exposure to heating, cooling, electromagnetic radiation, and/or light. The conditions sufficient to polymerize or gel the precursors may comprise any conditions sufficient to polymerize or gel the precursors. Following polymerization or gelling, a polymer or gel may be formed around the biological particle. The polymer or gel may be diffusively permeable to chemical or biochemical reagents. The polymer or gel may be diffusively impermeable to macromolecular constituents of the biological particle. In this manner, the polymer or gel may act to allow the biological particle to be subjected to chemical or biochemical operations while spatially confining the macromolecular constituents to a region of the droplet defined by the polymer or gel. The polymer or gel may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

The polymer or gel may be functionalized to bind to targeted analytes, such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical properties of the bead. For instance, the polymer or gel may be of a similar size to the bead. The polymer or gel may have a mechanical strength (e.g. tensile strength) similar to that of the bead. The polymer or gel may be of a lower density than an oil. The polymer or gel may be of a density that is roughly similar to that of a buffer. The polymer or gel may have a tunable pore size. The pore size may be chosen to, for instance, retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The polymer may comprise poly(acrylamide-co-acrylic acid) crosslinked with disulfide linkages. The preparation of the polymer may comprise a two-step reaction. In the first activation step, poly(acrylamide-co-acrylic acid) may be exposed to an acylating agent to convert carboxylic acids to esters. For instance, the poly(acrylamide-co-acrylic acid) may be exposed to 4-(4,6-dimethoxy-1,3,5-triazin-2-yl)-4-methylmorpholinium chloride (DMTMM). The polyacrylamide-co-acrylic acid may be exposed to other salts of 4-(4,6-dimethoxy-1,3,5-triazin-2-yl)-4-methylmorpholinium. In the second cross-linking step, the ester formed in the first step may be exposed to a disulfide crosslinking agent. For instance, the ester may be exposed to cystamine (2,2'-dithio-bis(ethylamine)). Following the two steps, the biological particle may be surrounded by polyacrylamide strands linked together by disulfide bridges. In this manner, the biological particle may be encased inside of or comprise a gel or matrix (e.g., polymer matrix) to form a "cell bead." A cell bead can contain biological particles (e.g., a cell) or macromolecular constituents (e.g., RNA, DNA, proteins, etc.) of biological particles. A cell bead may include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing biological particles and cell beads (and/or droplets or other partitions) containing macromolecular constituents of biological particles.

Encapsulated biological particles can provide certain potential advantages of being more storable and more portable than droplet-based partitioned biological particles. Furthermore, in some cases, it may be desirable to allow biological particles to incubate for a select period of time before analysis, such as in order to characterize changes in such biological particles over time, either in the presence or absence of different stimuli. In such cases, encapsulation may allow for longer incubation than partitioning in emulsion droplets, although in some cases, droplet partitioned biological particles may also be incubated for different periods of time, e.g., at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of biological particles may constitute the partitioning of the biological particles into which other reagents are co-partitioned. Alternatively or in addition, encapsulated biological particles may be readily deposited into other partitions (e.g., droplets) as described above.

**Samples and Cell Processing**

A sample may be derived from any useful source including any subject, such as a human subject. A sample may comprise material (e.g., one or more cells) from one or more different sources, such as one or more different subjects. Multiple samples, such as multiple samples from a single subject (e.g., multiple samples obtained in the same or different manners from the same or different bodily locations, and/or obtained at the same or different times (e.g., seconds, minutes, hours, days, weeks, months, or years apart)), or multiple samples from different subjects, may be obtained for analysis as described herein. For example, a first sample may be obtained from a subject at a first time and a second sample may be obtained from the subject at a second time later than the first time. The first time may be before a subject undergoes a treatment regimen or procedure (e.g., to address a disease or condition), and the second time may be during or after the subject undergoes the treatment regimen or procedure. In another example, a first sample may be obtained from a first bodily location or system of a subject (e.g., using a first collection technique) and a second sample may be obtained from a second bodily location or system of the subject (e.g., using a second collection technique), which second bodily location or system may be different than the first bodily location or system. In another example, multiple samples may be obtained from a subject at a same time from the same or different bodily locations. Different samples, such as different samples collected from different bodily locations of a same subject, at different times, from multiple different subjects, and/or using different collection techniques, may undergo the same or different processing (e.g., as described herein). For example, a first sample may undergo a first processing protocol and a second sample may undergo a second processing protocol.

A sample may be a biological sample, such as a cell sample (e.g., as described herein). A sample may include one or more analyte carriers, such as one or more cells and/or cellular constituents, such as one or more cell nuclei. For example, a sample may comprise a plurality of cells and/or cellular constituents. Components (e.g., cells or cellular constituents, such as cell nuclei) of a sample may be of a single type or a plurality of different types. For example, cells of a sample may include one or more different types of blood cells.

A biological sample may include a plurality of cells having different dimensions and features. In some cases, processing of the biological sample, such as cell separation and sorting (e.g., as described herein), may affect the distribution of dimensions and cellular features included in the sample by depleting cells having certain features and dimensions and/or isolating cells having certain features and dimensions.

A sample may undergo one or more processes in preparation for analysis (e.g., as described herein), including, but not limited to, filtration, selective precipitation, purification, centrifugation, permeabilization, isolation, agitation, heating, and/or other processes. For example, a sample may be filtered to remove a contaminant or other materials. In an example, a filtration process may comprise the use of microfluidics (e.g., to separate analyte carriers of different sizes, types, charges, or other features).

In an example, a sample comprising one or more cells may be processed to separate the one or more cells from other materials in the sample (e.g., using centrifugation and/or another process). In some cases, cells and/or cellular constituents of a sample may be processed to separate and/or sort groups of cells and/or cellular constituents, such as to separate and/or sort cells and/or cellular constituents of different types. Examples of cell separation include, but are not limited to, separation of white blood cells or immune cells from other blood cells and components, separation of circulating tumor cells from blood, and separation of bacteria from bodily cells and/or environmental materials. A separation process may comprise a positive selection process (e.g., targeting of a cell type of interest for retention for subsequent downstream analysis, such as by use of a monoclonal antibody that targets a surface marker of the cell type of interest), a negative selection process (e.g., removal of one or more cell types and retention of one or more other cell types of interest), and/or a depletion process (e.g., removal of a single cell type from a sample, such as removal of red blood cells from peripheral blood mononuclear cells).

Separation of one or more different types of cells may comprise, for example, centrifugation, filtration, microfluidic-based sorting, flow cytometry, fluorescence-activated cell sorting (FACS), magnetic-activated cell sorting (MACS), buoyancy-activated cell sorting (BACS), or any other useful method. For example, a flow cytometry method may be used to detect cells and/or cellular constituents based on a parameter such as a size, morphology, or protein expression. Flow cytometry-based cell sorting may comprise injecting a sample into a sheath fluid that conveys the cells and/or cellular constituents of the sample into a measurement region one at a time. In the measurement region, a light source such as a laser may interrogate the cells and/or cellular constituents and scattered light and/or fluorescence may be detected and converted into digital signals. A nozzle system (e.g., a vibrating nozzle system) may be used to generate droplets (e.g., aqueous droplets) comprising individual cells and/or cellular constituents. Droplets including cells and/or cellular constituents of interest (e.g., as determined via optical detection) may be labeled with an electric charge (e.g., using an electrical charging ring), which charge may be used to separate such droplets from droplets including other cells and/or cellular constituents. For example, FACS may comprise labeling cells and/or cellular constituents with fluorescent markers (e.g., using internal and/or external biomarkers). Cells and/or cellular constituents may then be measured and identified one by one and sorted based on the emitted fluorescence of the marker or absence thereof. MACS may use micro- or nano-scale magnetic particles to bind to cells and/or cellular constituents (e.g., via an antibody interaction with cell surface markers) to facilitate magnetic isolation of cells and/or cellular constituents of interest from other components of a sample (e.g., using a column-based analysis). BACS may use microbubbles (e.g., glass microbubbles) labeled with antibodies to target cells of interest. Cells and/or cellular components coupled to microbubbles may float to a surface of a solution, thereby separating target cells and/or cellular components from other components of a sample. Cell separation techniques may be used to enrich for populations of cells of interest (e.g., prior to partitioning, as described herein). For example, a sample comprising a plurality of cells including a plurality of cells of a given type may be subjected to a positive separation process. The plurality of cells of the given type may be labeled with a fluorescent marker (e.g., based on an expressed cell surface marker or another marker) and subjected to a FACS process to separate these cells from other cells of the plurality of cells. The selected cells may then be subjected to subsequent partition-based analysis (e.g., as described herein) or other downstream analysis. The fluorescent marker may be removed prior to such analysis or may be retained. The fluorescent marker may comprise an identifying feature, such as a nucleic acid barcode sequence and/or unique molecular identifier.

In another example, a first sample comprising a first plurality of cells including a first plurality of cells of a given type (e.g., immune cells expressing a particular marker or combination of markers) and a second sample comprising a second plurality of cells including a second plurality of cells of the given type may be subjected to a positive separation process. The first and second samples may be collected from the same or different subjects, at the same or different types, from the same or different bodily locations or systems, using the same or different collection techniques. For example, the first sample may be from a first subject and the second sample may be from a second subject different than the first subject. The first plurality of cells of the first sample may be provided a first plurality of fluorescent markers configured to label the first plurality of cells of the given type. The second plurality of cells of the second sample may be provided a second plurality of fluorescent markers configured to label the second plurality of cells of the given type. The first plurality of fluorescent markers may include a first identifying feature, such as a first barcode, while the second plurality of fluorescent markers may include a second identifying feature, such as a second barcode, that is different than the first identifying feature. The first plurality of fluorescent markers and the second plurality of fluorescent markers may fluoresce at the same intensities and over the same range of wavelengths upon excitation with a same excitation source (e.g., light source, such as a laser). The first and second samples may then be combined and subjected to a FACS process to separate cells of the given type from other cells based on the first plurality of fluorescent markers labeling the first plurality of cells of the given type and the second plurality of fluorescent markers labeling the second plurality of cells of the given type. Alternatively, the first and second samples may undergo separate FACS processes and the positively selected cells of the given type from the first sample and the positively selected cells of the given type from the second sample may then be combined for subsequent analysis. The encoded identifying features of the different fluorescent markers may be used to identify cells originating from the first sample and cells originating from the second sample. For example, the first and second identifying features may be configured to interact (e.g., in partitions, as described herein) with nucleic acid barcode molecules (e.g., as described herein) to generate barcoded nucleic acid products detectable using, e.g., nucleic acid sequencing.

**Multiplexing methods**

The present disclosure provides methods and systems for multiplexing, and otherwise increasing throughput of samples for analysis. For example, a single or integrated process workflow may permit the processing, identification, and/or analysis of more or multiple analytes, more or multiple types of analytes, and/or more or multiple types of analyte characterizations. For example, in the methods and systems described herein, one or more labelling agents capable of binding to or otherwise coupling to one or more cells or cell features may be used to characterize cells and/or cell features. In some instances, cell features include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have a first reporter oligonucleotide coupled thereto, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat.

Pub. 20190367969, each of which is herein entirely incorporated by reference for all purposes.

In a particular example, a library of potential cell feature labelling agents may be provided, where the respective cell feature labelling agents are associated with nucleic acid reporter molecules, such that a different reporter oligonucleotide sequence is associated with each labelling agent capable of binding to a specific cell feature. In other aspects, different members of the library may be characterized by the presence of a different oligonucleotide sequence label. For example, an antibody capable of binding to a first protein may have associated with it a first reporter oligonucleotide sequence, while an antibody capable of binding to a second protein may have a different reporter oligonucleotide sequence associated with it. The presence of the particular oligonucleotide sequence may be indicative of the presence of a particular antibody or cell feature which may be recognized or bound by the particular antibody.

Labelling agents capable of binding to or otherwise coupling to one or more cells may be used to characterize a cell as belonging to a particular set of cells. For example, labeling agents may be used to label a sample of cells or a group of cells. In this way, a group of cells may be labeled as different from another group of cells. In an example, a first group of cells may originate from a first sample and a second group of cells may originate from a second sample. Labelling agents may allow the first group and second group to have a different labeling agent (or reporter oligonucleotide associated with the labeling agent). This may, for example, facilitate multiplexing, where cells of the first group and cells of the second group may be labeled separately and then pooled together for downstream analysis. The downstream detection of a label may indicate analytes as belonging to a particular group.

For example, a reporter oligonucleotide may be linked to an antibody or an epitope binding fragment thereof, and labeling a cell may comprise subjecting the antibody-linked barcode molecule or the epitope binding fragment-linked barcode molecule to conditions suitable for binding the antibody to a molecule present on a surface of the cell. The binding affinity between the antibody or the epitope binding fragment thereof and the molecule present on the surface may be within a desired range to ensure that the antibody or the epitope binding fragment thereof remains bound to the molecule. For example, the binding affinity may be within a desired range to ensure that the antibody or the epitope binding fragment thereof remains bound to the molecule during various sample processing steps, such as partitioning and/or nucleic acid amplification or extension. A dissociation constant ($K_d$) between the antibody or an epitope binding fragment thereof and the molecule to which it binds may be less than about 100 $\mu$M, 90 $\mu$M, 80 $\mu$M, 70 $\mu$M, 60 $\mu$M, 50 $\mu$M, 40 $\mu$M, 30 $\mu$M, 20 $\mu$M, 10 $\mu$M, 9 $\mu$M, 8 $\mu$M, 7 $\mu$M, 6 $\mu$M, 5 $\mu$M, 4 $\mu$M, 3 $\mu$M, 2 $\mu$M, 1 $\mu$M, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant may be less than about 10 $\mu$M.

In another example, a reporter oligonucleotide may be coupled to a cell-penetrating peptide (CPP), and labeling cells may comprise delivering the CPP coupled reporter oligonucleotide into an analyte carrier. Labeling analyte carriers may comprise delivering the CPP conjugated oligonucleotide into a cell and/or cell bead by the cell-penetrating peptide. A CPP that can be used in the methods provided herein can comprise at least one nonfunctional cysteine residue, which may be either free or derivatized to form a disulfide link with an oligonucleotide that has been modified for such linkage. Non-limiting examples of CPPs that can be used in embodiments herein include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The CPP may be an arginine-rich peptide transporter. The CPP may be Penetratin or the Tat peptide. In another example, a reporter oligonucleotide may be coupled to a fluorophore or dye, and labeling cells may comprise subjecting the fluorophore-linked barcode molecule to conditions suitable for binding the fluorophore to the surface of the cell. In some instances, fluorophores can interact strongly with lipid bilayers and labeling cells may comprise subjecting the fluorophore-linked barcode molecule to conditions such that the fluorophore binds to or is inserted into a membrane of the cell. In some cases, the fluorophore is a water-soluble, organic fluorophore. In some instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649, which is hereby incorporated by reference in its entirety for all purposes, for a description of organic fluorophores.

A reporter oligonucleotide may be coupled to a lipophilic molecule, and labeling cells may comprise delivering the nucleic acid barcode molecule to a membrane of a cell or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and the cell or nuclear membrane may be such that the membrane retains the lipophilic molecule (e.g., and associated components, such as nucleic acid barcode molecules, thereof) during subsequent processing (e.g., partitioning, cell permeabilization, amplification, pooling, etc.). The reporter nucleotide may enter into the intracellular space and/or a cell nucleus. In one embodiment, a reporter oligonucleotide coupled to a lipophilic molecule will remain associated with and/or inserted into lipid membrane (as described herein) via the lipophilic molecule until lysis of the cell occurs, e.g., inside a partition.

A reporter oligonucleotide may be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells may be incubated with the library of labelling agents, that may be labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells) along with partition-specific barcode oligonucleotides (e.g., attached to a support, such as a bead or gel bead) as described elsewhere herein. As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. For example, the first plurality of the labeling agent and second plurality of the labeling agent may interact with different cells, cell populations or samples, allowing a particular report oligonucleotide to indicate a particular cell population (or cell or sample) and cell feature. In this way, different samples or groups can be independently processed and subsequently combined together for pooled analysis (e.g., partition-based barcoding as described elsewhere herein). See, e.g., U.S. Pat. Pub. 20190323088, which is hereby entirely incorporated by reference for all purposes.

As described elsewhere herein, libraries of labelling agents may be associated with a particular cell feature as well as be used to identify analytes as originating from a particular cell population, or sample. Cell populations may be incubated with a plurality of libraries such that a cell or cells comprise multiple labelling agents. For example, a cell may comprise coupled thereto a lipophilic labeling agent and an antibody. The lipophilic labeling agent may indicate that the cell is a member of a particular cell sample, whereas the antibody may indicate that the cell comprises a particular analyte. In this manner, the reporter oligonucleotides and labelling agents may allow multi-analyte, multiplexed analyses to be performed.

In some instances, these reporter oligonucleotides may comprise nucleic acid barcode sequences that permit identification of the labelling agent which the reporter oligonucleotide is coupled to. The use of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link® antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, which is entirely incorporated herein by reference for all purposes. Likewise, protein and peptide biotinylation techniques have been developed and are readily available. See, e.g., U.S. Pat. No. 6,265,552, which is entirely incorporated herein by reference for all purposes. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to an oligonucleotide that is complementary to a sequence of the reporter oligonucleotide, and the oligonucleotide may be allowed to hybridize to the reporter oligonucleotide.

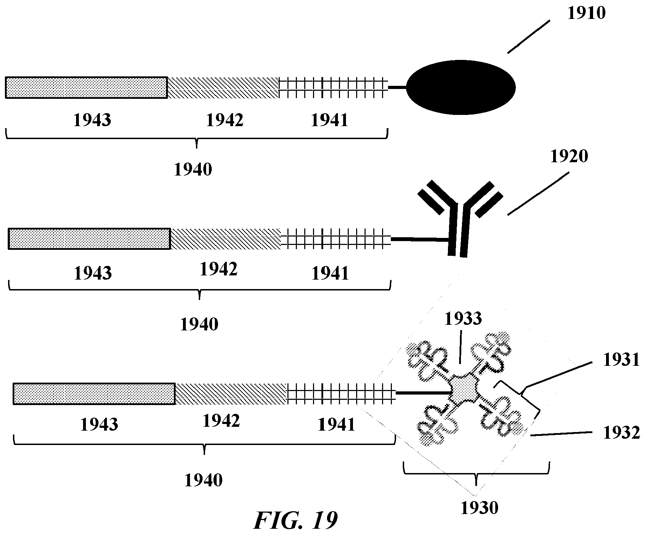

FIG. 19 describes exemplary labelling agents (1910, 1920, 1930) comprising reporter oligonucleotides (1940) attached thereto. Labelling agent 1910 (e.g., any of the labelling agents described herein) is attached (either directly, e.g., covalently attached, or indirectly) to reporter oligonucleotide 1940. Reporter oligonucleotide 1940 may comprise barcode sequence 1942 that identifies labelling agent 1910. Reporter oligonucleotide 1940 may also comprise one or more functional sequences 1943 that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to FIG. 19, in some instances, reporter oligonucleotide 1940 conjugated to a labelling agent (e.g., 1910, 1920, 1930) comprises a primer sequence 1941, a barcode sequence 1942 that identifies the labelling agent (e.g., 1910, 1920, 1930), and functional sequence 1943. Functional sequence 1943 may be configured to hybridize to a complementary sequence, such as a complementary sequence present on a nucleic acid barcode molecule 1990 (not shown), such as those described elsewhere herein. In some instances, nucleic acid barcode molecule 1990 is attached to a support (e.g., a bead, such as a gel bead), such as those described elsewhere herein. For example, nucleic acid barcode molecule 1990 may be attached to the support via a releasable linkage (e.g., comprising a labile bond), such as those described elsewhere herein. In some instances, reporter oligonucleotide 1940 comprises one or more additional functional sequences, such as those described above.

In some instances, the labelling agent 1910 is a protein or polypeptide (e.g., an antigen or prospective antigen) comprising reporter oligonucleotide 1940. Reporter oligonucleotide 1940 comprises barcode sequence 1942 that identifies polypeptide 1910 and can be used to infer the presence of an analyte, e.g., a binding partner of polypeptide 1910 (i.e., a molecule or compound to which polypeptide 1910 can bind). In some instances, the labelling agent 1910 is a lipophilic moiety (e.g., cholesterol) comprising reporter oligonucleotide 1940, where the lipophilic moiety is selected such that labelling agent 1910 integrates into a membrane of a cell or nucleus. Reporter oligonucleotide 1940 comprises barcode sequence 1942 that identifies lipophilic moiety 1910 which in some instances is used to tag cells (e.g., groups of cells, cell samples, etc.) and may be used for multiplex analyses as described elsewhere herein. In some instances, the labelling agent is an antibody 1920 (or an epitope binding fragment thereof) comprising reporter oligonucleotide 1940. Reporter oligonucleotide 1940 comprises barcode sequence 1942 that identifies antibody 1920 and can be used to infer the presence of, e.g., a target of antibody 1920 (i.e., a molecule or compound to which antibody 1920 binds). In other embodiments, labelling agent 1930 comprises an MHC molecule 1931 comprising peptide 1932 and reporter oligonucleotide 1940 that identifies peptide 1932. In some instances, the MHC molecule is coupled to a support 1933. In some instances, support 1933 may be a polypeptide, such as streptavidin, or a polysaccharide, such as dextran. In some instances, reporter oligonucleotide 1940 may be directly or indirectly coupled to MHC labelling agent 1930 in any suitable manner. For example, reporter oligonucleotide 1940 may be coupled to MHC molecule 1931, support 1933, or peptide 1932. In some embodiments, labelling agent 1930 comprises a plurality of MHC molecules, e.g., an MHC multimer, which may be coupled to a support (e.g., 1933). There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5® MHC Class I Pentamers, (ProImmune, Ltd.), MHC octamers, MHC dodecamers, MHC decorated dextran molecules (e.g., MHC Dextramer® (Immudex)), etc. For a description of exemplary labelling agents, including antibody and MHC-based labelling agents, reporter oligonucleotides, and methods of use, see, e.g., U.S. Pat. 10,550,429 and U.S. Pat. Pub. 20190367969, each of which is herein entirely incorporated by reference for all purposes.

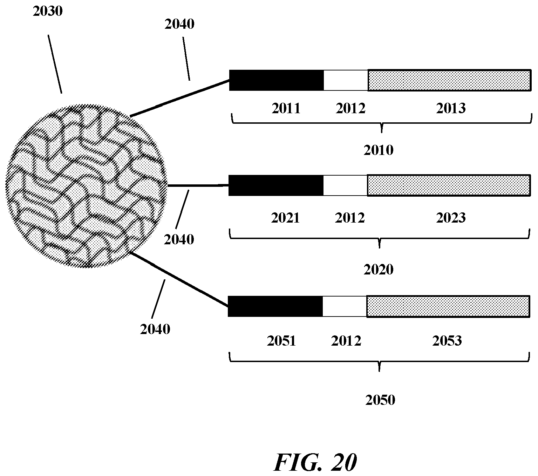

FIG. 20 illustrates another example of a barcode carrying bead. In some embodiments, analysis of multiple analytes (e.g., RNA and one or more analytes using labelling agents described herein) may comprise nucleic acid barcode molecules as generally depicted in FIG. 20. In some embodiments, nucleic acid barcode molecules

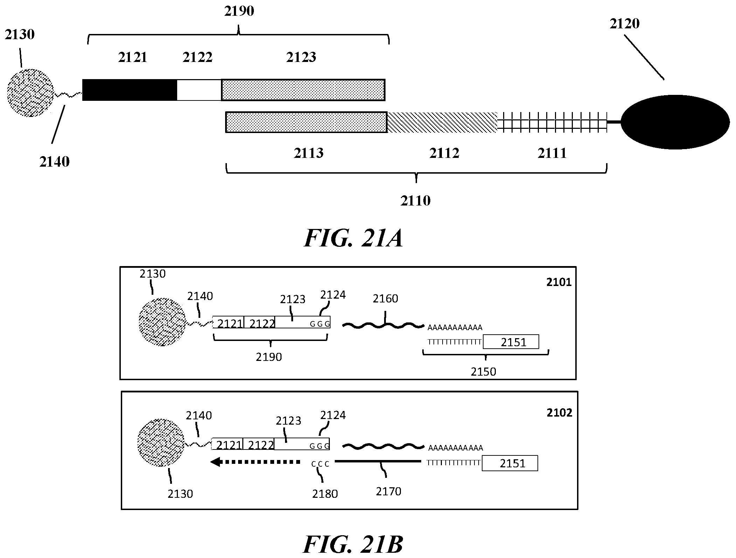

2010 and 2020 are attached to support 2030 via a releasable linkage 2040 (e.g., comprising a labile bond) as described elsewhere herein. Nucleic acid barcode molecule 2010 may comprise adapter sequence 2011, barcode sequence 2012 and adapter sequence 2013. Nucleic acid barcode molecule 2020 may comprise adapter sequence 2021, barcode sequence 2012, and adapter sequence 2023, wherein adapter sequence 2023 comprises a different sequence than adapter sequence 2013. In some instances, adapter 2011 and adapter 2021 comprise the same sequence. In some instances, adapter 2011 and adapter 2021 comprise different sequences. Although support 2030 is shown comprising nucleic acid barcode molecules 2010 and 2020, any suitable number of barcode molecules comprising common barcode sequence 2012 are contemplated herein. For example, in some embodiments, support 2030 further comprises nucleic acid barcode molecule 2050. Nucleic acid barcode molecule 2050 may comprise adapter sequence 2051, barcode sequence 2012 and adapter sequence 2053, wherein adapter sequence 2053 comprises a different sequence than adapter sequence 2013 and 2023. In some instances, nucleic acid barcode molecules (e.g., 2010, 2020, 2050) comprise one or more additional functional sequences, such as a UMI or other sequences described herein. The nucleic acid barcode molecules 2010, 2020 or 2050 may interact with analytes as described elsewhere herein, for example, as depicted in FIGS. 21A-C.

Referring to FIG. 21A, in an instance where cells are labelled with labeling agents, sequence 2123 may be complementary to an adapter sequence of a reporter oligonucleotide. Cells may be contacted with one or more reporter oligonucleotide 2110 conjugated labelling agents 2120 (e.g., polypeptide, antibody, or others described elsewhere herein). In some cases, the cells may be further processed prior to barcoding. For example, such processing steps may include one or more washing and/or cell sorting steps. In some instances, a cell that is bound to labelling agent 2120 which is conjugated to oligonucleotide 2110 and support 2130 (e.g., a bead, such as a gel bead) comprising nucleic acid barcode molecule 2190 is partitioned into a partition amongst a plurality of partitions (e.g., a droplet of a droplet emulsion or a well of a microwell array). In some instances, the partition comprises at most a single cell bound to labelling agent 2120. In some instances, reporter oligonucleotide 2110 conjugated to labelling agent 2120 (e.g., polypeptide, an antibody, pMHC molecule such as an MHC multimer, etc.) comprises a first adapter sequence 2111 (e.g., a primer sequence), a barcode sequence 2112 that identifies the labelling agent 2120 (e.g., the polypeptide, antibody, or peptide of a pMHC molecule or complex), and an adapter sequence 2113. Adapter sequence 2113 may be configured to hybridize to a complementary sequence, such as sequence 2123 present on a nucleic acid barcode molecule 2190. In some instances, oligonucleotide 2110 comprises one or more additional functional sequences, such as those described elsewhere herein.

Barcoded nucleic may be generated (e.g., via a nucleic acid reaction, such as nucleic acid extension or ligation) from the constructs described in **FIGS. 21A-C**. For example, sequence **2113** may then be hybridized to complementary sequence **2123** to generate (e.g., via a nucleic acid reaction, such as nucleic acid extension or ligation) a barcoded nucleic acid molecule comprising cell (e.g., partition specific) barcode sequence **2122** (or a reverse complement thereof) and reporter sequence **2112** (or a reverse complement thereof). Barcoded nucleic acid molecules can then be optionally processed as described elsewhere herein, e.g., to amplify the molecules and/or append sequencing platform specific sequences to the fragments. See, e.g., U.S. Pat. Pub. 2018/0105808, which is hereby entirely incorporated by reference for all purposes. Barcoded nucleic acid molecules, or derivatives generated therefrom, can then be sequenced on a suitable sequencing platform.

In some instances, analysis of multiple analytes (e.g., nucleic acids and one or more analytes using labelling agents described herein) may be performed. For example, the workflow may comprise a workflow as generally depicted in any of **FIGS. 21A-C**, or a combination of workflows for an individual analyte, as described elsewhere herein. For example, by using a combination of the workflows as generally depicted in **FIGS. 21A-C**, multiple analytes can be analyzed.

In some instances, analysis of an analyte (e.g. a nucleic acid, a polypeptide, a carbohydrate, a lipid, etc.) comprises a workflow as generally depicted in **FIG. 21A**. A nucleic acid barcode molecule **2190** may be co-partitioned with the one or more analytes. In some instances, nucleic acid barcode molecule **2190** is attached to a support **2130** (e.g., a bead, such as a gel bead), such as those described elsewhere herein. For example, nucleic acid barcode molecule **2190** may be attached to support **2130** via a releasable linkage **2140** (e.g., comprising a labile bond), such as those described elsewhere herein. Nucleic acid barcode molecule **2190** may comprise a barcode sequence **2121** and optionally comprise other additional sequences, for example, a UMI sequence **2122** (or other functional sequences described elsewhere herein). The nucleic acid barcode molecule **2190** may comprise a sequence **2123** that may be complementary to another nucleic acid sequence, such that it may hybridize to a particular sequence.

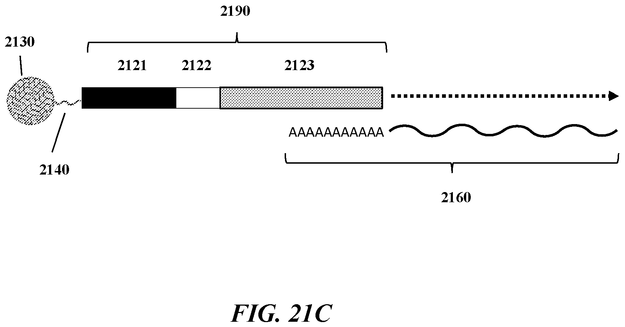

For example, sequence **2123** may comprise a poly-T sequence and may be used to hybridize to mRNA. Referring to **FIG. 21C,** in some embodiments, nucleic acid barcode molecule **2190** comprises sequence **2123** complementary to a sequence of RNA molecule **2160** from a cell. In some instances, sequence **2123** comprises a sequence specific for an RNA molecule. Sequence **2123** may comprise a known or targeted sequence or a random sequence. In some instances, a nucleic acid extension reaction may be performed, thereby generating a barcoded nucleic acid product comprising sequence **2123,** the barcode sequence **2121,** UMI sequence **2122,** any other functional sequence, and a sequence corresponding to the RNA molecule **2160.**

In another example, sequence **2123** may be complementary to an overhang sequence or an adapter sequence that has been appended to an analyte. For example, referring to **FIG. 21B,** in some embodiments, primer **2150** comprises a sequence complementary to a sequence of nucleic acid molecule **2160** (such as an RNA encoding for a BCR sequence) from an analyte carrier. In some instances, primer **2150** comprises one or more sequences **2151** that are not complementary to RNA molecule **2160.** Sequence **2151** may be a functional sequence as described elsewhere herein, for example, an adapter sequence, a sequencing primer sequence, or a sequence the facilitates coupling to a flow cell of a sequencer. In some instances, primer **2150** comprises a poly-T sequence. In some instances, primer **2150** comprises a sequence complementary to a target sequence in an RNA molecule. In some instances, primer **2150** comprises a sequence complementary to a region of an immune molecule, such as the constant region of a TCR or BCR sequence. Primer **2150** is hybridized to nucleic acid molecule **2160** and complementary molecule **2170** is generated. For example, complementary molecule **2170** may be cDNA generated in a reverse transcription reaction. In some instances, an additional sequence may be appended to complementary molecule **2170.** For example, the reverse transcriptase enzyme may be selected such that several non-templated bases **2180** (e.g., a poly-C sequence) are appended to the cDNA. In another example, a terminal transferase may also be used to append the additional sequence. Nucleic acid barcode molecule **2190** comprises a sequence **2124** complementary to the non-templated bases, and the reverse transcriptase performs a template switching reaction onto nucleic acid barcode molecule **2190** to generate a barcoded nucleic acid molecule comprising cell (e.g., partition specific) barcode sequence **2122** (or a reverse complement thereof) and a sequence of complementary molecule **2170** (or a portion thereof). In some instances, sequence **2123** comprises a sequence complementary to a region of an immune molecule, such as the constant region of a TCR or BCR sequence. Sequence **2123** is hybridized to nucleic acid molecule **2160** and a complementary molecule **2170** is generated. For example, complementary molecule **2170** may be generated in a reverse transcription reaction generating a barcoded nucleic acid molecule comprising cell (e.g., partition specific) barcode sequence **2122** (or a reverse complement thereof) and a sequence of complementary molecule **2170** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969, each of which applications is herein entirely incorporated by reference for all purposes.

## Beads

A partition may comprise one or more unique identifiers, such as barcodes. Barcodes may be previously, subsequently or concurrently delivered to the partitions that hold the compartmentalized or partitioned biological particle. For example, barcodes may be injected into droplets previous to, subsequent to, or concurrently with droplet generation. The delivery of the barcodes to a particular partition allows for the later attribution of the characteristics of the individual biological particle to the particular partition. Barcodes may be delivered, for example on a nucleic acid molecule (e.g., an oligonucleotide), to a partition via any suitable mechanism. Barcoded nucleic acid molecules can be delivered to a partition via a microcapsule. A microcapsule, in some instances, can comprise a bead. Beads are described in further detail below.

In some cases, barcoded nucleic acid molecules can be initially associated with the microcapsule and then released from the microcapsule. Release of the barcoded nucleic acid molecules can be passive (e.g., by diffusion out of the microcapsule). In addition or alternatively, release from the microcapsule can be upon application of a stimulus which allows the barcoded nucleic acid nucleic acid molecules to dissociate or to be released from the microcapsule. Such stimulus may disrupt the microcapsule, an interaction that couples the barcoded nucleic acid molecules to or within the microcapsule, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof. Methods and systems for partitioning barcode carrying beads into droplets are provided in US. Patent Publication Nos. 2019/0367997 and 2019/0064173, and International Application Nos. PCT/US2015/025197, PCT/US2020/017785 and PCT/US2020/020486, each of which is herein entirely incorporated by reference for all purposes.

FIG. 2 shows an example of a microfluidic channel structure 200 for delivering barcode carrying beads to droplets. The channel structure 200 can include channel segments 201, 202, 204, 206 and 208 communicating at a channel junction 210. In operation, the channel segment 201 may transport an aqueous fluid 212 that includes a plurality of beads 214 (e.g., with nucleic acid molecules, oligonucleotides, molecular tags) along the channel segment 201 into junction 210. The plurality of beads 214 may be sourced from a suspension of beads. For example, the channel segment 201 may be connected to a reservoir comprising an aqueous suspension of beads 214. The channel segment 202 may transport the aqueous fluid 212 that includes a plurality of biological particles 216 along the channel segment 202 into junction 210. The plurality of biological particles 216 may be sourced from a suspension of biological particles. For example, the channel segment 202 may be connected to a reservoir comprising an aqueous suspension of biological particles 216. In some instances, the aqueous fluid 212 in either the first channel segment 201 or the second channel segment 202, or in both segments, can include one or more reagents, as further described below. A second fluid 218 that is immiscible with the aqueous fluid 212 (e.g., oil) can be delivered to the junction 210 from each of channel segments 204 and 206. Upon meeting of the aqueous fluid 212 from each of channel segments 201 and 202 and the second fluid 218 from each of channel segments 204 and 206 at the channel junction 210, the aqueous fluid 212 can be partitioned as discrete droplets 220 in the second fluid 218 and flow away from the junction 210 along channel segment 208. The channel segment 208 may deliver the discrete droplets to an outlet reservoir fluidly coupled to the channel segment 208, where they may be harvested.

As an alternative, the channel segments 201 and 202 may meet at another junction upstream of the junction 210. At such junction, beads and biological particles may form a mixture that is directed along another channel to the junction 210 to yield droplets 220. The mixture may provide the beads and biological particles in an alternating fashion, such that, for example, a droplet comprises a single bead and a single biological particle.

In some examples, beads, biological particles, and droplets may flow along channels (e.g., the channels of a microfluidic device). Beads, biological particles and droplets may flow along channels at substantially regular flow profiles (e.g., at regular flow rates). Such regular flow profiles may permit a droplet to include a single bead and a single biological particle. Such regular flow profiles may permit the droplets to have an occupancy (e.g., droplets having beads and biological particles) greater than 5%, 10%, 20%, 30%, 40%, 50%, 60%, 70%, 80%, 90%, or 95%. Such regular flow profiles and devices that may be used to provide such regular flow profiles are provided in, for example, U.S. Patent Publication No. 2015/0292988, which is entirely incorporated herein by reference.

The second fluid 218 can comprise an oil, such as a fluorinated oil, that includes a fluorosurfactant for stabilizing the resulting droplets, for example, inhibiting subsequent coalescence of the resulting droplets 220.

A discrete droplet that is generated may include an individual biological particle 216. A discrete droplet that is generated may include a barcode or other reagent carrying bead 214. A discrete droplet generated may include both an individual biological particle and a barcode carrying bead, such as droplets 220. In some instances, a discrete droplet may include more than one individual biological particle or no biological particle. In some instances, a discrete droplet may include more than one bead or no bead. A discrete droplet may be unoccupied (e.g., no beads, no biological particles).

Beneficially, a discrete droplet partitioning a biological particle and a barcode carrying bead may effectively allow the attribution of the barcode to macromolecular constituents of the biological particle within the partition. The contents of a partition may remain discrete from the contents of other partitions.

As will be appreciated, the channel segments described herein may be coupled to any of a variety of different fluid sources or receiving components, including reservoirs, tubing, manifolds, or fluidic components of other systems. As will be appreciated, the microfluidic channel structure **200** may have other geometries. For example, a microfluidic channel structure can have more than one channel junctions. For example, a microfluidic channel structure can have 2, 3, 4, or 5 channel segments each carrying beads that meet at a channel junction. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

A bead may be porous, non-porous, solid, semi-solid, semi-fluidic, fluidic, and/or a combination thereof. In some instances, a bead may be dissolvable, disruptable, and/or degradable. In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in some cases less than 15%, less than 10%, less than 5%, or less.

A bead may comprise natural and/or synthetic materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (e.g., co-polymers) thereof. Beads may also be formed from materials other than polymers, including lipids, micelles, ceramics, glass-ceramics, material composites, metals, other inorganic materials, and others.

In some instances, the bead may contain molecular precursors (e.g., monomers or polymers), which may form a polymer network via polymerization of the molecular precursors. In some cases, a precursor may be an already polymerized species capable of undergoing further polymerization via, for example, a chemical cross-linkage. In some cases, a precursor can comprise one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers. In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carbon-carbon bonds, thioether bonds, or carbon-heteroatom bonds.

Cross-linking may be permanent or reversible, depending upon the particular cross-linker used. Reversible cross-linking may allow for the polymer to linearize or dissociate under appropriate conditions. In some cases, reversible cross-linking may also allow for reversible attachment of a material bound to the surface of a bead. In some cases, a cross-linker may form disulfide linkages. In some cases, the chemical cross-linker forming disulfide linkages may be cystamine or a modified cystamine.

In some cases, disulfide linkages can be formed between molecular precursor units (e.g., monomers, oligomers, or linear polymers) or precursors incorporated into a bead and nucleic acid molecules (e.g., oligonucleotides). Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible cross-linkers). The disulfide linkages may permit the bead to be degraded (or dissolved) upon exposure of the bead to a reducing agent.

In some cases, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

In some cases, a bead may comprise an acrydite moiety, which in certain aspects may be used to attach one or more nucleic acid molecules (e.g., barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment can be reversible, such that when the disulfide bond is broken (e.g., in the presence of a reducing agent), the attached species is released from the bead. In other cases, an acrydite moiety can comprise a reactive hydroxyl group that may be used for attachment.

Functionalization of beads for attachment of nucleic acid molecules (e.g., oligonucleotides) may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule (e.g., oligonucleotide), which may include a priming sequence (e.g., a primer for amplifying target nucleic acids, random primer, primer sequence for messenger RNA) and/or one or more barcode sequences. The one more barcode sequences may include sequences that are the same for all nucleic acid molecules coupled to a given bead and/or sequences that are different across all nucleic acid molecules coupled to the given bead. The nucleic acid molecule may be incorporated into the bead.

In some cases, the nucleic acid molecule can comprise a functional sequence, for example, for attachment to a sequencing flow cell, such as, for example, a P5 sequence for Illumina® sequencing. In some cases, the nucleic acid molecule or derivative thereof (e.g., oligonucleotide or polynucleotide generated from the nucleic acid molecule) can comprise another functional sequence, such as, for example, a P7 sequence for attachment to a sequencing flow cell for Illumina sequencing. In some cases, the nucleic acid molecule can comprise a barcode sequence. In some cases, the primer can further comprise a unique molecular identifier (UMI). In some cases, the primer can comprise an R1 primer sequence for Illumina sequencing. In some cases, the primer can comprise an R2 primer sequence for Illumina sequencing. Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609, each of which is entirely incorporated herein by reference.

FIG. 8 illustrates an example of a barcode carrying bead. A nucleic acid molecule 802, such as an oligonucleotide, can be coupled to a bead 804 by a releasable linkage 806, such as, for example, a disulfide linker. The same bead 804 may be coupled (e.g., via releasable linkage) to one or more other nucleic acid molecules 818, 820. The nucleic acid molecule 802 may be or comprise a barcode. As noted elsewhere herein, the structure of the barcode may comprise a number of sequence elements. The nucleic acid molecule **802** may comprise a functional sequence **808** that may be used in subsequent processing. For example, the functional sequence **808** may include one or more of a sequencer specific flow cell attachment sequence (e.g., a P5 sequence for Illumina® sequencing systems) and a sequencing primer sequence (e.g., a R1 primer for Illumina® sequencing systems). The nucleic acid molecule **802** may comprise a barcode sequence **810** for use in barcoding the sample (e.g., DNA, RNA, protein, etc.). In some cases, the barcode sequence **810** can be bead-specific such that the barcode sequence **810** is common to all nucleic acid molecules (e.g., including nucleic acid molecule **802**) coupled to the same bead **804**. Alternatively or in addition, the barcode sequence **810** can be partition-specific such that the barcode sequence **810** is common to all nucleic acid molecules coupled to one or more beads that are partitioned into the same partition. The nucleic acid molecule **802** may comprise a specific priming sequence **812,** such as an mRNA specific priming sequence (e.g., poly-T sequence), a targeted priming sequence, and/or a random priming sequence. The nucleic acid molecule **802** may comprise an anchoring sequence **814** to ensure that the specific priming sequence **812** hybridizes at the sequence end (e.g., of the mRNA). For example, the anchoring sequence **814** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule **802** may comprise a unique molecular identifying sequence **816** (e.g., unique molecular identifier (UMI)). In some cases, the unique molecular identifying sequence **816** may comprise from about 5 to about 8 nucleotides. Alternatively, the unique molecular identifying sequence **816** may compress less than about 5 or more than about 8 nucleotides. The unique molecular identifying sequence **816** may be a unique sequence that varies across individual nucleic acid molecules (e.g., **802, 818, 820,** etc.) coupled to a single bead (e.g., bead **804**). In some cases, the unique molecular identifying sequence **816** may be a random sequence (e.g., such as a random N-mer sequence). For example, the UMI may provide a unique identifier of the starting mRNA molecule that was captured, in order to allow quantitation of the number of original expressed RNA. As will be appreciated, although **FIG. 8** shows three nucleic acid molecules **802, 818, 820** coupled to the surface of the bead **804,** an individual bead may be coupled to any number of individual nucleic acid molecules, for example, from one to tens to hundreds of thousands or even millions of individual nucleic acid molecules. The respective barcodes for the individual nucleic acid molecules can comprise both common sequence segments or relatively common sequence segments (e.g., **808, 810, 812,** etc.) and variable or unique sequence segments (e.g., **816)** between different individual nucleic acid molecules coupled to the same bead.

In operation, a biological particle (e.g., cell, DNA, RNA, etc.) can be co-partitioned along with a barcode bearing bead **804**. The barcoded nucleic acid molecules **802, 818, 820** can be released from the bead **804** in the partition. By way of example, in the context of analyzing sample RNA, the poly-T segment (e.g., **812)** of one of the released nucleic acid molecules (e.g., **802)** can hybridize to the poly-A tail of a mRNA molecule. Reverse transcription may result in a cDNA transcript of the mRNA, but which transcript includes each of the sequence segments **808, 810, 816** of the nucleic acid molecule **802**. Because the nucleic acid molecule **802** comprises an anchoring sequence **814,** it will more likely hybridize to and prime reverse transcription at the sequence end of the poly-A tail of the mRNA. Within any given partition, all of the cDNA transcripts of the individual mRNA molecules may include a common barcode sequence segment **810**. However, the transcripts made from the different mRNA molecules within a given partition may vary at the unique molecular identifying sequence **812** segment (e.g., UMI segment). Beneficially, even following any subsequent amplification of the contents of a given partition, the number of different UMIs can be indicative of the quantity of mRNA originating from a given partition, and thus from the biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcoded oligonucleotides into the partition, in some cases, the nucleic acid molecules bound to the bead (e.g., gel bead) may be used to hybridize and capture the mRNA on the solid phase of the bead, for example, in order to facilitate the separation of the RNA from other cell contents. In such cases, further processing may be performed, in the partitions or outside the partitions (e.g., in bulk). For instance, the RNA molecules on the beads may be subjected to reverse transcription or other nucleic acid processing, additional adapter sequences may be added to the barcoded nucleic acid molecules, or other nucleic acid reactions (e.g., amplification, nucleic acid extension) may be performed. The beads or products thereof (e.g., barcoded nucleic acid molecules) may be collected from the partitions, and/or pooled together and subsequently subjected to clean up and further characterization (e.g., sequencing).

The operations described herein may be performed at any useful or convenient step. For instance, the beads comprising nucleic acid barcode molecules may be introduced into a partition (e.g., well or droplet) prior to, during, or following introduction of a sample into the partition. The nucleic acid molecules of a sample may be subjected to barcoding, which may occur on the bead (in cases where the nucleic acid molecules remain coupled to the bead) or following release of the nucleic acid barcode molecules into the partition. In cases where the nucleic acid molecules from the sample remain attached to the bead, the beads from various partitions may be collected, pooled, and subjected to further processing (e.g., reverse transcription, adapter attachment, amplification, clean up, sequencing). In other instances, the processing may occur in the partition. For example, conditions sufficient for barcoding, adapter attachment, reverse transcription, or other nucleic acid processing operations may be provided in the partition and performed prior to clean up and sequencing.

In some instances, a bead may comprise a capture sequence or binding sequence configured to bind to a corresponding capture sequence or binding sequence. In some instances, a bead may comprise a plurality of different capture sequences or binding sequences configured to bind to different respective corresponding capture sequences or binding sequences. For example, a bead may comprise a first subset of one or more capture sequences each configured to bind to a first corresponding capture sequence, a second subset of one or more capture sequences each configured to bind to a second corresponding capture sequence, a third subset of one or more capture sequences each configured to bind to a third corresponding capture sequence, and etc. A bead may comprise any number of different capture sequences. In some instances, a bead may comprise at least 2, 3, 4, 5, 6, 7, 8, 9, 10 or more different capture sequences or binding sequences configured to bind to different respective capture sequences or binding sequences, respectively. Alternatively or in addition, a bead may comprise at most about 10, 9, 8, 7, 6, 5, 4, 3, or 2 different capture sequences or binding sequences configured to bind to different respective capture sequences or binding sequences. In some instances, the different capture sequences or binding sequences may be configured to facilitate analysis of a same type of analyte. In some instances, the different capture sequences or binding sequences may be configured to facilitate analysis of different types of analytes (with the same bead). The capture sequence may be designed to attach to a corresponding capture sequence. Beneficially, such corresponding capture sequence may be introduced to, or otherwise induced in, a biological particle (e.g., cell, cell bead, etc.) for performing different assays in various formats (e.g., barcoded antibodies comprising the corresponding capture sequence, barcoded MHC dextramers comprising the corresponding capture sequence, barcoded guide RNA molecules comprising the corresponding capture sequence, etc.), such that the corresponding capture sequence may later interact with the capture sequence associated with the bead. In some instances, a capture sequence coupled to a bead (or other support) may be configured to attach to a linker molecule, such as a splint molecule, wherein the linker molecule is configured to couple the bead (or other support) to other molecules through the linker molecule, such as to one or more analytes or one or more other linker molecules.

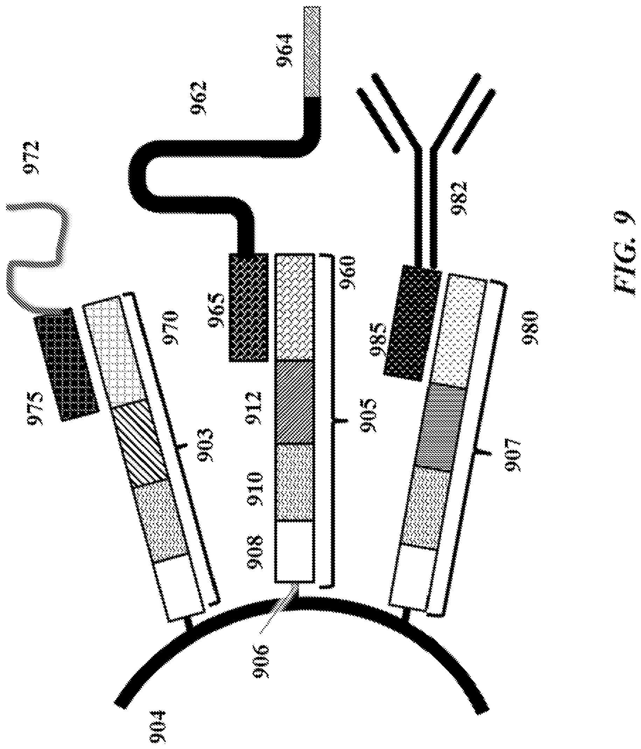

FIG. 9 illustrates another example of a barcode carrying bead. A nucleic acid molecule **905,** such as an oligonucleotide, can be coupled to a bead **904** by a releasable linkage **906,** such as, for example, a disulfide linker. The nucleic acid molecule **905** may comprise a first capture sequence **960.** The same bead **904** may be coupled (e.g., via releasable linkage) to one or more other nucleic acid molecules **903, 907** comprising other capture sequences. The nucleic acid molecule **905** may be or comprise a barcode. As noted elsewhere herein, the structure of the barcode may comprise a number of sequence elements, such as a functional sequence **908** (e.g., flow cell attachment sequence, sequencing primer sequence, etc.), a barcode sequence **910** (e.g., bead-specific sequence common to bead, partition-specific sequence common to partition, etc.), and a unique molecular identifier **912** (e.g., unique sequence within different molecules attached to the bead), or partial sequences thereof. The capture sequence **960** may be configured to attach to a corresponding capture sequence **965.** In some instances, the corresponding capture sequence **965** may be coupled to another molecule that may be an analyte or an intermediary carrier. For example, as illustrated in **FIG. 9,** the corresponding capture sequence **965** is coupled to a guide RNA molecule **962** comprising a target sequence **964,** wherein the target sequence **964** is configured to attach to the analyte. Another oligonucleotide molecule **907** attached to the bead **904** comprises a second capture sequence **980** which is configured to attach to a second corresponding capture sequence **985.** As illustrated in **FIG. 9,** the second corresponding capture sequence **985** is coupled to an antibody **982.** In some cases, the antibody **982** may have binding specificity to an analyte (e.g., surface protein). Alternatively, the antibody **982** may not have binding specificity. Another oligonucleotide molecule **903** attached to the bead **904** comprises a third capture sequence **970** which is configured to attach to a second corresponding capture sequence **975.** As illustrated in **FIG. 9,** the third corresponding capture sequence **975** is coupled to a molecule **972.** The molecule **972** may or may not be configured to target an analyte. The other oligonucleotide molecules **903, 907** may comprise the other sequences (e.g., functional sequence, barcode sequence, UMI, etc.) described with respect to oligonucleotide molecule **905.** While a single oligonucleotide molecule comprising each capture sequence is illustrated in **FIG. 9,** it will be appreciated that, for each capture sequence, the bead may comprise a set of one or more oligonucleotide molecules each comprising the capture sequence. For example, the bead may comprise any number of sets of one or more different capture sequences. Alternatively or in addition, the bead **904** may comprise other capture sequences. Alternatively or in addition, the bead **904** may comprise fewer types of capture sequences (e.g., two capture sequences). Alternatively or in addition, the bead **904** may comprise oligonucleotide molecule(s) comprising a priming sequence, such as a specific priming sequence such as an mRNA specific priming sequence (e.g., poly-T sequence), a targeted priming sequence, and/or a random priming sequence, for example, to facilitate an assay for gene expression.

In operation, the barcoded oligonucleotides may be released (e.g., in a partition), as described elsewhere herein. Alternatively, the nucleic acid molecules bound to the bead (e.g., gel bead) may be used to hybridize and capture analytes (e.g., one or more types of analytes) on the solid phase of the bead.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. For example, some precursors comprising a carboxylic acid (COOH) group can co-polymerize with other precursors to form a gel bead that also comprises a COOH functional group. In some cases, acrylic acid (a species comprising free COOH groups), acrylamide, and bis(acryloyl)cystamine can be co-polymerized together to generate a gel bead comprising free COOH groups. The COOH groups of the gel bead can be activated (e.g., via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (e.g., reactive to amine functional groups where EDC/NHS or DMTMM are used for activation). The activated COOH groups can then react with an appropriate species (e.g., a species comprising an amine functional group where the carboxylic acid groups are activated to be reactive with an amine functional group) comprising a moiety to be linked to the bead.

Beads comprising disulfide linkages in their polymeric network may be functionalized with additional species via reduction of some of the disulfide linkages to free thiols. The disulfide linkages may be reduced via, for example, the action of a reducing agent (e.g., DTT, TCEP, etc.) to generate free thiol groups, without dissolution of the bead. Free thiols of the beads can then react with free thiols of a species or a species comprising another disulfide bond (e.g., via thiol-disulfide exchange) such that the species can be linked to the beads (e.g., via a generated disulfide bond). In some cases, free thiols of the beads may react with any other suitable group. For example, free thiols of the beads may react with species comprising an acrydite moiety. The free thiol groups of the beads can react with the acrydite via Michael addition chemistry, such that the species comprising the acrydite is linked to the bead. In some cases, uncontrolled reactions can be prevented by inclusion of a thiol capping agent such as N-ethylmalieamide or iodoacetate.

Activation of disulfide linkages within a bead can be controlled such that only a small number of disulfide linkages are activated. Control may be exerted, for example, by controlling the concentration of a reducing agent used to generate free thiol groups and/or concentration of reagents used to form disulfide bonds in bead polymerization. In some cases, a low concentration (e.g., molecules of reducing agent:gel bead ratios of less than or equal to about 1:100,000,000,000, less than or equal to about 1:10,000,000,000, less than or equal to about 1:1,000,000,000, less than or equal to about 1:100,000,000, less than or equal to about 1:10,000,000, less than or equal to about 1:1,000,000, less than or equal to about 1:100,000, less than or equal to about 1:10,000) of reducing agent may be used for reduction. Controlling the number of disulfide linkages that are reduced to free thiols may be useful in ensuring bead structural integrity during functionalization. In some cases, optically-active agents, such as fluorescent dyes may be coupled to beads via free thiol groups of the beads and used to quantify the number of free thiols present in a bead and/or track a bead.

In some cases, addition of moieties to a gel bead after gel bead formation may be advantageous. For example, addition of an oligonucleotide (e.g., barcoded oligonucleotide) after gel bead formation may avoid loss of the species during chain transfer termination that can occur during polymerization. Moreover, smaller precursors (e.g., monomers or cross linkers that do not comprise side chain groups and linked moieties) may be used for polymerization and can be minimally hindered from growing chain ends due to viscous effects. In some cases, functionalization after gel bead synthesis can minimize exposure of species (e.g., oligonucleotides) to be loaded with potentially damaging agents (e.g., free radicals) and/or chemical environments. In some cases, the generated gel may possess an upper critical solution temperature (UCST) that can permit temperature driven swelling and collapse of a bead. Such functionality may aid in oligonucleotide (e.g., a primer) infiltration into the bead during subsequent functionalization of the bead with the oligonucleotide. Post-production functionalization may also be useful in controlling loading ratios of species in beads, such that, for example, the variability in loading ratio is minimized. Species loading may also be performed in a batch process such that a plurality of beads can be functionalized with the species in a single batch.

A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (e.g., barcoded oligonucleotides), the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., a nucleic acid molecule, e.g., barcoded oligonucleotide) may result in release of the species from the bead.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent can break the various disulfide bonds, resulting in bead degradation and release of the barcode sequence into the aqueous, inner environment of the droplet. In another example, heating of a droplet comprising a bead-bound barcode sequence in basic solution may also result in bead degradation and release of the attached barcode sequence into the aqueous, inner environment of the droplet.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing nucleic acid molecule (e.g., oligonucleotide) bearing beads.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

In some cases, an acrydite moiety linked to a precursor, another species linked to a precursor, or a precursor itself can comprise a labile bond, such as chemically, thermally, or photo-sensitive bond e.g., disulfide bond, UV sensitive bond, or the like. Once acrydite moieties or other moieties comprising a labile bond are incorporated into a bead, the bead may also comprise the labile bond. The labile bond may be, for example, useful in reversibly linking (e.g., covalently linking) species (e.g., barcodes, primers, etc.) to a bead. In some cases, a thermally labile bond may include a nucleic acid hybridization based attachment, e.g., where an oligonucleotide is hybridized to a complementary sequence that is attached to the bead, such that thermal melting of the hybrid releases the oligonucleotide, e.g., a barcode containing sequence, from the bead or microcapsule.

The addition of multiple types of labile bonds to a gel bead may result in the generation of a bead capable of responding to varied stimuli. Each type of labile bond may be sensitive to an associated stimulus (e.g., chemical stimulus, light, temperature, enzymatic, etc.) such that release of species attached to a bead via each labile bond may be controlled by the application of the appropriate stimulus. Such functionality may be useful in controlled release of species from a gel bead. In some cases, another species comprising a labile bond may be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)). A bond may be cleavable via other nucleic acid molecule targeting enzymes, such as restriction enzymes (e.g., restriction endonucleases), as described further below.

Species may be encapsulated in beads during bead generation (e.g., during polymerization of precursors). Such species may or may not participate in polymerization. Such species may be entered into polymerization reaction mixtures such that generated beads comprise the species upon bead formation. In some cases, such species may be added to the gel beads after formation. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera® for Illumina®). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Trapping of such species may be controlled by the polymer network density generated during polymerization of precursors, control of ionic charge within the gel bead (e.g., via ionic species linked to polymerized species), or by the release of other species. Encapsulated species may be released from a bead upon bead degradation and/or by application of a stimulus capable of releasing the species from the bead. Alternatively or in addition, species may be partitioned in a partition (e.g., droplet) during or subsequent to partition formation. Such species may include, without limitation, the abovementioned species that may also be encapsulated in a bead.

A degradable bead may comprise one or more species with a labile bond such that, when the bead/species is exposed to the appropriate stimuli, the bond is broken and the bead degrades. The labile bond may be a chemical bond (e.g., covalent bond, ionic bond) or may be another type of physical interaction (e.g., van der Waals interactions, dipole-dipole interactions, etc.). In some cases, a crosslinker used to generate a bead may comprise a labile bond. Upon exposure to the appropriate conditions, the labile bond can be broken and the bead degraded. For example, upon exposure of a polyacrylamide gel bead comprising cystamine crosslinkers to a reducing agent, the disulfide bonds of the cystamine can be broken and the bead degraded.

A degradable bead may be useful in more quickly releasing an attached species (e.g., a nucleic acid molecule, a barcode sequence, a primer, etc) from the bead when the appropriate stimulus is applied to the bead as compared to a bead that does not degrade. For example, for a species bound to an inner surface of a porous bead or in the case of an encapsulated species, the species may have greater mobility and accessibility to other species in solution upon degradation of the bead. In some cases, a species may also be attached to a degradable bead via a degradable linker (e.g., disulfide linker). The degradable linker may respond to the same stimuli as the degradable bead or the two degradable species may respond to different stimuli. For example, a barcode sequence may be attached, via a disulfide bond, to a polyacrylamide bead comprising cystamine. Upon exposure of the barcoded-bead to a reducing agent, the bead degrades and the barcode sequence is released upon breakage of both the disulfide linkage between the barcode sequence and the bead and the disulfide linkages of the cystamine in the bead.

As will be appreciated from the above disclosure, while referred to as degradation of a bead, in many instances as noted above, that degradation may refer to the disassociation of a bound or entrained species from a bead, both with and without structurally degrading the physical bead itself. For example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

Where degradable beads are provided, it may be beneficial to avoid exposing such beads to the stimulus or stimuli that cause such degradation prior to a given time, in order to, for example, avoid premature bead degradation and issues that arise from such degradation, including for example poor flow characteristics and aggregation. By way of example, where beads comprise reducible cross-linking groups, such as disulfide groups, it will be desirable to avoid contacting such beads with reducing agents, e.g., DTT or other disulfide cleaving reagents. In such cases, treatment to the beads described herein will, in some cases be provided free of reducing agents, such as DTT. Because reducing agents are often provided in commercial enzyme preparations, it may be desirable to provide reducing agent free (or DTT free) enzyme preparations in treating the beads described herein. Examples of such enzymes include, e.g., polymerase enzyme preparations, reverse transcriptase enzyme preparations, ligase enzyme preparations, as well as many other enzyme preparations that may be used to treat the beads described herein. The terms "reducing agent free" or "DTT free" preparations can refer to a preparation having less than about 1/10th, less than about 1/50th, or even less than about 1/100th of the lower ranges for such materials used in degrading the beads. For example, for DTT, the reducing agent free preparation can have less than about 0.01 millimolar (mM), 0.005 mM, 0.001 mM DTT, 0.0005 mM DTT, or even less than about 0.0001 mM DTT. In many cases, the amount of DTT can be undetectable.

Numerous chemical triggers may be used to trigger the degradation of beads. Examples of these chemical changes may comprise pH-mediated changes to the integrity of a component within the bead, degradation of a component of a bead via cleavage of cross-linked bonds, and depolymerization of a component of a bead.

In some embodiments, a bead may be formed from materials that comprise degradable chemical crosslinkers, such as BAC or cystamine. Degradation of such degradable crosslinkers may be accomplished through a number of mechanisms. In some examples, a bead may be contacted with a chemical degrading agent that may induce oxidation, reduction or other chemical changes. For example, a chemical degrading agent may be a reducing agent, such as dithiothreitol (DTT). Additional examples of reducing agents may include $\beta$-mercaptoethanol, (2S)-2-amino-1,4-dimercaptobutane (dithiobutylamine or DTBA), tris(2-carboxyethyl) phosphine (TCEP), or combinations thereof. A reducing agent may degrade the disulfide bonds formed between gel precursors forming the bead, and thus, degrade the bead. In other cases, a change in pH of a solution, such as an increase in pH, may trigger degradation of a bead. In other cases, exposure to an aqueous solution, such as water, may trigger hydrolytic degradation, and thus degradation of the bead. In some cases, any combination of stimuli may trigger degradation of a bead. For example, a change in pH may enable a chemical agent (e.g., DTT) to become an effective reducing agent.

Beads may also be induced to release their contents upon the application of a thermal stimulus. A change in temperature can cause a variety of changes to a bead. For example, heat can cause a solid bead to liquefy. A change in heat may cause melting of a bead such that a portion of the bead degrades. In other cases, heat may increase the internal pressure of the bead components such that the bead ruptures or explodes. Heat may also act upon heat-sensitive polymers used as materials to construct beads.

Any suitable agent may degrade beads. In some embodiments, changes in temperature or pH may be used to degrade thermo-sensitive or pH-sensitive bonds within beads. In some embodiments, chemical degrading agents may be used to degrade chemical bonds within beads by oxidation, reduction or other chemical changes. For example, a chemical degrading agent may be a reducing agent, such as DTT, wherein DTT may degrade the disulfide bonds formed between a crosslinker and gel precursors, thus degrading the bead. In some embodiments, a reducing agent may be added to degrade the bead, which may or may not cause the bead to release its contents. Examples of reducing agents may include dithiothreitol (DTT), $\beta$-mercaptoethanol, (2S)-2-amino-1,4-dimercaptobutane (dithiobutylamine or DTBA), tris(2-carboxyethyl) phosphine (TCEP), or combinations thereof. The reducing agent may be present at a concentration of about 0.1mM, 0.5mM, 1mM, 5mM, 10mM. The reducing agent may be present at a concentration of at least about 0.1mM, 0.5mM, 1mM, 5mM, 10mM, or greater than 10 mM. The reducing agent may be present at concentration of at most about 10mM, 5mM, 1mM, 0.5mM, 0.1mM, or less.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing oligonucleotide bearing beads.

Although **FIG. 1** and **FIG. 2** have been described in terms of providing substantially singly occupied partitions, above, in certain cases, it may be desirable to provide multiply occupied partitions, e.g., containing two, three, four or more cells and/or microcapsules (e.g., beads) comprising barcoded nucleic acid molecules (e.g., oligonucleotides) within a single partition. Accordingly, as noted above, the flow characteristics of the biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in some cases greater than about 80%, 90%, 95%, or higher.

In some cases, additional microcapsules can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction (e.g., junction **210)**. In such cases, the flow and frequency of the different beads into the channel or junction may be controlled to provide for a certain ratio of microcapsules from each source, while ensuring a given pairing or combination of such beads into a partition with a given number of biological particles (e.g., one biological particle and one bead per partition).

The partitions described herein may comprise small volumes, for example, less than about 10 microliters ($\mu$L), 5$\mu$L, 1$\mu$L, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with microcapsules, it will be appreciated that the sample fluid volume, e.g., including co-partitioned biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

## Microwells

As described herein, one or more processes may be performed in a partition, which may be a well. The well may be a well of a plurality of wells of a substrate, such as a microwell of a microwell array or plate, or the well may be a microwell or microchamber of a device (e.g., microfluidic device) comprising a substrate. The well may be a well of a well array or plate, or the well may be a well or chamber of a device (e.g., fluidic device). Accordingly, the wells or microwells may assume an "open" configuration, in which the wells or microwells are exposed to the environment (e.g., contain an open surface) and are accessible on one planar face of the substrate, or the wells or microwells may assume a "closed" or "sealed" configuration, in which the microwells are not accessible on a planar face of the substrate. In some instances, the wells or microwells may be configured to toggle between "open" and "closed" configurations. For instance, an "open" microwell or set of microwells may be "closed" or "sealed" using a membrane (e.g., semi-permeable membrane), an oil (e.g., fluorinated oil to cover an aqueous solution), or a lid, as described elsewhere herein. The wells or microwells may be initially provided in a "closed" or "sealed" configuration, wherein they are not accessible on a planar surface of the substrate without an external force. For instance, the "closed" or "sealed" configuration may comprise a substrate such as a sealing film or foil that is puncturable or pierceable by pipette tip(s). Suitable materials for the substrate include, without limitation, polyester, polypropylene, polyethylene, vinyl, and aluminum foil.

The well may have a volume of less than 1 milliliter (mL). For instance, the well may be configured to hold a volume of at most 1000 microliters ($\mu$L), at most 100 $\mu$L, at most 10 $\mu$L, at most 1 $\mu$L, at most 100 nano-

liters (nL), at most 10 nL, at most 1 nL, at most 100 picoliters (pL), at most 10 (pL), or less. The well may be configured to hold a volume of about 1000 μL, about 100 μL, about 10 μL, about 1 μL, about 100 nL, about 10 nL, about 1 nL, about 100 pL, about 10 pL, etc. The well may be configured to hold a volume of at least 10 pL, at least 100 pL, at least 1 nL, at least 10 nL, at least 100 nL, at least 1 μL, at least 10 μL, at least 100 μL, at least 1000 μL, or more. The well may be configured to hold a volume in a range of volumes listed herein, for example, from about 5 nL to about 20 nL, from about 1 nL to about 100 nL, from about 500 pL to about 100 μL, etc. The well may be of a plurality of wells that have varying volumes and may be configured to hold a volume appropriate to accommodate any of the partition volumes described herein.

In some instances, a microwell array or plate comprises a single variety of microwells. In some instances, a microwell array or plate comprises a variety of microwells. For instance, the microwell array or plate may comprise one or more types of microwells within a single microwell array or plate. The types of microwells may have different dimensions (e.g., length, width, diameter, depth, cross-sectional area, etc.), shapes (e.g., circular, triangular, square, rectangular, pentagonal, hexagonal, heptagonal, octagonal, nonagonal, decagonal, etc.), aspect ratios, or other physical characteristics. The microwell array or plate may comprise any number of different types of microwells. For example, the microwell array or plate may comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well may have any dimension (e.g., length, width, diameter, depth, cross-sectional area, volume, etc.), shape (e.g., circular, triangular, square, rectangular, pentagonal, hexagonal, heptagonal, octagonal, nonagonal, decagonal, other polygonal, etc.), aspect ratios, or other physical characteristics described herein with respect to any well.

In certain instances, the microwell array or plate comprises different types of microwells that are located adjacent to one another within the array or plate. For instance, a microwell with one set of dimensions may be located adjacent to and in contact with another microwell with a different set of dimensions. Similarly, microwells of different geometries may be placed adjacent to or in contact with one another. The adjacent microwells may be configured to hold different articles; for example, one microwell may be used to contain a cell, cell bead, or other sample (e.g., cellular components, nucleic acid molecules, etc.) while the adjacent microwell may be used to contain a microcapsule, droplet, bead, or other reagent. In some cases, the adjacent microwells may be configured to merge the contents held within, e.g., upon application of a stimulus, or spontaneously, upon contact of the articles in each microwell.

As is described elsewhere herein, a plurality of partitions may be used in the systems, compositions, and methods described herein. For example, any suitable number of partitions (e.g., wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells may comprise both unoccupied wells (e.g., empty wells) and occupied wells.

A well may comprise any of the reagents described herein, or combinations thereof. These reagents may include, for example, barcode molecules, enzymes, adapters, and combinations thereof. The reagents may be physically separated from a sample (e.g., a cell, cell bead, or cellular components, e.g., proteins, nucleic acid molecules, etc.) that is placed in the well. This physical separation may be accomplished by containing the reagents within, or coupling to, a microcapsule or bead that is placed within a well. The physical separation may also be accomplished by dispensing the reagents in the well and overlaying the reagents with a layer that is, for example, dissolvable, meltable, or permeable prior to introducing the polynucleotide sample into the well. This layer may be, for example, an oil, wax, membrane (e.g., semipermeable membrane), or the like. The well may be sealed at any point, for example, after addition of the microcapsule or bead, after addition of the reagents, or after addition of either of these components. The sealing of the well may be useful for a variety of purposes, including preventing escape of beads or loaded reagents from the well, permitting select delivery of certain reagents (e.g., via the use of a semi-permeable membrane), for storage of the well prior to or following further processing, etc.

A well may comprise free reagents and/or reagents encapsulated in, or otherwise coupled to or associated with, microcapsules, beads, or droplets. Any of the reagents described in this disclosure may be encapsulated in, or otherwise coupled to, a microcapsule, droplet, or bead, with any chemicals, particles, and elements suitable for sample processing reactions involving biomolecules, such as, but not limited to, nucleic acid molecules and proteins. For example, a bead or droplet used in a sample preparation reaction for DNA sequencing may comprise one or more of the following reagents: enzymes, restriction enzymes (e.g., multiple cutters), ligase, polymerase, fluorophores, oligonucleotide barcodes, adapters, buffers, nucleotides (e.g., dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, oligonucleotides, nucleotides, deoxyribonucleotide triphosphates (dNTPs), dideoxyribonucleotide triphosphates (ddNTPs), DNA, RNA, peptide polynucleotides, complementary DNA (cDNA), double stranded DNA (dsDNA), single stranded DNA (ssDNA), plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA, polymerase, ligase, restriction enzymes, proteases, nucleases, protease inhibitors, nuclease inhibitors, chelating agents, reducing agents, oxidizing agents, fluorophores, probes, chromophores, dyes, organics, emulsifiers, surfactants, stabilizers, polymers, water, small molecules, pharmaceuticals, radioactive molecules, preservatives, antibiotics, aptamers, and pharmaceutical drug compounds. As described herein, one or more reagents in the well may be used to perform one or more reactions, including but not limited to: cell lysis, cell fixation, permeabilization, nucleic acid reactions, e.g., nucleic acid extension reactions, amplification, reverse transcription, transposase reactions (e.g., tagmentation), etc.

The wells may be provided as a part of a kit. For example, a kit may comprise instructions for use, a microwell array or device, and reagents (e.g., beads). The kit may comprise any useful reagents for performing the processes described herein, e.g., nucleic acid reactions, barcoding of nucleic acid molecules, sample processing (e.g., for cell lysis, fixation, and/or permeabilization).

In some cases, a well comprises a microcapsule, bead, or droplet that comprises a set of reagents that has a similar attribute (e.g., a set of enzymes, a set of minerals, a set of oligonucleotides, a mixture of different barcode molecules, a mixture of identical barcode molecules). In other cases, a microcapsule, bead, or droplet comprises a heterogeneous mixture of reagents. In some cases, the heterogeneous mixture of reagents can comprise all components necessary to perform a reaction. In some cases, such mixture can comprise all components necessary to perform a reaction, except for 1, 2, 3, 4, 5, or more components necessary to perform a reaction. In some cases, such additional components are contained within, or otherwise coupled to, a different microcapsule, droplet, or bead, or within a solution within a partition (e.g., microwell) of the system.

FIG. 10 schematically illustrates an example of a microwell array. The array can be contained within a substrate **1000**. The substrate **1000** comprises a plurality of wells **1002**. The wells **1002** may be of any size or shape, and the spacing between the wells, the number of wells per substrate, as well as the density of the wells on the substrate **1000** can be modified, depending on the particular application. In one such example application, a sample molecule **1006,** which may comprise a cell or cellular components (e.g., nucleic acid molecules) is co-partitioned with a bead **1004,** which may comprise a nucleic acid barcode molecule coupled thereto. The wells **1002** may be loaded using gravity or other loading technique (e.g., centrifugation, liquid handler, acoustic loading, optoelectronic, etc.). In some instances, at least one of the wells **1002** contains a single sample molecule **1006** (e.g., cell) and a single bead **1004.**

Reagents may be loaded into a well either sequentially or concurrently. In some cases, reagents are introduced to the device either before or after a particular operation. In some cases, reagents (which may be provided, in certain instances, in microcapsules, droplets, or beads) are introduced sequentially such that different reactions or operations occur at different steps. The reagents (or microcapsules, droplets, or beads) may also be loaded at operations interspersed with a reaction or operation step. For example, microcapsules (or droplets or beads) comprising reagents for fragmenting polynucleotides (e.g., restriction enzymes) and/or other enzymes (e.g., transposases, ligases, polymerases, etc.) may be loaded into the well or plurality of wells, followed by loading of microcapsules, droplets, or beads comprising reagents for attaching nucleic acid barcode molecules to a sample nucleic acid molecule. Reagents may be provided concurrently or sequentially with a sample, e.g., a cell or cellular components (e.g., organelles, proteins, nucleic acid molecules, carbohydrates, lipids, etc.). Accordingly, use of wells may be useful in performing multi-step operations or reactions.

As described elsewhere herein, the nucleic acid barcode molecules and other reagents may be contained within a microcapsule, bead, or droplet. These microcapsules, beads, or droplets may be loaded into a partition (e.g., a microwell) before, after, or concurrently with the loading of a cell, such that each cell is contacted with a different microcapsule, bead, or droplet. This technique may be used to attach a unique nucleic acid barcode molecule to nucleic acid molecules obtained from each cell. Alternatively or in addition to, the sample nucleic acid molecules may be attached to a support. For instance, the partition (e.g., microwell) may comprise a bead which has coupled thereto a plurality of nucleic acid barcode molecules. The sample nucleic acid molecules, or derivatives thereof, may couple or attach to the nucleic acid barcode molecules on the support. The resulting barcoded nucleic acid molecules may then be removed from the partition, and in some instances, pooled and sequenced. In such cases, the nucleic acid barcode sequences may be used to trace the origin of the sample nucleic acid molecule. For example, polynucleotides with identical barcodes may be determined to originate from the same cell or partition, while polynucleotides with different barcodes may be determined to originate from different cells or partitions.

The samples or reagents may be loaded in the wells or microwells using a variety of approaches. The samples (e.g., a cell, cell bead, or cellular component) or reagents (as described herein) may be loaded into the well or microwell using an external force, e.g., gravitational force, electrical force, magnetic force, or using mechanisms to drive the sample or reagents into the well, e.g., via pressure-driven flow, centrifugation, optoelectronics, acoustic loading, electrokinetic pumping, vacuum, capillary flow, etc. In certain cases, a fluid handling system may be used to load the samples or reagents into the well. The loading of the samples or reagents may follow a Poissonian distribution or a non-Poissonian distribution, e.g., super Poisson or sub-Poisson. The geometry, spacing between wells, density, and size of the microwells may be modified to accommodate a useful sample or reagent distribution; for instance, the size and spacing of the microwells may be adjusted such that the sample or reagents may be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which may, in some instances, also be encapsulated in a droplet). The droplet and the bead (or droplet containing the bead) may be loaded simultaneously or sequentially, and the droplet and the bead may be merged, e.g., upon contact of the droplet and the bead, or upon application of a stimulus (e.g., external force, agitation, heat, light, magnetic or electric force, etc.). In some cases, the loading of the droplet and the bead is super-Poissonian. In other examples of pairs of microwells, the wells are configured to hold two droplets comprising different reagents and/or samples, which are merged upon contact or upon application of a stimulus. In such instances, the droplet of one microwell of the pair can comprise reagents that may react with an agent in the droplet of the other microwell of the pair. For instance, one droplet can comprise reagents that are configured to release the nucleic acid barcode molecules of a bead contained in another droplet, located in the adjacent microwell. Upon merging of the droplets, the nucleic acid barcode molecules may be released from the bead into the partition (e.g., the microwell or microwell pair that are in contact), and further processing may be performed (e.g., barcoding, nucleic acid reactions, etc.). In cases where intact or live cells are loaded in the microwells, one of the droplets may comprise lysis reagents for lysing the cell upon droplet merging.

A droplet or microcapsule may be partitioned into a well. The droplets may be selected or subjected to pre-processing prior to loading into a well. For instance, the droplets may comprise cells, and only certain droplets, such as those containing a single cell (or at least one cell), may be selected for use in loading of the wells. Such a pre-selection process may be useful in efficient loading of single cells, such as to obtain a non-Poissonian distribution, or to pre-filter cells for a selected characteristic prior to further partitioning in the wells. Additionally, the technique may be useful in obtaining or preventing cell doublet or multiplet formation prior to or during loading of the microwell.

In some instances, the wells can comprise nucleic acid barcode molecules attached thereto. The nucleic acid barcode molecules may be attached to a surface of the well (e.g., a wall of the well). The nucleic acid barcode molecule (e.g., a partition barcode sequence) of one well may differ from the nucleic acid barcode molecule of another well, which can permit identification of the contents contained with a single partition or well. In some cases, the nucleic acid barcode molecule can comprise a spatial barcode sequence that can identify a spatial coordinate of a well, such as within the well array or well plate. In some cases, the nucleic acid barcode molecule can comprise a unique molecular identifier for individual molecule identification. In some instances, the nucleic acid barcode molecules may be configured to attach to or capture a nucleic acid molecule within a sample or cell distributed in the well. For example, the nucleic acid barcode molecules may comprise a capture sequence that may be used to capture or hybridize to a nucleic acid molecule (e.g., RNA, DNA) within the sample. In some instances, the nucleic acid barcode molecules may be releasable from the microwell. For instance, the nucleic acid barcode molecules may comprise a chemical cross-linker which may be cleaved upon application of a stimulus (e.g., photo-, magnetic, chemical, biological, stimulus). The released nucleic acid barcode molecules, which may be hybridized or configured to hybridize to a sample nucleic acid molecule, may be collected and pooled for further processing, which can include nucleic acid processing (e.g., amplification, extension, reverse transcription, etc.) and/or characterization (e.g., sequencing). In such cases, the unique partition barcode sequences may be used to identify the cell or partition from which a nucleic acid molecule originated.

Characterization of samples within a well may be performed. Such characterization can include, in non-limiting examples, imaging of the sample (e.g., cell, cell bead, or cellular components) or derivatives thereof. Characterization techniques such as microscopy or imaging may be useful in measuring sample profiles in fixed spatial locations. For instance, when cells are partitioned, optionally with beads, imaging of each microwell and the contents contained therein may provide useful information on cell doublet formation (e.g., frequency, spatial locations, etc.), cell-bead pair efficiency, cell viability, cell size, cell morphology, expression level of a biomarker (e.g., a surface marker, a fluorescently labeled molecule therein, etc.), cell or bead loading rate, number of cell-bead pairs, etc. In some instances, imaging may be used to characterize live cells in the wells, including, but not limited to: dynamic live-cell tracking, cell-cell interactions (when two or more cells are co-partitioned), cell proliferation, etc. Alternatively or in addition to, imaging may be used to characterize a quantity of amplification products in the well.

In operation, a well may be loaded with a sample and reagents, simultaneously or sequentially. When cells or cell beads are loaded, the well may be subjected to washing, e.g., to remove excess cells from the well, microwell array, or plate. Similarly, washing may be performed to remove excess beads or other reagents from the well, microwell array, or plate. In the instances where live cells are used, the cells may be lysed in the individual partitions to release the intracellular components or cellular analytes. Alternatively, the cells may be fixed or permeabilized in the individual partitions. The intracellular components or cellular analytes may couple to a support, e.g., on a surface of the microwell, on a solid support (e.g., bead), or they may be collected for further downstream processing. For instance, after cell lysis, the intracellular components or cellular analytes may be transferred to individual droplets or other partitions for barcoding. Alternatively, or in addition to, the intracellular components or cellular analytes (e.g., nucleic acid molecules) may couple to a bead comprising a nucleic acid barcode molecule; subsequently, the bead may be collected and further processed, e.g., subjected to nucleic acid reaction such as reverse transcription, amplification, or extension, and the nucleic acid molecules thereon may be further characterized, e.g., via sequencing. Alternatively, or in addition to, the intracellular components or cellular analytes may be barcoded in the well (e.g., using a bead comprising nucleic acid barcode molecules that are releasable or on a surface of the microwell comprising nucleic acid barcode molecules). The barcoded nucleic acid molecules or analytes may be further processed in the well, or the barcoded nucleic acid molecules or analytes may be collected from the individual partitions and subjected to further processing outside the partition. Further processing can include nucleic acid processing (e.g., performing an amplification, extension) or characterization (e.g., fluorescence monitoring of amplified molecules, sequencing). At any convenient or useful step, the well (or microwell array or plate) may be sealed (e.g., using an oil, membrane, wax, etc.), which enables storage of the assay or selective introduction of additional reagents.

FIG. 11 schematically shows an example workflow for processing nucleic acid molecules within a sample. A substrate **1100** comprising a plurality of microwells **1102** may be provided. A sample **1106** which may comprise a cell, cell bead, cellular components or analytes (e.g., proteins and/or nucleic acid molecules) can be co-partitioned, in a plurality of microwells **1102,** with a plurality of beads **1104** comprising nucleic acid barcode molecules. During process **1110,** the sample **1106** may be processed within the partition. For instance, in the case of live cells, the cell may be subjected to conditions sufficient to lyse the cells and release the analytes contained therein. In process **1120,** the bead **1104** may be further processed. By way of example, processes **1120a** and **1120b** schematically illustrate different workflows, depending on the properties of the bead **1104.**

In **1120a,** the bead comprises nucleic acid barcode molecules that are attached thereto, and sample nucleic acid molecules (e.g., RNA, DNA) may attach, e.g., via hybridization of ligation, to the nucleic acid barcode molecules. Such attachment may occur on the bead. In process **1130,** the beads **1104** from multiple wells **1102** may be collected and pooled. Further processing may be performed in process **1140.** For example, one or more nucleic acid reactions may be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences may be appended to each end of the nucleic acid molecule. In process **1150,** further characterization, such as sequencing may be performed to generate sequencing reads. The sequencing reads may yield information on individual cells or populations of cells, which may be represented visually or graphically, e.g., in a plot **1155.**

In **1120b,** the bead comprises nucleic acid barcode molecules that are releasably attached thereto, as described below. The bead may degrade or otherwise release the nucleic acid barcode molecules into the well **1102;** the nucleic acid barcode molecules may then be used to barcode nucleic acid molecules within the well **1102.** Further processing may be performed either inside the partition or outside the partition. For example, one or more nucleic acid reactions may be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences may be appended to each end of the nucleic acid molecule. In process **1150,** further characterization, such as sequencing may be performed to generate sequencing reads. The sequencing reads may yield information on individual cells or populations of cells, which may be represented visually or graphically, e.g., in a plot **1155.**

### Cell Beads and Methods for Cell Bead Generation

Methods of the present disclosure may comprise generation of one or more cell beads comprising one or more of the polymers disclosed herein. See, e.g., U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878, which are incorporated by reference in their entirety, for exemplary cell bead generation systems and methods. For example, cells and polymer or gel precursors are mixed with an immiscible fluid (e.g., an oil), thereby generating a plurality of aqueous droplets, including a droplet comprising a cell. A droplet may comprise a charged species, as described herein. A droplet may be subjected to conditions sufficient for polymerization or gelation of the polymer or gel precursors to generate a cell bead comprising a cell. Gelation may comprise any of the gelation mechanisms and polymers described herein, including those utilizing a click chemistry reaction, as described elsewhere herein. In some instances, a cell bead is subjected to treatment conditions sufficient to lyse the cell, releasing components of the cell into the cell bead. In other embodiments, the cell is lysed in a droplet prior to polymerization or gelation of the polymer or gel precursors to generate a cell bead. In still other embodiments, a cell is permeabilized before or after polymerization or gelation of the polymer or gel precursors. Cell beads may be collected in an aqueous phase to generate a plurality of cell beads. Cell beads may be stored for further processing. In some cases, charged species may be attached to the cell beads subsequent to polymerization or gelation of the polymer or gel precursor. For instance, polymer or gel precursors may comprise one or more functional groups that facilitate the attachment of the charged species subsequent to polymerization or gelation of the polymer or gel precursors. In other embodiments, the polymer or gel precursors comprise functional groups comprising the charged species, which are incorporated into the cell bead during polymerization or gelation of the polymer or gel precursors.

In an aspect, the present disclosure provides methods for generating a cell bead comprising a charged species. First, a partition may be generated comprising a cell from a plurality of cells, a polymeric or gel precursor, and a charged species. Next, the partition may be subjected to conditions sufficient to react the polymeric or gel precursor to generate a polymer or gel network comprising the cell or a derivative thereof and the charged species, thereby generating a cell bead. The partition may be subjected to conditions sufficient to polymerize or gel the polymeric or gel precursors. Conditions sufficient to polymerize or gel polymeric or gel precursors are described elsewhere herein. In some embodiments, the cell is lysed to release components of the cell into the cell bead. The cell may be lysed prior to polymerization or gelling of the polymeric or gel precursors, concurrently with polymerization or gelling of the polymeric or gel precursors, or subsequent to polymerization or gelling of the polymeric or gel precursors. In other embodiments, the cell in the cell bead is not lysed, but is permeabilized to allow access to components within the nucleus.

In another aspect, the present disclosure provides methods for generating a cell bead comprising a charged species. First, a partition may be generated comprising a nucleus isolated from a cell, a polymeric or gel precursor, and a charged species. Next, the partition may be subjected to conditions sufficient to react the polymeric or gel precursors to generate a polymer or gel network comprising the nucleus and the charged species, thereby generating a cell bead. The partition may be subjected to conditions sufficient to polymerize or gel the polymeric or gel precursors. Conditions sufficient to polymerize or gel polymeric or gel precursors are described elsewhere herein. For example, a copper catalyst may be used to catalyze a click chemistry reaction, thereby generating a hydrogel. In some embodiments, the nucleus is lysed to release components of the nucleus into the cell bead. The nucleus may be lysed prior to polymerizing or gelling the polymeric or gel precursors, concurrently with polymerizing or gelling the polymeric or gel precursors, or subsequent to polymerizing or gelling the polymeric or gel precursors. In other embodiments, the nucleus in the cell bead is not lysed, but is permeabilized to allow access to nuclear components within the nucleus.

A charged species may be a positively charged species. A positively charged species may be a reagent comprising a positive charge. A positively charged species may comprise trimethylammonium. A positively charged species may be (3-Acrylamidopropyl)-trimethylammonium. A charged species may be a negatively charged species. A negatively charged species may comprise phosphate. A charged species may be attached to the polymer or gel network. A charged species may be incorporated into a polymer or gel network during polymerization. A cell bead may comprise one or more chemical cross-linkers. A chemical cross-linker may be a disulfide bond. A charged species may be attached to one or more chemical cross-linkers. A derivative of a cell may be a component from a cell (e.g., DNA, RNA, protein, etc.). A method of generating a cell bead may comprise lysing a cell within a partition (e.g., a droplet) to release a component from the cell. A component may be a nucleic acid. A nucleic acid may be DNA (e.g., genomic DNA) or RNA (e.g., mRNA, siRNA). A component may be a protein. A component may be a negatively charged component, for example, DNA, RNA, or miRNA. A component may be a positively charged component, for example, a protein. A component from a cell may interact with a charged species. A component from a cell may be non-covalently attached to a charged species.

In some embodiments, a negatively charged component from or derived from a cell (e.g., DNA) interacts with a positively charged species (e.g., ((3-Acrylamidopropyl)-trimethylammonium) of the cell bead (e.g., a positively charged functional group of the cell bead polymers) via ionic interactions. In other embodiments, a positively charged component from or derived from a cell (e.g., a protein) interacts with a negatively charged species (e.g., phosphate) of the cell bead (e.g., a negatively charged functional group of the cell bead polymers) via ionic interactions. In still other embodiments, a negatively charged component from or derived from a cell (e.g., DNA) interacts with a positively charged species (e.g., ((3-Acrylamidopropyl)-trimethylammonium) of the cell bead (e.g., a positively charged functional group of the cell bead polymers) and a positively charged component from or derived from a cell (e.g., a protein) interacts with a negatively charged species (e.g., phosphate) of the cell bead (e.g., a negatively charged functional group of the cell bead polymers). Thus, one or more components from a cell may be capable of being retained within the cell bead, for example, due to electrostatic interactions with a charged species of a cell bead. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

In an aspect, the present disclosure provides methods for generating a cell bead comprising an electrically charged polymer or gel network (e.g., a cell bead comprising a charged species). First, a partition may be generated comprising a cell from a plurality of cells and a polymeric or gel precursor. Next, the partition may be subjected to conditions sufficient to react said electrically charged polymeric or gel precursor to generate an electrically charged polymer or gel network comprising the cell or a derivative thereof, thereby providing the cell bead comprising the charged species. The reaction may be such that the net charge on the polymer or gel precursor is changed, thereby generating an electrically charged polymer or gel network. The reaction may be such that the net charge on the polymer or gel network is changed, thereby generating an electrically charged polymer or gel network.

The polymer or gel precursor may be positively charged. The polymer or gel precursor may comprise chitosan. The polymer or gel precursor may comprise polyethyleneimine (PEI). The polymer or gel precursor may be negatively charged. The polymer or gel precursor may comprise alginate. A derivative of a cell may be a component from a cell (e.g., DNA, RNA, protein, etc.). A method of generating a cell bead may comprise lysing a cell within a partition (e.g., a droplet) to release a component from the cell. A component may be a nucleic acid. A nucleic acid may be DNA (e.g., genomic DNA) or RNA (e.g., mRNA, siRNA). A component may be a protein. A component may be a negatively charged component, for example, DNA, RNA, or miRNA. A component may be a positively charged component, for example, a protein. A component from a cell may interact with the electrically charged polymer or gel network. A component from a cell may be non-covalently attached to the polymer or gel network of a cell bead comprising a charged species. In some embodiments, a negatively charged component from or derived from a cell (e.g., DNA) interacts with a positively charged species of the cell bead (e.g., a positive charged polymer or gel network) via ionic interactions. In other embodiments, a positively charged component from or derived from a cell (e.g., a protein) interacts with a negatively charged species of the cell bead (e.g., a negatively charged polymer or gel network) via ionic interactions. In still other embodiments, a negatively charged component from or derived from a cell (e.g., DNA) interacts with a positively charged species of the cell bead (e.g., a positive charged polymer or gel network) and a positively charged component from or derived from a cell (e.g., a protein) interacts with a negatively charged species of the cell bead (e.g., a negatively charged polymer or gel network). Thus, one or more components from a cell may be capable of being retained within the cell bead, for example, due to electrostatic interactions with a charged species of a cell bead. A component from a cell may be capable of being retained within the cell bead, for example, due to interactions with an electrically charged polymer or gel network. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

In an aspect, the present disclosure provides methods for generating a cell bead comprising a charged species. First, a partition may be generated comprising a cell from a plurality of cells and a polymeric or gel precursor. Next, the partition may be subjected to conditions sufficient to react the polymeric or gel precursor to generate a polymer or gel network comprising the cell or a derivative thereof. Next, a charged species may be coupled to the polymer or gel network, thereby providing the cell bead comprising the charged species. The partition may be subjected to conditions sufficient to polymerize or gel the polymeric or gel precursors. Conditions sufficient to polymerize or gel polymeric or gel precursors are described elsewhere herein. For example, a copper catalyst may be used to catalyze a click chemistry reaction, thereby generating a hydrogel. In some cases, the cell is lysed to release cellular components. The cell may be lysed prior to polymerizing or gelling the polymeric or gel precursors, concurrently with polymerizing or gelling the polymeric or gel precursors, or subsequent to polymerizing or gelling the polymeric or gel precursors.

A polymer or gel network can be a degradable polymer or gel network, as described herein, such that a cell bead is a degradable cell bead. Any number of cell beads may be generated by generating a plurality of partitions. In some cases, about 1, about 2, about 3, about 4, about 5, about 10, about 50, about 100, about 500, about 1000, about 5000, about 10000, about 20000, about 50000, about 100000, or more cell beads are generated, thereby generating a plurality of cell beads. A cell bead may be partitioned together with a barcode bead (e.g., a gel bead) for analysis of a cell or components thereof.

Cell beads may be generated by methods as described herein, for example by polymerization of molecular precursors (e.g., polymer or gel precursors) in a partition comprising a cell or constituents from a cell. Cell beads can comprise one or more different types of components from a cell, including, for example, DNA (e.g., gDNA, chromatin, etc.), RNA (e.g., mRNA, miRNA), proteins, and/or metabolites. Components may be comprised in and/or attached to cell beads. Cell beads can be generated by encapsulating a cell in a polymer or gel matrix and lysing the cell in the gel or polymer matrix, lysing the cell while it is being encapsulated in the polymer or gel matrix, or lysing the cell so that its constituents are encapsulated in the polymer or gel matrix. The polymer or gel matrix may comprise one or more charged species configured to interact with a component from a cell (e.g., DNA, RNA, proteins, etc.).

The partition used in generating a cell bead may comprise one or more reagents for conducting one or more reactions. Species may include, for example, reagents for a nucleic acid amplification or extension reaction (e.g., primers, polymerases, nucleotides, co-factors (e.g., ionic co-factors), buffers, etc.) including those described herein, reagents for enzymatic reactions (e.g., enzymes, co-factors, substrates, buffers, etc.), reagents for nucleic acid modification reactions, such as polymerization, ligation, or digestion, and/or reagents for template preparation.

Reagents may comprise reagents for minimizing damage of nucleic acids resulting from a click chemistry reaction. For example, a radical scavenger may be added to a partition, thereby reducing the risk of damage to nucleic acids caused by free radicals generated during a click chemistry reaction. In some cases, the radical scavenger comprises dimethyl sulfoxide (DMSO). DMSO may be added to a partition used in generating a cell bead at a sufficient concentration for preventing nucleic acid damage. In some embodiments, DMSO is added to a partition at an amount of at least about 1%, about 2%, about 3%, about 4%, about 5%, about 6%, about 7%, about 8%, about 9%, about 10%, or greater.

One or more reagents within a partition may be attached to precursors (e.g., polymer or gel precursors). Reagents may be covalently attached to precursors. Reagents may be reversibly or irreversible attached to precursors. Reagents may be attached to precursors via an acrydite moiety.

In some cases, oligonucleotides may be attached to the precursors. Oligonucleotides attached to precursors may be useful in, for example, capturing RNA and/or performing a reverse transcription reaction. Oligonucleotides may comprise a poly-T sequence or a poly-U sequence (e.g., may be a poly-T primer). In some embodiments, a poly-T sequence is used to hybridize to a poly-A sequence, for example, from mRNA of a cell. In some embodiments, a poly-U sequence is used to hybridize to a poly-A sequence, for example, from mRNA of a cell.

In some cases, an oligonucleotide, such as a poly-T sequence, can be attached to a precursor (e.g., polymer) via an irreversible click chemistry reaction. In some embodiments, this click chemistry attachment of an oligonucleotide can be carried out during the crosslinking of the polymers that results in a gel matrix. For example, a propargylated poly-T oligonucleotide introduced into an emulsion droplet together with the polymers modified with azide and alkyne click chemistry groups is attached via CuAAC click chemistry resulting in a 1,2,3-triazole linkage with some of the azide-modified linker sites of the azide-modified polymer. The other sites form crosslinks with the alkyne-modified polymers resulting in a gel matrix comprising covalently attached poly-T reagents capable of capturing polyadenylated RNA transcripts.

A partition used in generating a cell bead may comprise one or more particles (e.g., magnetic particles). One or more reagents within a partition may be attached to the particle. Reagents may be covalently attached to the particle. Reagents may be reversibly or irreversibly attached to the particle. Regents may be attached to the particle via an acrydite moiety. In some cases, oligonucleotides may be attached to the particle. Oligonucleotides attached to the particle may be useful in, for example, capturing RNA and performing a reverse transcription reaction. In some embodiments, the particles (which are optionally magnetic particles) comprise oligonucleotides attached thereto that comprise a poly-T sequence capable of hybridizing to a poly-A sequence, for example, from mRNA of a cell.

A cell within a partition may be lysed as described herein, thereby releasing constituents from the cell into the partition. Constituents may include multiple types of cellular components, including proteins, metabolites, and/or nucleic acid molecules (e.g., DNA, RNA (e.g. messenger RNA), etc.). Alternatively, or in addition, a cell within a partition may by permeabilized. Permeabilization may allow for transfer of certain reagents, species, constituents, etc. into and/or out of a cell with or without complete cellular lysis. In some embodiments, the cell is lysed or permeabilized prior to the polymerization or gelling of the cell bead. In other embodiments, the cell is lysed or permeabilized concurrent with the polymerization or gelling of the cell bead. In some embodiments, the cell is lysed or permeabilized subsequent to the polymerization or gelling of the cell bead. In still other embodiments, the cell is not lysed or permeabilized while in the cell bead.

Reagents can be included within a partition, including reagents attached to precursors, particles, etc., and may be used to perform one or more reactions on the cell or constituents from or derived from a cell. A reaction may be, for example, amplification, reverse transcription, or deamination reaction. In some embodiments, the one or more reactions are performed prior to the polymerization or gelling of the cell bead. In some embodiments, the one or more reactions are performed concurrent with the polymerization or gelling of the cell bead. In some embodiments, the one or more reactions are performed subsequent to the polymerization or gelling of the cell bead. In some cases, oligonucleotides (e.g., primers) are used to perform a reverse transcription reaction on messenger RNA from a cell, thereby generating complementary DNA (cDNA). Reverse transcription may comprise the addition of additional nucleotides, e.g., a polynucleotide such as polyC, to the cDNA. In some cases, template switching may be performed to further extend the cDNA. Template switching may append one or more additional sequences to the cDNA. Additional sequences may, in some cases, be used to facilitate nucleic acid extension/amplification and/or barcoding, as described herein. cDNA may be attached to precursors and/or particles. In some cases, oligonucleotides are used to capture messenger RNA from a cell, (e.g., via hybridization) prior to generation of a cell bead.

In some embodiments, a partition is subjected to conditions sufficient to generate a cell bead comprising one or more reagents. For example, a partition droplet comprising polymer precursors attached to reagents (e.g., primers, nucleic acid molecules, etc.) may be polymerized or gelled such that the reagents are attached to the polymer or gel matrix (e.g., attached to a cell bead). In some instances, the reagents are releasably attached to the gel precursor via a labile bond (e.g., a chemically labile bond, thermally labile bond, or photo-labile bond). Reagents may be covalently attached to a cell bead. Reagents may be reversible or irreversibly attached to a cell bead. Reagents may be attached to the surface of a gel bead. Reagents may be attached to the inside of a cell bead. In some cases, mRNA is attached to a cell bead. For example, polymer precursors attached to mRNA from a cell may be polymerized or gelled to generate a cell bead such that the mRNA is attached to the cell bead. In some cases, cDNA is attached to a cell bead. For example, polymer precursors attached to cDNA derived from a cell may be polymerized to generate a cell bead such that the cDNA is attached to the cell bead. In some cases, one or more oligonucleotides are attached to a cell bead. For example, polymer precursors attached to the oligonucleotides may be polymerized or gelled to generate a cell bead such that the oligonucleotides are attached to the cell bead.

Attaching macromolecular constituents (e.g., nucleic acid molecules, protein, etc.) to a cell bead or a particle within a cell bead may be useful in preparing the species for further processing. For example, nucleic acid molecules attached to a cell bead or particle may be processed while remaining attached to the cell bead or particle. Following processing, a nucleic acid may be released (e.g., released into a partition) from a cell bead and/or particle for analysis. In some cases, it may be useful to attach one type of cellular component or derivative thereof (e.g., mRNA, cDNA) to a cell bead or a particle within a cell bead, while encapsulating but not attaching another type of cellular component (e.g., genomic DNA). This may be useful in, for example, facilitating separate processing of multiple types of components. For example, following cell bead formation, cell beads may be transferred to an aqueous solution and subjected to additional processing as described herein. For example, cell beads may be subjected, in bulk, to reverse transcription to generate cDNA from captured mRNA (e.g., hybridized to an oligonucleotide attached to the cell bead matrix or a particle, such as a magnetic particle).

## Click Chemistry

As used herein, the term "click chemistry," generally refers to reactions that are modular, wide in scope, give high yields, generate only inoffensive byproducts, such as those that can be removed by nonchromatographic methods, and are stereospecific (but not necessarily enantioselective). See, e.g., U.S. Pat. Pub. 2019/0100632 (now U.S. Pat. 10,590,244), U.S. Pat. Pub. 2019/0233878, and Angew. Chem. Int. Ed., 2001, 40(11):2004-2021, which are entirely incorporated herein by reference for all purposes.

In some cases, click chemistry can describe pairs of functional groups that can selectively react with each other in mild, aqueous conditions. An example of click chemistry reaction can be the Huisgen 1,3-dipolar cycloaddition of an azide and an alkynes, e.g., Coppercatalyzed reaction of an azide with an alkyne to form a 5-membered heteroatom ring called 1,2,3-triazole. The reaction can also be known as a Cu(I)-Catalyzed Azide-Alkyne Cycloaddition (CuAAC), a Cu(I) click chemistry or a Cu$^+$ click chemistry. Catalyst for the click chemistry can be Cu(I) salts, or Cu(I) salts made in situ by reducing Cu(II) reagent to Cu(I) reagent with a reducing reagent (Pharm Res. 2008, 25(10): 2216-2230). Known Cu(II) reagents for the click chemistry can include, but are not limited to, Cu(II)-(TBTA) complex and Cu(II) (THPTA) complex. TBTA, which is tris-[(1-benzyl-1H-1,2,3-triazol-4-yl)methyl]amine, also known as tris-(benzyltriazolylmethyl)amine, can be a stabilizing ligand for Cu(I) salts. THPTA, which is tris-(hydroxypropyltriazolylmethyl)amine, can be another example of stabilizing agent for Cu(I). Other conditions can also be accomplished to construct the 1,2,3-triazole ring from an azide and an alkyne using copper-free click chemistry, such as by the Strain-promoted Azide-Alkyne Click chemistry reaction (SPAAC, see, e.g., Chem. Commun., 2011, 47:6257-6259 and Nature, 2015, 519(7544):486-90), each of which is entirely incorporated herein by reference for all purposes.

In some cases, the present disclosure also contemplates the use of click chemistry reactions resulting in chemical linkages that are not a 1,2,3-triazole. See, e.g., U.S. Pat. Pub. 2019/0233878, which is incorporated by reference in its entirety. A range of such click chemistry reactions useful for preparing biocompatible gels are well-known in the art. See e.g., Madl and Heilshorn, "Bioorthogonal Strategies for Engineering Extracellular Matrices," Adv. Funct. Mater. 2018, 28: 1706046, which is hereby incorporated by reference herein.

An example of a click chemistry reaction useful in the compositions and methods of the present disclosure that is copper-free and does not result in a 1,2,3-triazole linkage is an Inverse-electron demand Diels-Alder (IED-DA) reaction. (See e.g., Madl and Heilshorn 2018.) As described elsewhere herein, in the IED-DA click chemistry reaction, the pair of click chemistry functional groups comprises a tetrazine group and a trans-cyclooctene (TCO) group, or a tetrazine group and a norbonene group. This reaction is copper free and results in a linkage comprising a dihydropyridazine group rather than a 1,2,3-triazole.

Other specific biorthogonal click chemistry reactions that are useful in the compositions and methods of the present disclosure, but which result in a chemical linkage other than a 1,2,3-triazole include a Diels-Alder reaction between a pair of furan and maleimide functional groups, a Staudinger ligation, and nitrile oxide cycloaddition. These click chemistry reactions and others are well-known in the art and described in e.g., Madl and Heilshorn 2018.

Accordingly, in some embodiments the copper-free click chemistry useful in forming crosslinked polymers of the present disclosure can be selected from: (a) strain-promoted azide/dibenzocyclooctyne-amine (DBCO) click chemistry; (b) inverse electron demand Diels-Alder (IED-DA) tetrazine/trans-cyclooctene (TCO) click chemistry; (c) inverse electron demand Diels-Alder (IED-DA) tetrazine/norbonene click chemistry; (d) Diels-Alder maleimide/furan click-chemistry; (e) Staudinger ligation; and (f) nitrile-oxide/norbonene cycloaddition click chemistry.

**Reagents**

In accordance with certain aspects, biological particles may be partitioned along with lysis reagents in order to release the contents of the biological particles within the partition. See, e.g., U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878, which are incorporated by reference in their entirety. Cell beads may be partitioned together with nucleic acid barcode molecules and the nucleic acid molecules of or derived from the cell bead (e.g., mRNA, cDNA, gDNA, etc.,) can be barcoded as described elsewhere herein. In some embodiments, cell beads are co-partitioned with barcode carrying beads (e.g., gel beads) and the nucleic acid molecules of or derived from the cell bead are barcoded as described elsewhere herein. In such cases, the lysis agents can be contacted with the biological particle suspension concurrently with, or immediately prior to, the introduction of the biological particles into the partitioning junction/droplet generation zone (e.g., junction **210**), such as through an additional channel or channels upstream of the channel junction. In accordance with other aspects, additionally or alternatively, biological particles may be partitioned along with other reagents, as will be described further below.

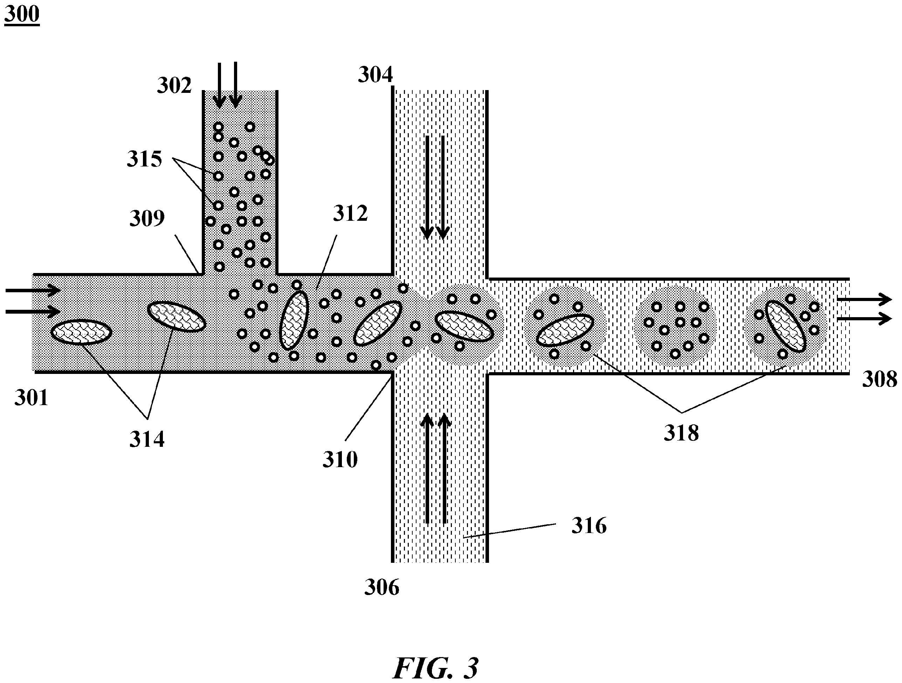

**FIG. 3** shows an example of a microfluidic channel structure **300** for co-partitioning biological particles and reagents. The channel structure **300** can include channel segments **301, 302, 304, 306** and **308**. Channel segments **301** and **302** communicate at a first channel junction **309**. Channel segments **302, 304, 306,** and **308** communicate at a second channel junction **310**.

In an example operation, the channel segment **301** may transport an aqueous fluid **312** that includes a plurality of biological particles **314** along the channel segment **301** into the second junction **310**. As an alternative or in addition to, channel segment **301** may transport beads (e.g., gel beads). The beads may comprise barcode molecules.

For example, the channel segment **301** may be connected to a reservoir comprising an aqueous suspension of biological particles **314.** Upstream of, and immediately prior to reaching, the second junction **310,** the channel segment **301** may meet the channel segment **302** at the first junction **309.** The channel segment **302** may transport a plurality of reagents **315** (e.g., lysis agents) suspended in the aqueous fluid **312** along the channel segment **302** into the first junction **309.** For example, the channel segment **302** may be connected to a reservoir comprising the reagents **315.** After the first junction **309,** the aqueous fluid **312** in the channel segment **301** can carry both the biological particles **314** and the reagents **315** towards the second junction **310.** In some instances, the aqueous fluid **312** in the channel segment **301** can include one or more reagents, which can be the same or different reagents as the reagents **315.** A second fluid **316** that is immiscible with the aqueous fluid **312** (e.g., oil) can be delivered to the second junction **310** from each of channel segments **304** and **306.** Upon meeting of the aqueous fluid **312** from the channel segment **301** and the second fluid **316** from each of channel segments **304** and **306** at the second channel junction **310,** the aqueous fluid **312** can be partitioned as discrete droplets **318** in the second fluid **316** and flow away from the second junction **310** along channel segment **308.** The channel segment **308** may deliver the discrete droplets **318** to an outlet reservoir fluidly coupled to the channel segment **308,** where they may be harvested.

The second fluid **316** can comprise an oil, such as a fluorinated oil, that includes a fluorosurfactant for stabilizing the resulting droplets, for example, inhibiting subsequent coalescence of the resulting droplets **318.**

A discrete droplet generated may include an individual biological particle **314** and/or one or more reagents **315.** In some instances, a discrete droplet generated may include a barcode carrying bead (not shown), such as via other microfluidics structures described elsewhere herein. In some instances, a discrete droplet may be unoccupied (e.g., no reagents, no biological particles).

Beneficially, when lysis reagents and biological particles are co-partitioned, the lysis reagents can facilitate the release of the contents of the biological particles within the partition. The contents released in a partition may remain discrete from the contents of other partitions.

As will be appreciated, the channel segments described herein may be coupled to any of a variety of different fluid sources or receiving components, including reservoirs, tubing, manifolds, or fluidic components of other systems. As will be appreciated, the microfluidic channel structure **300** may have other geometries. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the biological particles to cause the release of the biological particle's contents into the partitions. For example, in some cases, surfactant-based lysis solutions may be used to lyse cells, although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion based partitioning such as encapsulation of biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles, the biological particles may be exposed to an appropriate stimulus to release the biological particles or their contents from a co-partitioned microcapsule. For example, in some cases, a chemical stimulus may be co-partitioned along with an encapsulated biological particle to allow for the degradation of the microcapsule and release of the cell or its contents into the larger partition. In some cases, this stimulus may be the same as the stimulus described elsewhere herein for release of nucleic acid molecules (e.g., oligonucleotides) from their respective microcapsule (e.g., bead). In alternative aspects, this may be a different and non-overlapping stimulus, in order to allow an encapsulated biological particle to be released into a partition at a different time from the release of nucleic acid molecules into the same partition.

Additional reagents may also be co-partitioned with the biological particles, such as endonucleases to fragment a biological particle's DNA, DNA polymerase enzymes and dNTPs used to amplify the biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In an example of template switching, cDNA can be generated from reverse transcription of a template, e.g., cellular mRNA, where a reverse transcriptase with terminal transferase activity can add additional nucleotides, e.g., polyC, to the cDNA in a template independent manner. Switch oligos can include sequences complementary to the additional nucleotides, e.g., polyG. The additional nucleotides (e.g., polyC) on the cDNA can hybridize to the additional nucleotides (e.g., polyG) on the switch oligo, whereby the switch oligo can be used by the reverse transcriptase as template to further extend the cDNA. Template switching oligonucleotides may comprise a hybridization region and a template region. The hybridization region can comprise any sequence capable of hybridizing to the target. In some cases, as previously described, the hybridization region comprises a series of G bases to complement the overhanging C bases at the 3' end of a cDNA molecule. The series of G bases may comprise 1 G base, 2 G bases, 3 G bases, 4 G bases, 5 G bases or more than 5 G bases. The template sequence can comprise any sequence to be incorporated into the cDNA. In some cases, the template region comprises at least 1 (e.g., at least 2, 3, 4, 5 or more) tag sequences and/or functional sequences. Switch oligos may comprise deoxyribonucleic acids; ribonucleic acids; modified nucleic acids including 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, 2' Fluoro bases (e.g., Fluoro C, Fluoro U, Fluoro A, and Fluoro G), or any combination.

In some cases, the length of a switch oligo may be at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo may be at most about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides.

Once the contents of the cells are released into their respective partitions, the macromolecular components (e.g., macromolecular constituents of biological particles, such as RNA, DNA, or proteins) contained therein may be further processed within the partitions. In accordance with the methods and systems described herein, the macromolecular component contents of individual biological particles can be provided with unique identifiers such that, upon characterization of those macromolecular components they may be attributed as having been derived from the same biological particle or particles. The ability to attribute characteristics to individual biological particles or groups of biological particles is provided by the assignment of unique identifiers specifically to an individual biological particle or groups of biological particles. Unique identifiers, e.g., in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles.

In some aspects, this is performed by co-partitioning the individual biological particle or groups of biological particles with the unique identifiers, such as described above (with reference to **FIG. 2**). In some aspects, the unique identifiers are provided in the form of nucleic acid molecules (e.g., oligonucleotides) that comprise nucleic acid barcode sequences that may be attached to or otherwise associated with the nucleic acid contents of individual biological particle, or to other components of the biological particle, and particularly to fragments of those nucleic acids. The nucleic acid molecules are partitioned such that as between nucleic acid molecules in a given partition, the nucleic acid barcode sequences contained therein are the same, but as between different partitions, the nucleic acid molecule can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences may be present.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In some cases, the length of a barcode sequence may be about 6, 7, 8, 9, 10, 11, 12, 13, 14, 15, 16, 17, 18, 19, 20 nucleotides or longer. In some cases, the length of a barcode sequence may be at least about 6, 7, 8, 9, 10, 11, 12, 13, 14, 15, 16, 17, 18, 19, 20 nucleotides or longer. In some cases, the length of a barcode sequence may be at most about 6, 7, 8, 9, 10, 11, 12, 13, 14, 15, 16, 17, 18, 19, 20 nucleotides or shorter. These nucleotides may be completely contiguous, e.g., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be about 4, 5, 6, 7, 8, 9, 10, 11, 12, 13, 14, 15, 16 nucleotides or longer. In some cases, the barcode subsequence may be at least about 4, 5, 6, 7, 8, 9, 10, 11, 12, 13, 14, 15, 16 nucleotides or longer. In some cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned biological particles. These sequences include, e.g., targeted or random/universal amplification primer sequences for amplifying the genomic DNA from the individual biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides into partitions, e.g., droplets within microfluidic systems.

In an example, microcapsules, such as beads, are provided that each include large numbers of the above described barcoded nucleic acid molecules (e.g., barcoded oligonucleotides) releasably attached to the beads, where all of the nucleic acid molecules attached to a particular bead will include the same nucleic acid barcode sequence, but where a large number of diverse barcode sequences are represented across the population of beads used. In some embodiments, hydrogel beads, e.g., comprising polyacrylamide polymer matrices, are used as a solid support and delivery vehicle for the nucleic acid molecules into the partitions, as they are capable of carrying large numbers of nucleic acid molecules, and may be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, it may be desirable to incorporate multiple different barcodes within a given partition, either attached to a single or multiple beads within the partition. For example, in some cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

In some aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

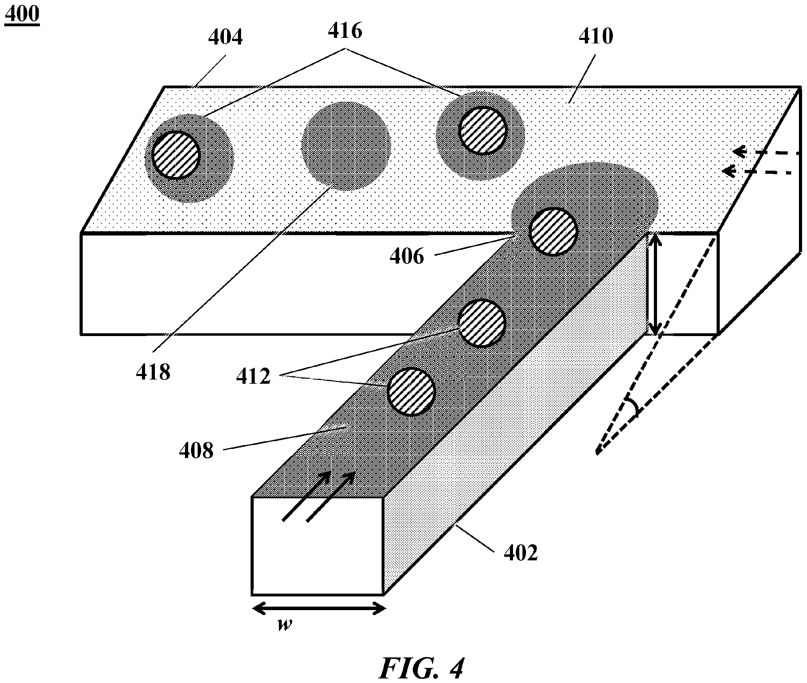

FIG. 4 shows an example of a microfluidic channel structure for the controlled partitioning of beads into discrete droplets. A channel structure **400** can include a channel segment **402** communicating at a channel junction **406** (or intersection) with a reservoir **404**. The reservoir **404** can be a chamber. Any reference to "reservoir," as used herein, can also refer to a "chamber." In operation, an aqueous fluid **408** that includes suspended beads **412** may be transported along the channel segment **402** into the junction **406** to meet a second fluid **410** that is immiscible with the aqueous fluid **408** in the reservoir **404** to create droplets **416, 418** of the aqueous fluid **408** flowing into the reservoir **404**. At the junction **406** where the aqueous fluid **408** and the second fluid **410** meet, droplets can form based on factors such as the hydrodynamic forces at the junction **406**, flow rates of the two fluids **408, 410**, fluid properties, and certain geometric parameters (e.g., $w$, $h_0$, $\alpha$, etc.) of the channel structure **400**. A plurality of droplets can be collected in the reservoir **404** by continuously injecting the aqueous fluid **408** from the channel segment **402** through the junction **406**.

A discrete droplet generated may include a bead (e.g., as in occupied droplets **416**). Alternatively, a discrete droplet generated may include more than one bead. Alternatively, a discrete droplet generated may not include any beads (e.g., as in unoccupied droplet **418**). In some instances, a discrete droplet generated may contain one or more biological particles, as described elsewhere herein. In some instances, a discrete droplet generated may comprise one or more reagents, as described elsewhere herein.

In some instances, the aqueous fluid **408** can have a substantially uniform concentration or frequency of beads **412**. The beads **412** can be introduced into the channel segment **402** from a separate channel (not shown in **FIG. 4**). The frequency of beads **412** in the channel segment **402** may be controlled by controlling the frequency in which the beads **412** are introduced into the channel segment **402** and/or the relative flow rates of the fluids in the channel segment **402** and the separate channel. In some instances, the beads can be introduced into the channel segment **402** from a plurality of different channels, and the frequency controlled accordingly.

In some instances, the aqueous fluid **408** in the channel segment **402** can comprise biological particles (e.g., described with reference to **FIGS. 1** and **2**). In some instances, the aqueous fluid **408** can have a substantially uniform concentration or frequency of biological particles. As with the beads, the biological particles can be introduced into the channel segment **402** from a separate channel. The frequency or concentration of the biological particles in the aqueous fluid **408** in the channel segment **402** may be controlled by controlling the frequency in which the biological particles are introduced into the channel segment **402** and/or the relative flow rates of the fluids in the channel segment **402** and the separate channel. In some instances, the biological particles can be introduced into the channel segment **402** from a plurality of different channels, and the frequency controlled accordingly. In some instances, a first separate channel can introduce beads and a second separate channel can introduce biological particles into the channel segment **402**. The first separate channel introducing the beads may be upstream or downstream of the second separate channel introducing the biological particles.

The second fluid **410** can comprise an oil, such as a fluorinated oil, that includes a fluorosurfactant for stabilizing the resulting droplets, for example, inhibiting subsequent coalescence of the resulting droplets.

In some instances, the second fluid **410** may not be subjected to and/or directed to any flow in or out of the reservoir **404**. For example, the second fluid **410** may be substantially stationary in the reservoir **404**. In some instances, the second fluid **410** may be subjected to flow within the reservoir **404**, but not in or out of the reservoir **404**, such as via application of pressure to the reservoir **404** and/or as affected by the incoming flow of the aqueous fluid **408** at the junction **406**. Alternatively, the second fluid **410** may be subjected and/or directed to flow in or out of the reservoir **404**. For example, the reservoir **404** can be a channel directing the second fluid **410** from upstream to downstream, transporting the generated droplets.

The channel structure **400** at or near the junction **406** may have certain geometric features that at least partly determine the sizes of the droplets formed by the channel structure **400**. The channel segment **402** can

have a height, $h_0$ and width, w, at or near the junction **406**. By way of example, the channel segment **402** can comprise a rectangular cross-section that leads to a reservoir **404** having a wider cross-section (such as in width or diameter). Alternatively, the cross-section of the channel segment **402** can be other shapes, such as a circular shape, trapezoidal shape, polygonal shape, or any other shapes. The top and bottom walls of the reservoir **404** at or near the junction **406** can be inclined at an expansion angle, $\alpha$. The expansion angle, $\alpha$, allows the tongue (portion of the aqueous fluid **408** leaving channel segment **402** at junction **406** and entering the reservoir **404** before droplet formation) to increase in depth and facilitate decrease in curvature of the intermediately formed droplet. Droplet size may decrease with increasing expansion angle. The resulting droplet radius, $R_d$, may be predicted by the following equation for the aforementioned geometric parameters of $h_0$, w, and $\alpha$:

$$R_d \approx 0.44 \left( 1 + 2.2\sqrt{\tan\alpha}\, \frac{w}{h_0} \right) \frac{h_0}{\sqrt{\tan\alpha}}$$

By way of example, for a channel structure with $w$ = 21 $\mu$m, $h$ = 21 $\mu$m, and $\alpha$ = 3°, the predicted droplet size is 121 $\mu$m. In another example, for a channel structure with $w$ = 25 $\mu$m, $h$ = 25 $\mu$m, and $\alpha$ = 5°, the predicted droplet size is 123 $\mu$m. In another example, for a channel structure with $w$ = 28 $\mu$m, $h$ = 28 $\mu$m, and $\alpha$ = 7°, the predicted droplet size is 124 $\mu$m.

In some instances, the expansion angle, $\alpha$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less. In some instances, the width, w, can be between a range of from about 100 micrometers ($\mu$m) to about 500 $\mu$m. In some instances, the width, w, can be between a range of from about 10 $\mu$m to about 200 $\mu$m. Alternatively, the width can be less than about 10 $\mu$m. Alternatively, the width can be greater than about 500 $\mu$m. In some instances, the flow rate of the aqueous fluid **408** entering the junction **406** can be between about 0.04 microliters ($\mu$L)/minute (min) and about 40 $\mu$L/min. In some instances, the flow rate of the aqueous fluid **408** entering the junction **406** can be between about 0.01 microliters ($\mu$L)/minute (min) and about 100 $\mu$L/min. Alternatively, the flow rate of the aqueous fluid **408** entering the junction **406** can be less than about 0.01 $\mu$L/min. Alternatively, the flow rate of the aqueous fluid **408** entering the junction **406** can be greater than about 40 $\mu$L/min, such as 45 $\mu$L/min, 50 $\mu$L/min, 55 $\mu$L/min, 60 $\mu$L/min, 65 $\mu$L/min, 70

$\mu$L/min, 75 $\mu$L/min, 80 $\mu$L/min, 85 $\mu$L/min, 90 $\mu$L/min, 95 $\mu$L/min, 100 $\mu$L/min, 110 $\mu$L/min , 120 $\mu$L/min , 130 $\mu$L/min , 140 $\mu$L/min , 150 $\mu$L/min, or greater. At lower flow rates, such as flow rates of about less than or equal to 10 microliters/minute, the droplet radius may not be dependent on the flow rate of the aqueous fluid **408** entering the junction **406**.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

The throughput of droplet generation can be increased by increasing the points of generation, such as increasing the number of junctions (e.g., junction **406**) between aqueous fluid **408** channel segments (e.g., channel segment **402**) and the reservoir **404**. Alternatively or in addition, the throughput of droplet generation can be increased by increasing the flow rate of the aqueous fluid **408** in the channel segment **402**.

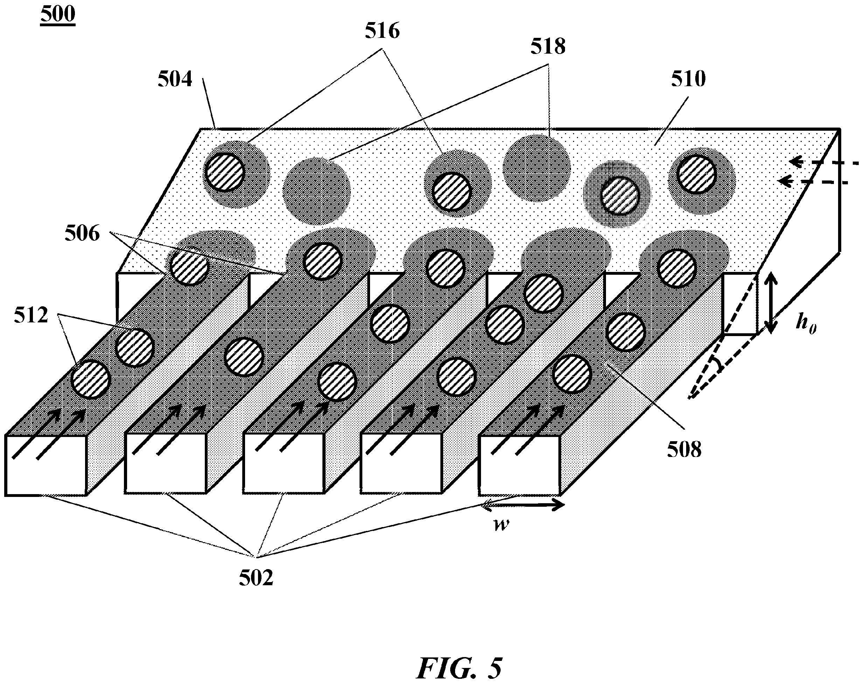

FIG. 5 shows an example of a microfluidic channel structure for increased droplet generation throughput. A microfluidic channel structure **500** can comprise a plurality of channel segments **502** and a reservoir **504**. Each of the plurality of channel segments **502** may be in fluid communication with the reservoir **504**. The channel structure **500** can comprise a plurality of channel junctions **506** between the plurality of channel segments **502** and the reservoir **504**. Each channel junction can be a point of droplet generation. The channel segment **402** from the channel structure **400** in **FIG. 4** and any description to the components thereof may correspond to a given channel segment of the plurality of channel segments **502** in channel structure **500** and any description to the corresponding components thereof. The reservoir **404** from the channel structure **400** and any description to the components thereof may correspond to the reservoir **504** from the channel structure **500** and any description to the corresponding components thereof.

Each channel segment of the plurality of channel segments **502** may comprise an aqueous fluid **508** that includes suspended beads **512**. The reservoir **504** may comprise a second fluid **510** that is immiscible with the aqueous fluid **508**. In some instances, the second fluid **510** may not be subjected to and/or directed to any flow in or out of the reservoir **504**. For example, the second fluid **510** may be substantially stationary in the reservoir **504**. In some instances, the second fluid **510** may be subjected to flow within the reservoir **504**, but not in or out of the reservoir **504**, such as via application of pressure to the reservoir **504** and/or as affected by the incoming flow of the aqueous fluid **508** at the junctions. Alternatively, the second fluid **510** may be subjected and/or directed to flow in or out of the reservoir **504**. For example, the reservoir **504** can be a channel directing the second fluid **510** from upstream to downstream, transporting the generated droplets.

In operation, the aqueous fluid **508** that includes suspended beads **512** may be transported along the plurality of channel segments **502** into the plurality of junctions **506** to meet the second fluid **510** in the reservoir **504** to create droplets **516, 518.** A droplet may form from each channel segment at each corresponding junction with the reservoir **504.** At the junction where the aqueous fluid **508** and the second fluid **510** meet, droplets can form based on factors such as the hydrodynamic forces at the junction, flow rates of the two fluids **508, 510,** fluid properties, and certain geometric parameters (e.g., $w$, $h_0$, $\alpha$, etc.) of the channel structure **500,** as described elsewhere herein. A plurality of droplets can be collected in the reservoir **504** by continuously injecting the aqueous fluid **508** from the plurality of channel segments **502** through the plurality of junctions **506.** Throughput may significantly increase with the parallel channel configuration of channel structure **500.** For example, a channel structure having five inlet channel segments comprising the aqueous fluid **508** may generate droplets five times as frequently than a channel structure having one inlet channel segment, provided that the fluid flow rate in the channel segments are substantially the same. The fluid flow rate in the different inlet channel segments may or may not be substantially the same. A channel structure may have as many parallel channel segments as is practical and allowed for the size of the reservoir. For example, the channel structure may have at least about 2, 3, 4, 5, 6, 7, 8, 9, 10, 20, 30, 40, 50, 60, 70, 80, 90, 100, 150, 500, 250, 300, 350, 400, 450, 500, 600, 700, 800, 900, 1000, 1500, 5000 or more parallel or substantially parallel channel segments.

The geometric parameters, $w$, $h_0$, and $\alpha$, may or may not be uniform for each of the channel segments in the plurality of channel segments **502.** For example, each channel segment may have the same or different widths at or near its respective channel junction with the reservoir **504.** For example, each channel segment may have the same or different height at or near its respective channel junction with the reservoir **504.** In another example, the reservoir **504** may have the same or different expansion angle at the different channel junctions with the plurality of channel segments **502.** When the geometric parameters are uniform, beneficially, droplet size may also be controlled to be uniform even with the increased throughput. In some instances, when it is desirable to have a different distribution of droplet sizes, the geometric parameters for the plurality of channel segments **502** may be varied accordingly.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

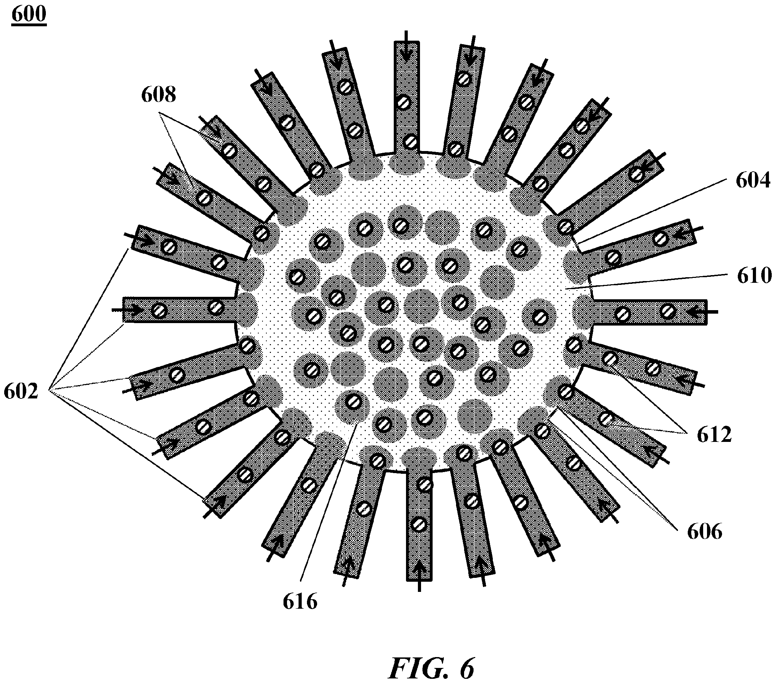

**FIG. 6** shows another example of a microfluidic channel structure for increased droplet generation throughput. A microfluidic channel structure **600** can comprise a plurality of channel segments **602** arranged generally circularly around the perimeter of a reservoir **604.** Each of the plurality of channel segments **602** may be in fluid communication with the reservoir **604.** The channel structure **600** can comprise a plurality of channel junctions **606** between the plurality of channel segments **602** and the reservoir **604.** Each channel junction can be a point of droplet generation. The channel segment **402** from the channel structure **400** in **FIG. 4** and any description to the components thereof may correspond to a given channel segment of the plurality of channel segments **602** in channel structure **600** and any description to the corresponding components thereof. The reservoir **404** from the channel structure **400** and any description to the components thereof may correspond to the reservoir **604** from the channel structure **600** and any description to the corresponding components thereof.

Each channel segment of the plurality of channel segments **602** may comprise an aqueous fluid **608** that includes suspended beads **612.** The reservoir **604** may comprise a second fluid **610** that is immiscible with the aqueous fluid **608.** In some instances, the second fluid **610** may not be subjected to and/or directed to any flow in or out of the reservoir **604.** For example, the second fluid **610** may be substantially stationary in the reservoir **604.** In some instances, the second fluid **610** may be subjected to flow within the reservoir **604,** but not in or out of the reservoir **604,** such as via application of pressure to the reservoir **604** and/or as affected by the incoming flow of the aqueous fluid **608** at the junctions. Alternatively, the second fluid **610** may be subjected and/or directed to flow in or out of the reservoir **604.** For example, the reservoir **604** can be a channel directing the second fluid **610** from upstream to downstream, transporting the generated droplets.

In operation, the aqueous fluid **608** that includes suspended beads **612** may be transported along the plurality of channel segments **602** into the plurality of junctions **606** to meet the second fluid **610** in the reservoir **604** to create a plurality of droplets **616.** A droplet may form from each channel segment at each corresponding junction with the reservoir **604.** At the junction where the aqueous fluid **608** and the second fluid **610** meet, droplets can form based on factors such as the hydrodynamic forces at the junction, flow rates of the two fluids **608, 610,** fluid properties, and certain geometric parameters (e.g., widths and heights of the channel segments **602,** expansion angle of the reservoir **604,** etc.) of the channel structure **600,** as described elsewhere herein. A plurality of droplets can be collected in the reservoir **604** by continuously injecting the aqueous fluid **608** from the plurality of channel segments **602** through the plurality of junctions **606.** Throughput may significantly increase with the substantially parallel channel configuration of the channel structure **600.** A channel structure may have as many substantially parallel channel segments as is practical and allowed for by the size of the reservoir. For example, the channel structure may have at least about 2, 3, 4, 5, 6, 7, 8, 9, 10, 20, 30, 40, 50, 60, 70, 80, 90, 100, 150, 200, 250, 300, 350, 400, 450, 500, 600, 700, 800, 900, 1000, 1500, 5000 or more parallel or substantially parallel channel segments. The plurality of channel segments may be substantially evenly spaced apart, for example, around an edge or perimeter of the reservoir. Alternatively, the spacing of the plurality of channel segments may be uneven.

The reservoir **604** may have an expansion angle, $\alpha$ (not shown in **FIG. 6**) at or near each channel junction. Each channel segment of the plurality of channel segments **602** may have a width, $w$, and a height, $h_0$, at or near the channel junction. The geometric parameters, $w$, $h_0$, and $\alpha$, may or may not be uniform for each of the channel segments in the plurality of channel segments **602**. For example, each channel segment may have the same or different widths at or near its respective channel junction with the reservoir **604**. For example, each channel segment may have the same or different height at or near its respective channel junction with the reservoir **604**.

The reservoir **604** may have the same or different expansion angle at the different channel junctions with the plurality of channel segments **602**. For example, a circular reservoir (as shown in **FIG. 6**) may have a conical, dome-like, or hemispherical ceiling (e.g., top wall) to provide the same or substantially same expansion angle for each channel segments **602** at or near the plurality of channel junctions **606**. When the geometric parameters are uniform, beneficially, resulting droplet size may be controlled to be uniform even with the increased throughput. In some instances, when it is desirable to have a different distribution of droplet sizes, the geometric parameters for the plurality of channel segments **602** may be varied accordingly.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size. The beads and/or biological particle injected into the droplets may or may not have uniform size.

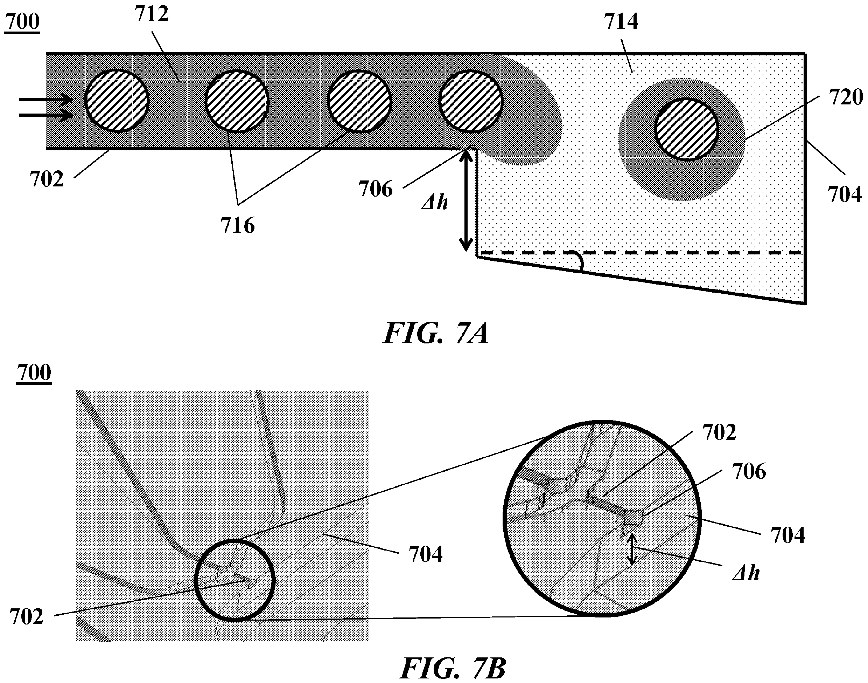

**FIG. 7A** shows a cross-section view of another example of a microfluidic channel structure with a geometric feature for controlled partitioning. A channel structure **700** can include a channel segment **702** communicating at a channel junction **706** (or intersection) with a reservoir **704**. In some instances, the channel structure **700** and one or more of its components can correspond to the channel structure **100** and one or more of its components. **FIG. 7B** shows a perspective view of the channel structure **700** of **FIG. 7A.**

An aqueous fluid **712** comprising a plurality of particles **716** may be transported along the channel segment **702** into the junction **706** to meet a second fluid **714** (e.g., oil, etc.) that is immiscible with the aqueous fluid **712** in the reservoir **704** to create droplets **720** of the aqueous fluid **712** flowing into the reservoir **704**. At the junction **706** where the aqueous fluid **712** and the second fluid **714** meet, droplets can form based on factors such as the hydrodynamic forces at the junction **706,** relative flow rates of the two fluids **712, 714**, fluid properties, and certain geometric parameters (e.g., $\Delta h$, etc.) of the channel structure **700**. A plurality of droplets can be collected in the reservoir **704** by continuously injecting the aqueous fluid **712** from the channel segment **702** at the junction **706**.

A discrete droplet generated may comprise one or more particles of the plurality of particles **716**. As described elsewhere herein, a particle may be any particle, such as a bead, cell bead, gel bead, biological particle, macromolecular constituents of biological particle, or other particles. Alternatively, a discrete droplet generated may not include any particles.

In some instances, the aqueous fluid **712** can have a substantially uniform concentration or frequency of particles **716**. As described elsewhere herein (e.g., with reference to **FIG. 4**), the particles **716** (e.g., beads) can be introduced into the channel segment **702** from a separate channel (not shown in **FIG. 7A** or **7B**). The frequency of particles **716** in the channel segment **702** may be controlled by controlling the frequency in which the particles **716** are introduced into the channel segment **702** and/or the relative flow rates of the fluids in the channel segment **702** and the separate channel. In some instances, the particles **716** can be introduced into the channel segment **702** from a plurality of different channels, and the frequency controlled accordingly. In some instances, different particles may be introduced via separate channels. For example, a first separate channel can introduce beads and a second separate channel can introduce biological particles into the channel segment **702**. The first separate channel introducing the beads may be upstream or downstream of the second separate channel introducing the biological particles.

In some instances, the second fluid **714** may not be subjected to and/or directed to any flow in or out of the reservoir **704**. For example, the second fluid **714** may be substantially stationary in the reservoir **704**. In some instances, the second fluid **714** may be subjected to flow within the reservoir **704**, but not in or out of the reservoir **704**, such as via application of pressure to the reservoir **704** and/or as affected by the incoming flow of the aqueous fluid **712** at the junction **706**. Alternatively, the second fluid **714** may be subjected and/or directed to flow in or out of the reservoir **704**. For example, the reservoir **704** can be a channel directing the second fluid **714** from upstream to downstream, transporting the generated droplets.

The channel structure **700** at or near the junction **706** may have certain geometric features that at least partly determine the sizes and/or shapes of the droplets formed by the channel structure **700**. The channel segment **702** can have a first cross-section height, $h_1$, and the reservoir **704** can have a second cross-section height, $h_2$. The first cross-section height, $h_1$, and the second cross-section height, $h_2$, may be different, such that at the junction **706**, there is a height difference of $\Delta h$. The second cross-section height, $h_2$, may be greater than the first cross-section height, $h_1$. In some instances, the reservoir may thereafter gradually increase in cross-section height, for example, the more distant it is from the junction **706**. In some instances, the cross-section height of the reservoir may increase in accordance with expansion angle, $\beta$, at or near the junction **706**. The height difference, $\Delta h$, and/or expansion angle, $\beta$, can allow the tongue (portion of the aqueous fluid **712** leaving channel segment **702** at junction **706** and entering the reservoir **704** before droplet formation) to increase in depth and facilitate decrease in curvature of the intermediately formed droplet. For example, droplet size may decrease with increasing height difference and/or increasing expansion angle.

The height difference, $\Delta h$, can be at least about 1 $\mu$m. Alternatively, the height difference can be at least about 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500 $\mu$m or more. Alternatively, the height difference can be at most about 500, 400, 300, 200, 100, 90, 80, 70, 60, 50, 45, 40, 35, 30, 25, 20, 19, 18, 17, 16, 15, 14, 13, 12, 11, 10, 9, 8, 7, 6, 5, 4, 3, 2, 1 $\mu$m or less. In some instances, the expansion angle, $\beta$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less.

In some instances, the flow rate of the aqueous fluid **712** entering the junction **706** can be between about 0.04 microliters ($\mu$L)/minute (min) and about 40 $\mu$L/min. In some instances, the flow rate of the aqueous fluid **712** entering the junction **706** can be between about 0.01 microliters ($\mu$L)/minute (min) and about 100 $\mu$L/min. Alternatively, the flow rate of the aqueous fluid **712** entering the junction **706** can be less than about 0.01 $\mu$L/min. Alternatively, the flow rate of the aqueous fluid **712** entering the junction **706** can be greater than about 40 $\mu$L/min, such as 45 $\mu$L/min, 50 $\mu$L/min, 55 $\mu$L/min, 60 $\mu$L/min, 65 $\mu$L/min, 70 $\mu$L/min, 75 $\mu$L/min, 80 $\mu$L/min, 85 $\mu$L/min, 90 $\mu$L/min, 95 $\mu$L/min, 100 $\mu$L/min, 110 $\mu$L/min , 120 $\mu$L/min , 130 $\mu$L/min , 140 $\mu$L/min , 150 $\mu$L/min, or greater. At lower flow rates, such as flow rates of about less than or equal to 10 microliters/minute, the droplet radius may not be dependent on the flow rate of the aqueous fluid **712** entering the junction **706**. The second fluid **714** may be stationary, or substantially stationary, in the reservoir **704**. Alternatively, the second fluid **714** may be flowing, such as at the above flow rates described for the aqueous fluid **712**.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

While **FIGS. 7A** and **7B** illustrate the height difference, $\Delta h$, being abrupt at the junction **706** (e.g., a step increase), the height difference may increase gradually (e.g., from about 0 $\mu$m to a maximum height difference). Alternatively, the height difference may decrease gradually (e.g., taper) from a maximum height difference. A gradual increase or decrease in height difference, as used herein, may refer to a continuous incremental increase or decrease in height difference, wherein an angle between any one differential segment of a height profile and an immediately adjacent differential segment of the height profile is greater than 90°. For example, at the junction **706**, a bottom wall of the channel and a bottom wall of the reservoir can meet at an angle greater than 90°. Alternatively or in addition, a top wall (e.g., ceiling) of the channel and a top wall (e.g., ceiling) of the reservoir can meet an angle greater than 90°. A gradual increase or decrease may be linear or non-linear (e.g., exponential, sinusoidal, etc.). Alternatively or in addition, the height difference may variably increase and/or decrease linearly or non-linearly. While **FIGS. 7A** and **7B** illustrate the expanding reservoir cross-section height as linear (e.g., constant expansion angle, $\beta$), the cross-section height may expand non-linearly. For example, the reservoir may be defined at least partially by a dome-like (e.g., hemispherical) shape having variable expansion angles. The cross-section height may expand in any shape.

The channel networks, e.g., as described above or elsewhere herein, can be fluidly coupled to appropriate fluidic components. For example, the inlet channel segments are fluidly coupled to appropriate sources of the materials they are to deliver to a channel junction. These sources may include any of a variety of different fluidic components, from simple reservoirs defined in or connected to a body structure of a microfluidic device, to fluid conduits that deliver fluids from off-device sources, manifolds, fluid flow units (e.g., actuators, pumps, compressors) or the like. Likewise, the outlet channel segment (e.g., channel segment **208,** reservoir **604,** etc.) may be fluidly coupled to a receiving vessel or conduit for the partitioned cells for subsequent processing. Again, this may be a reservoir defined in the body of a microfluidic device, or it may be a fluidic conduit for delivering the partitioned cells to a subsequent process operation, instrument or component.

The methods and systems described herein may be used to greatly increase the efficiency of single cell applications and/or other applications receiving droplet-based input. For example, following the sorting of occupied cells and/or appropriately-sized cells, subsequent operations that can be performed can include reverse transcription in a partition (e.g., gel beads in emulsion), generation of amplification products, purification (e.g., via solid phase reversible immobilization (SPRI)), further processing (e.g., shearing, ligation of functional sequences, and subsequent amplification (e.g., via PCR)), enrichment, library construction, and/or sequencing, e.g., as shown in **FIG. 17B.** These operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA from cells. Alternatively, rRNA removal agents may be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, may be subject to sequencing for sequence analysis. In some cases, amplification may be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, e.g., in tracing contamination or the like.

Partitions comprising a barcode bead (e.g., a gel bead) associated with barcode molecules and a bead encapsulating cellular constituents (e.g., a cell bead) such as cellular nucleic acids can be useful in constituent analysis as is described in U.S. Patent Publication No. 2018/0216162, which is herein incorporated by reference in its entirety for all purposes.

## Computer systems

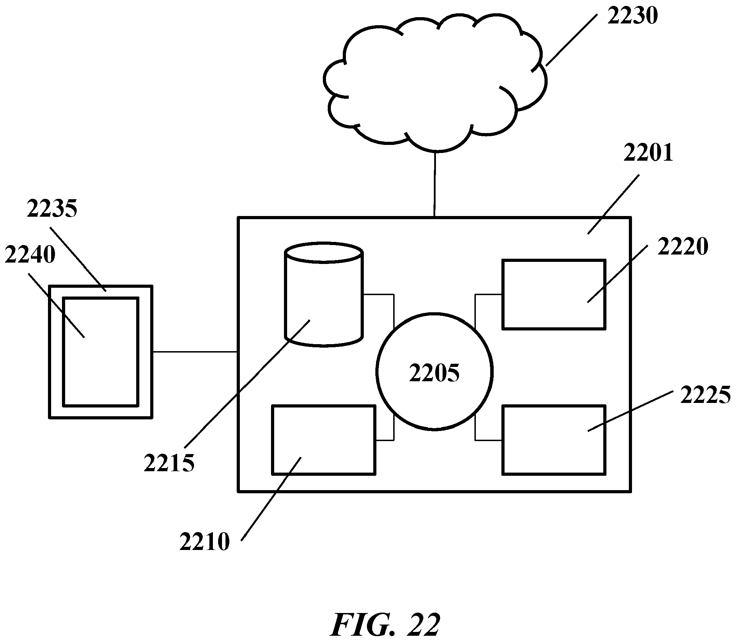

The present disclosure provides computer systems that are programmed to implement methods of the disclosure. **FIG. 22** shows a computer system **2201** that is programmed or otherwise configured to (i) control a microfluidics system (e.g., fluid flow), (ii) sort occupied droplets from unoccupied droplets, (iii) polymerize droplets, (iv) partition cell beads or cells into partitions (e.g., droplets or wells), (v) lysate cells and cell beads, (vi) perform sequencing applications, (vii) generate and maintain libraries of cytokine or other analyte specific antibody barcode sequences, MHC multimer barcode sequences, cell surface protein barcode sequences, and cDNAs generated from mRNAs respectively (vi) analyze such libraries. The computer system **2201** can regulate various aspects of the present disclosure, such as, for example, regulating fluid flow rate in one or more channels in a microfluidic structure, regulating polymerization application units, regulating sequence application unit, etc. The computer system **2201** can be an electronic device of a user or a computer system that is remotely located with respect to the electronic device. The electronic device can be a mobile electronic device.

The computer system **2201** includes a central processing unit (CPU, also "processor" and "computer processor" herein) **2205,** which can be a single core or multi core processor, or a plurality of processors for parallel processing. The computer system **2201** also includes memory or memory location **2210** (e.g., random-access memory, read-only memory, flash memory), electronic storage unit **2215** (e.g., hard disk), communication interface **2220** (e.g., network adapter) for communicating with one or more other systems, and peripheral devices **2225,** such as cache, other memory, data storage and/or electronic display adapters. The memory **2210,** storage unit **2215,** interface **2220** and peripheral devices **2225** are in communication with the CPU **2205** through a communication bus (solid lines), such as a motherboard. The storage unit **2215** can be a data storage unit (or data repository) for storing data. The computer system **2201** can be operatively coupled to a computer network ("network") **2230** with the aid of the communication interface **2220.** The network **2230** can be the Internet, an internet and/or extranet, or an intranet and/or extranet that is in communication with the Internet. The network **2230** in some cases is a telecommunication and/or data network. The network **2230** can include one or more computer servers, which can enable distributed computing, such as cloud computing. The network **2230,** in some cases with the aid of the computer system **2201,** can implement a peer-to-peer network, which may enable devices coupled to the computer system **2201** to behave as a client or a server.

The CPU **2205** can execute a sequence of machine-readable instructions, which can be embodied in a program or software. The instructions may be stored in a memory location, such as the memory **2210.** The instructions can be directed to the CPU **2205,** which can subsequently program or otherwise configure the CPU **2205** to implement methods of the present disclosure. Examples of operations performed by the CPU **2205** can include fetch, decode, execute, and writeback.

The CPU **2205** can be part of a circuit, such as an integrated circuit. One or more other components of the system **2201** can be included in the circuit. In some cases, the circuit is an application specific integrated circuit (ASIC).

The storage unit **2215** can store files, such as drivers, libraries and saved programs. The storage unit **2215** can store user data, e.g., user preferences and user programs. The computer system **2201** in some cases can include one or more additional data storage units that are external to the computer system **2201,** such as located on a remote server that is in communication with the computer system **2201** through an intranet or the Internet.

The computer system **2201** can communicate with one or more remote computer systems through the network **2230.** For instance, the computer system **2201** can communicate with a remote computer system of a user (e.g., operator). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system **2201** via the network **2230.**

Methods as described herein can be implemented by way of machine (e.g., computer processor) executable code stored on an electronic storage location of the computer system **2201,** such as, for example, on the memory **2210** or electronic storage unit **2215.** The machine executable or machine readable code can be provided in the form of software. During use, the code can be executed by the processor **2205.** In some cases, the code can be retrieved from the storage unit **2215** and stored on the memory **2210** for ready access by the processor **2205.** In some situations, the electronic storage unit **2215** can be precluded, and machine-executable instructions are stored on memory **2210.**

The code can be pre-compiled and configured for use with a machine having a processor adapted to execute the code, or can be compiled during runtime. The code can be supplied in a programming language that can be selected to enable the code to execute in a pre-compiled or as-compiled fashion.

Aspects of the systems and methods provided herein, such as the computer system **2201,** can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **2201** can include or be in communication with an electronic display 1935 that comprises a user interface (UI) **2240** for providing, for example, results of sequencing analysis, etc. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit **2205.** The algorithm can, for example, perform nucleotide sequence amplification, sequencing sorting based on barcode sizes, sequencing amplified barcode sequences, analyzing sequencing data, etc.

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) from a single cell. For example, a biological particle (e.g., a cell or cell bead) is partitioned in a partition (e.g., droplet), and multiple analytes from the biological particle are processed for subsequent processing. The multiple analytes may be from the single cell. This may enable, for example, simultaneous proteomic, transcriptomic and genomic analysis of the cell.

## EXEMPLARY EMBODIMENTS

Among the provided embodiments are:
A method of processing a secreted molecule from a cell, comprising:

(a) coupling the secreted molecule to a first polypeptide coupled to a surface of the cell to form a first conjugate; and
(b) coupling a second polypeptide to the secreted molecule to form a second conjugate, wherein the second polypeptide comprises a nucleic acid reporter molecule comprising a first barcode sequence.

The method of embodiment 1, further comprising, prior to (a), incubating the cell with the first polypeptide under a sufficient condition such that the first polypeptide couples to the surface of the cell.

The method of any one of embodiments 1-2, further comprising, prior to (b), stimulating the cell to induce secretion of the secreted molecule.

The method of any one of embodiments 1-3, wherein the cell is stimulated with an antigen.

The method of embodiment 4, wherein the antigen is a Pattern recognition receptor (PRR) ligand.

The method of embodiment 5, wherein the PRR ligand is a Toll-like receptors (TLR) ligand, a NOD-like receptor (NLRs) ligand, a RIG-I-like receptor (RLR) ligand, a C-type lectin receptor (CLR) ligand, or a cytosolic dsDNA sensor (CDS) ligand.

The method of embodiment 4, wherein the antigen is a lipopolysaccharide (LPS), a double-stranded DNA (dsDNA), a double-stranded RNA (dsRNA), a synthetic dsRNA, or CpG oligodeoxynucleotides (CpG ODN).

The method of embodiment 7, wherein the synthetic dsRNA is polyinosinicpolycytidylic acid (poly I:C) or polyadenylic-polyuridylic acid (poly(A:U).

The method of any one of embodiments 3-8, further comprising providing a plurality of antigens to the cell at a concentration that is sufficient to induce secretion of the secreted molecule.

The method of embodiment 9, wherein the plurality of antigens is provided to the cell for a length of time that is sufficient to induce the secretion of the secreted molecule.

The method of any one of embodiments 3-10, wherein an antigen of the plurality of antigens is coupled to a major histocompatibility complex (MHC) molecule.

The method of embodiment 11, wherein the MHC molecule is an MHC multimer.

The method of any one of embodiments 11-12, wherein the MHC multimer comprises a dextran polymer.

The method of any one of embodiments 11-13, wherein the MHC molecule is present in an antigen presenting cell.

The method of any one of embodiments 1-14, further comprising contacting the cell with one or more co-stimulatory molecules.

The method of embodiment 15, wherein said one or more co-stimulatory molecules comprises one or more antibodies.

The method of embodiment 16, wherein said one or more antibodies are anti-CD3 or anti-CD28 antibodies.

The method of any one of embodiment 15-17, wherein said one or more co-stimulatory molecules comprises one or more cytokines.

The method of embodiment 18, wherein said one or more cytokines comprises an interleukin.

The method of any one of embodiment 15-19, wherein said one or more co-stimulatory molecules are present on an antigen presenting cell.

The method of any one of embodiment 1-20, further comprising co-partitioning a plurality of nucleic acid barcode molecules and the cell comprising the first and the second conjugate into a partition, wherein each nucleic acid molecule of the plurality of nucleic acid molecules comprises a second barcode sequence, and wherein the second barcode sequence is different from the first barcode sequence.

The method of embodiment 21, wherein the plurality of nucleic acid barcode molecules is attached to a bead.

The method of embodiment 22, wherein the plurality of nucleic acid barcode molecules is releasably attached to said bead.

The method of embodiment 23, wherein the plurality of nucleic acid barcode molecules is releasably attached to the bead via a labile bond.

The method of embodiment 23 or 24, wherein the plurality of nucleic acid barcode molecules is releasable from the bead upon application of a stimulus.

The method of embodiment 25, wherein the stimulus is a thermal stimulus, chemical stimulus, biological stimulus, or a photo-stimulus.

The method of embodiment 26, wherein the chemical stimulus is a reducing agent.

The method of embodiment 24, wherein the labile bond is a disulfide bond.

The method of any one of embodiments 22-28, further comprising releasing nucleic acid barcode molecules of the plurality of nucleic acid barcode molecules.

The method of any one of embodiments 22-29, wherein the bead is a gel bead.

The method of embodiment 29, wherein the bead is a degradable gel bead.

The method of embodiment 30, wherein the gel bead is degradable upon application of a stimulus.

The method of embodiment 25, wherein the stimulus is a thermal stimulus, chemical stimulus, biological stimulus, or a photo-stimulus.

The method of embodiment 26, wherein the chemical stimulus is a reducing agent.

The method of any one of embodiments 21-23, wherein the partition is a droplet or a well.

The method of any one of embodiments 21-24, further comprising lysing the cell.

The method of embodiment 25, further comprising releasing a messenger ribonucleic acid (mRNA) molecule from the cell.

The method of any one or embodiments 1-26, further comprising using a nucleic acid barcode molecule of the plurality of nucleic acid barcode molecules and the reporter molecule to generate a first barcoded nucleic acid molecule comprising a sequence corresponding to the first barcode sequence and a sequence corresponding to the second barcode sequence.

The method of embodiment 38, wherein the nucleic acid barcode molecule comprises a sequence complementary to a sequence in the reporter molecule.

The method of embodiment 39, further comprising hybridizing the nucleic acid barcode molecule to the reporter molecule and performing a nucleic acid reaction to generate the first barcoded nucleic acid molecule.

The method of embodiment 40, wherein the nucleic acid reaction is a ligation reaction or a nucleic acid extension reaction.

The method of any one or embodiments 26-27, further comprising using the mRNA molecule and a nucleic acid barcode molecule of the plurality of nucleic acid barcode molecules to generate a second barcoded nucleic acid molecule comprising a sequence corresponding to the mRNA molecule and a sequence corresponding to the second barcode sequence

The method of embodiment 42, wherein the nucleic acid barcode molecule comprises a sequence complementary to a sequence in the mRNA molecule.

The method of embodiment 43, further comprising hybridizing the nucleic acid barcode molecule to the mRNA molecule or a cDNA thereof and performing a nucleic acid reaction to generate the barcoded nucleic acid molecule.

The method of embodiment 44, wherein the nucleic acid reaction is a reverse transcription reaction, a ligation reaction, or a nucleic acid extension reaction.

The method of any one of embodiments 1-29, further comprising amplifying the reporter molecule and/or a cDNA molecule derived from the mRNA molecule.

The method of embodiment 30, wherein the amplification comprises a polymerase chain reaction (PCR).

The method of any one of embodiments 46-47, wherein the amplification is isothermal.

The method of any one of embodiments 1-31, further comprising sorting the nucleic acid molecule and/or cDNA molecule according to their sizes.

The method of any one of embodiments 1-32, further comprising sequencing the first barcoded nucleic acid molecule or a derivative thereof and/or the second barcoded nucleic acid molecule or a derivative thereof.

The method of any one of embodiments 42-50, further comprising performing one or more nucleic acid reactions to add one or more functional sequences to the first barcoded nucleic acid molecule and/or the second barcoded nucleic acid molecule.

The method of embodiment 51, wherein the one or more functional sequences are a primer sequence, a sequencing primer sequence, or a sequence configured to attach to a flow cell of a sequencer.

The method of any one of embodiments 1-52, wherein the reporter molecule comprises one or more functional sequences selected from the group consisting of a primer sequence, a sequencing primer sequence, a sequence configured to attach to a flow cell of a sequencer, and a unique molecular index (UMI).

The method of any one of embodiments 21-53, wherein the plurality of nucleic acid barcode molecules each comprise one or more functional sequences selected from the group consisting of a primer sequence, a sequencing primer sequence, a partial sequencing primer sequence, a sequence configured to attach to a flow cell of a sequencer, and a unique molecular index (UMI).

The method of any one of embodiments 1-33, further comprising identifying the first barcode sequence and the second barcode sequence and associating the secreted molecule and/or the mRNA molecule with the cell.

The method of any one of embodiments 1-34, wherein the secreted molecule is a cytokine.

The method of any one of embodiments 1-35, wherein the first polypeptide is coupled to the cell via a cell surface protein.

The method of any one of embodiments 1-36, wherein the first polypeptide comprises a first antibody or antibody fragment and a second antibody of antibody fragment, wherein the first antibody or antibody fragment and a second antibody of antibody fragment are associated.

The method of any one of embodiments 1-37, wherein the first antibody or antibody fragment is capable of coupling to the secreted molecule.

The method of any one of embodiments 1-38, wherein the second antibody of antibody fragment is capable of coupling to the surface of the cell.

The method of any one of embodiments 1-39, wherein the second polypeptide is an antibody or antibody fragment.

The method of any one of embodiments 1-61, further comprising contacting the cell with a plurality of first polypeptide molecules and, prior to (b), removing first polypeptide molecules not bound to the surface of the cell.

The method of any one of embodiments 1-62, further comprising contacting the cell with a plurality of second polypeptide molecules and, subsequent to (b), removing second polypeptide molecules not bound to the secreted molecule.

A method of processing a molecule from a cell, comprising:

(a) generating a cell bead, wherein the cell bead comprises a cell encapsulated by a polymer matrix, wherein the polymer matrix comprises a plurality of first polypeptides; and
(b) coupling a molecule secreted from the cell to a first polypeptide of the plurality of first polypeptides to form a first conjugate.

The method of embodiment 41, wherein the polymer matrix is a hydrogel matrix.

The method of embodiment 41, wherein the polymer matrix comprises collagen, laminin, and/or fibronectin.

The method of any one of embodiments 64-66, wherein the plurality of first polypeptides is coupled to a polymer backbone of the polymer matrix.

The method of any one of embodiments 64-66, further comprising, prior to (b), stimulating the cell bead containing the cell to induce secretion of the molecule from the cell.

The method of any one of embodiments 64-66, wherein the cell bead is stimulated with an antigen.

The method of embodiment 69, wherein the antigen is a Pattern recognition receptor (PRR) ligand.

The method of embodiment 69, wherein the PRR ligand is a Toll-like receptors (TLR) ligand, a NOD-like receptor (NLRs) ligand, a RIG-I-like receptor (RLR) ligand, a C-type lectin receptor (CLR) ligand, or a cytosolic dsDNA sensor (CDS) ligand.

The method of embodiment 69, wherein the antigen is a lipopolysaccharide (LPS), a double-stranded DNA (dsDNA), a double-stranded RNA (dsRNA), a synthetic dsRNA, or CpG oligodeoxynucleotides (CpG ODN).

The method of embodiment 72, wherein the synthetic dsRNA is polyinosinicpolycytidylic acid (poly I:C) or polyadenylic-polyuridylic acid (poly(A:U)).

The method of any one of embodiments 64-73, further comprising providing a plurality of antigens to the cell bead at a concentration that is sufficient to induce secretion of the secreted molecule.

The method of any one of embodiments 64-74, wherein the plurality of antigens is provided to the cell bead for a length of time that is sufficient to induce the secretion of the secreted molecule.

The method of any one of embodiments 64-75, wherein an antigen of the plurality of antigens is coupled to a major histocompatibility complex (MHC) molecule.

The method of embodiment 76, wherein the MHC molecule is an MHC multimer.

The method of any one of embodiments 76-77, wherein the MHC multimer comprises a dextran polymer.

The method of any one of embodiments 76-78, wherein the MHC molecule is present in an antigen presenting cell.

The method of any one of embodiments 64-79, further comprising contacting the cell bead with one or more co-stimulatory molecules.

The method of embodiment 80, wherein said one or more co-stimulatory molecules comprises one or more antibodies.

The method of embodiment 81, wherein said one or more antibodies are anti-CD3 or anti-CD28 antibodies.

The method of any one of embodiment 80-82, wherein said one or more co-stimulatory molecules comprises one or more cytokines.

The method of embodiment 83, wherein said one or more cytokines comprises an interleukin.

The method of any one of embodiment 80-84, wherein said one or more co-stimulatory molecules are present on an antigen presenting cell.

The method of any one of embodiments 64-85, further comprising coupling a second polypeptide to the molecule secreted from the cell to form a second conjugate, wherein the second polypeptide comprises a nucleic acid reporter molecule comprising a first barcode sequence.

The method of embodiment 86, further comprising removing unbound second polypeptide molecules from the cell bead.

The method of embodiment 64, subsequent to (b), further comprising (c) partially digesting the cell bead with an enzyme, thereby generating a partially digested cell bead.

The method of embodiment 88, the enzyme is collagenase or dispase.

The method of embodiment 88, further comprising contacting a plurality of second polypeptides comprising a barcode to the partially digested cell bead, under a condition so that a second polypeptide from the plurality of second polypeptides couples to the molecule secreted from the cell.

The method of any one of embodiment 64-90, further comprising co-partitioning a plurality of nucleic acid barcode molecules and the cell bead comprising the first and the second conjugate into a partition, wherein each nucleic acid barcode molecule of the plurality of nucleic acid barcode molecules comprises a second barcode sequence, and wherein the second barcode sequence is different from the first barcode sequence.

The method of embodiment 91, wherein the plurality of nucleic acid barcode molecules is attached to a bead.

The method of embodiment 91, wherein the plurality of nucleic acid barcode molecules is releasably attached to said bead.

The method of embodiment 91, wherein the plurality of nucleic acid barcode molecules is releasably attached to the bead via a labile bond.

The method of embodiment 91 or 92, wherein the plurality of nucleic acid barcode molecules is releasable from the bead upon application of a stimulus.

The method of embodiment 95, wherein the stimulus is a thermal stimulus, chemical stimulus, biological stimulus, or a photo-stimulus.

The method of embodiment 96, wherein the chemical stimulus is a reducing agent.

The method of embodiment 94, wherein the labile bond is a disulfide bond.

The method of any one of embodiments 93-98, further comprising releasing nucleic acid barcode molecules of the plurality of nucleic acid barcode molecules.

The method of any one of embodiments 92-99, wherein the bead is a gel bead.

The method of embodiment 100, wherein the bead is a degradable gel bead.

The method of embodiment 101, wherein the gel bead is degradable upon application of a stimulus.

The method of embodiment 102, wherein the stimulus is a thermal stimulus, chemical stimulus, biological stimulus, or a photo-stimulus.

The method of embodiment 103, wherein the chemical stimulus is a reducing agent.

The method of any one of embodiments 91-104, wherein the partition is a droplet or a well.

The method of any one of embodiments 91-104, further comprising degrading the cell bead.

The method of any one of embodiments 91-104, further comprising lysing the cell.

The method of embodiment 107, further comprising releasing a messenger ribonucleic acid (mRNA) molecule from the cell.

The method of any one or embodiments 91-108, further comprising using a nucleic acid barcode molecule of the plurality of nucleic acid barcode molecules and the reporter molecule to generate a first barcoded nucleic acid molecule comprising a sequence corresponding to the first barcode sequence and a sequence corresponding to the second barcode sequence.

The method of embodiment 109, wherein the nucleic acid barcode molecule comprises a sequence complementary to a sequence in the reporter molecule.

The method of embodiment 110, further comprising hybridizing the nucleic acid barcode molecule to the reporter molecule and performing a nucleic acid reaction to generate the first barcoded nucleic acid molecule.

The method of embodiment 111, wherein the nucleic acid reaction is a ligation reaction or a nucleic acid extension reaction.

The method of any one or embodiments 91-108, further comprising using the mRNA molecule and a nucleic acid barcode molecule of the plurality of nucleic acid barcode molecules to generate a second barcoded nucleic acid molecule comprising a sequence corresponding to the mRNA molecule and a sequence corresponding to the second barcode sequence.

The method of embodiment 113, wherein the nucleic acid barcode molecule comprises a sequence complementary to a sequence in the mRNA molecule.

The method of embodiment 114, further comprising hybridizing the nucleic acid barcode molecule to the mRNA molecule or a cDNA thereof and performing a nucleic acid reaction to generate the barcoded nucleic acid molecule.

The method of embodiment 115, wherein the nucleic acid reaction is a reverse transcription reaction, a ligation reaction, or a nucleic acid extension reaction.

The method of any one of embodiments 41-73, further comprising amplifying the reporter molecule and/or a cDNA molecule derived from the mRNA molecule.

The method of embodiment 117, wherein the amplification comprises a polymerase chain reaction (PCR).

The method of any one of embodiments 118, wherein the amplification is isothermal.

The method of any one of embodiments 117, further comprising sorting the nucleic acid molecule and/or cDNA molecule according to their sizes.

The method of any one of embodiments 64-120, further comprising sequencing the first barcoded nucleic acid molecule or a derivative thereof and/or the second barcoded nucleic acid molecule or a derivative thereof.

The method of any one of embodiments 109-121, further comprising performing one or more nucleic acid reactions to add one or more functional sequences to the first barcoded nucleic acid molecule and/or the second barcoded nucleic acid molecule.

The method of embodiment 122, wherein the one or more functional sequences are a primer sequence, a sequencing primer sequence, or a sequence configured to attach to a flow cell of a sequencer.

The method of any one of embodiments 64-123, wherein the reporter molecule comprises one or more functional sequences selected from the group consisting of a primer sequence, a sequencing primer sequence, a sequence configured to attach to a flow cell of a sequencer, and a unique molecular index (UMI).

The method of any one of embodiments 91-124, wherein the plurality of nucleic acid barcode molecules each comprise one or more functional sequences selected from the group consisting of a primer sequence, a sequencing primer sequence, a partial sequencing primer sequence, a sequence configured to attach to a flow cell of a sequencer, and a unique molecular index (UMI).

The method of any one of embodiments 64-125, further comprising identifying the first barcode sequence and the second barcode sequence and associating the secreted molecule and/or the mRNA molecule with the cell.

The method of any one of embodiments 64-126, wherein the secreted molecule is a cytokine.

The method of any one of embodiments 64-127, wherein the second polypeptide is an antibody or antibody fragment.

The method of any one of embodiments 11-14 and 76-79, wherein the MHC molecule comprises a nucleic acid molecule comprising a third barcode sequence.

The method of embodiment 129, wherein the third barcode sequence is different from the first barcode sequence or the second barcode sequence.

**EXAMPLES**

**Example 1: Barcoding Analytes from a Single Cell**

This example illustrates the production and identification of barcoded, secreted analytes from cells *in vitro,* using a capture agent.

Cells (e.g., immune cells) are isolated from a subject. The cells are incubated with a solution of capture agents (e.g., first polypeptides) under a sufficient condition so that a plurality of capture agents (a first binding agent) bind to the surface of each cell. The capture agent is comprised of two different polypeptides (e.g., antibodies). The first polypeptide is the analyte-specific polypeptide, that may be directly or indirectly linked to, conjugated to, or fused to a second polypeptide that targets a cell surface protein, which attaches the capture agent to the cell. It is possible to alter either of these polypeptides so that any cell surface marker, or secreted protein, may be captured. The capture reagent may also comprise multiple polypeptides that target a cell surface protein (or proteins) and/or multiple antibodies that target a secreted cell protein (or proteins). The analyte-specific capture polypeptide, may be conjugated to a known DNA oligonucleotide sequence (e.g., reporter barcode sequence). After incubating with the solution of capture agents, the cells are stimulated with specific antigens or other suitable stimuli under a sufficient condition to allow secretion of molecules (e.g., cytokines or other intracellular analytes), as shown herein in. The antigen is optionally bound to a known DNA oligonucleotide barcoded MHC multimer.

Further, the cells are incubated with a plurality of barcoded reporter agents with reporter molecules (e.g., barcoded second polypeptides) that have specific affinity to the secreted molecules (e.g., cytokines or other secreted intracellular analytes). Reporter agents specific to the secreted molecule of interest may contain a fluorophore, chromophore, heavy metal, DNA oligonucleotide or combinations thereof. Furthermore, the coupling chemistry and type of covalent bond between the reporter agent and these labels may be altered. Optionally, DNA oligonucleotide barcoded antibodies specific to cell surface proteins are also added at this stage. The secreted molecules bind to the previously described analyte-specific capture agent located on the cell surface forming a complex thereon.

In some examples, cells are partitioned (e.g., into emulsion droplets) and partitions may comprise beads that comprise a different barcode (e.g., a partition/cell-specific barcode) that is different from the analyte-specific barcodes conjugated to the reporter agents. Cells may be lysed inside the partitions to release intracellular contents and two different types of barcodes (e.g., analyte-specific barcodes and partition-specific/cell-specific barcodes). DNA amplifications and sequencing of the barcodes may be performed for the measurement of the secreted molecules (e.g., cytokines and other intracellular analytes). As a result, the secreted molecules (e.g., cytokines and other intracellular analytes) may be identified and quantified.

**Example 2: Barcoding Analytes from a Single Cell Using Cell Beads**

This example illustrates the production and identification of barcoded, secreted analytes from cells *in vitro,* using cell beads.

Cells (e.g., immune cells) are isolated from a subject, and each cell is further encapsulated into a droplet, which subsequently gels into a hydrogel matrix. The polymeric precursors are subjected to conditions sufficient to polymerize the precursors, such that cell beads are generated, each comprising a single cell. The backbone of the hydrogel matrix attaches to the plurality of capture agents (e.g., first polypeptides).

Cell beads are then placed in a solution comprising specific antigens or other suitable stimuli under a sufficient condition to allow secretion of molecules (e.g., cytokines or other intracellular analytes). Further, the cell beads are incubated in another solution comprising a plurality of barcoded reporter agents with reporter molecules (e.g., barcoded second polypeptides), which have specific affinity to the secreted molecules (e.g., cytokines or other secreted intracellular analytes). In some examples, a co-stimulatory agent such as CD3 is added to cells to induce secretion of an analyte. Reporter agents specific to the secreted molecule of interest may contain a fluorophore, chromophore, heavy metal, DNA oligonucleotide or combinations thereof. Furthermore, the coupling chemistry and type of covalent bond between the reporter agent and these labels may be altered. Optionally, DNA oligonucleotide barcoded antibodies specific to cell surface proteins are also added at this stage.

In some examples, cell beads may be partitioned (e.g., into emulsion droplets) and partitions may comprise beads that comprise a different barcode (e.g., partition/cell-specific barcode) that is different from the analyte-specific barcodes conjugated to the reporter agents. Cells may be lysed inside the partitions to release intracellular contents and two different types of barcodes (e.g., analyte-specific barcodes and partition-specific/cell-specific barcodes). DNA amplifications and sequencing of the barcodes may be performed for the measurement of the secreted molecules (e.g., cytokines and other intracellular analytes). As a result, the secreted molecules (e.g., cytokines and other intracellular analytes) may be identified and quantified.

**Example 3: Staining Analytes from a Single Cell Using Cell beads**

This example describes the staining of barcoded-secreted molecules.

Cell beads each comprising a single cell are formed. After incubation with specific antigens or other suitable stimuli under a sufficient condition, the cell beads are treated with suitable enzymes under suitable conditions so that the extra-cellular matrix (ECM) comprising of each bead is partially digested. Further, the partially digested cell beads are incubated with a plurality of barcoded reporter agent with reporter molecule (e.g., barcoded second polypeptide), which have specific affinity to the secreted molecules (e.g., cytokines or other secreted intracellular analytes). The partially digested ECM allows the barcoded polypeptide to bind to secreted molecule (e.g., cytokine or other secreted intracellular analytes). In addition, washing away the excess reporter agents for imaging of the secreted molecules (e.g., cytokines or intracellular analytes).

**Example 4: Identifying and Quantifying Secreted Analytes from a Single Cell**

This example describes the analytical processing of barcoded-secreted molecules.

Once the DNA barcoding procedure outlined in Examples 1 and 2 is complete, the emulsion droplets are coalesced into a bulk solution. The amplified cDNAs and reporter agent-derived DNA oligonucleotides are separated by size, and are then independently converted into Illumina sequencing libraries. The partition-specific DNA barcode computationally infers that all mRNA and reporter agent-derived DNA oligonucleotides possessing the same sequence were derived from the same single cell (e.g., T cell). DNA amplifications and sequencing of the analyte-specific polypeptide DNA barcodes (e.g., reporter barcode sequence) identifies and quantifies the secreted molecule. In some examples, there may be simultaneous measurement of secreted molecules, mRNAs, cell surface proteins, paired $\alpha\beta$ T-cell receptor sequences, and antigen binding specificity.

Antigens are optionally identified through the known MHC-multimer DNA barcode, while the identity and quantity of cell surface proteins are determined through the known DNA barcode attached to analyte-specific polypeptides (e.g., reporter barcode sequence), as previously described.

**Example 5: Assessing Cytokine Release Potential of a Monoclonal Antibody**

This example demonstrates the quantification of cytokine release induced by a given monoclonal antibody, and identification of the cellular populations responsible for this cytokine release, using single cell technologies.

One or more wells of a plate are coated with the monoclonal antibody of interest (e.g., a candidate monoclonal antibody). As a positive control, monoclonal antibodies targeting CD3 and/or CD28, or phorbol 12-myristate and ionomycin, may be added to one or more wells of the same plate. Additional wells within the same plate may be coated with an irrelevant monoclonal antibody, human myoglobin, and/or human hemoglobin to serve as a negative control. An immune sample of interest (e.g., PBMCs, tumor biopsy, or another immune sample) is then added to each well.

A first reporter barcoded capture agent is then added to the plate. The capture agents are comprised of a bispecific F(ab')2 or other bispecific antibody-based molecule, with one specificity for the canonical lymphocyte marker PTPRC/CD45, and with a second specificity for the cytokine of interest. The capture agent is conjugated to a first reporter barcode oligonucleotide, attached in a site-specific format (e.g., via glycosylation of the Ig Fc region, sortase conjugation, or non-canonical amino acid attachment), or in a non-site specific format (e.g., via click chemistry or ReACT chemistry). In some examples, the capture agent could also be lipid-conjugated, enabling insertion of the capture agent into the cell membrane of the immune cells, prior to incubation with the targets.

The cells and targets are incubated for 6, 12, and 18 hours in parallel, in replicates. During this time, the capture agent binds and retains secreted cytokines from the cells. At each timepoint, a second reporter barcoded monoclonal antibody against the cytokine of interest (e.g., reporter agent) is added. Optionally, the reporter agent may also be a scFv, nanobody, or other molecule with specificity to the cytokine, so long as a distinct reporter barcoding oligonucleotide may be attached.

In an alternative example, a monoclonal antibody candidate of interest may be engineered to contain either a) a kinetically activate bacterial sortase A domain, fragment, or complete enzyme or b) a sortase A motif (LPXTG) on the N terminus. To evaluate Fc bindinginduced cytokine release, a patient-derived, autologous, or human lymphoblastoid cell line is engineered, wherein the Fc neonatal receptor contains either a) a kinetically activate bacterial sortase A domain, fragment, or complete enzyme or b) a sortase A motif (LPXTG) on the N terminus. If the candidate monoclonal antibody contains the sortase A domain, fragment, or complete enzyme, then the Fc neonatal engineered receptor must contain the LPXTG motif, or the reverse.

The engineered cellular populations are then co-incubated as previously described. A polyglycine peptide (GGGGG) with a reporter barcode oligonucleotide conjugated to the peptide may be added to the wells. Optionally, the peptide may be conjugated to a fluorophore or other large biomolecule, with a reporter barcode oligonucleotide similarly attached. Through this process, a recognizable domain is transferred from the antibody to cells recognizing or binding the antibody, or the reverse, depending on whether the antibody is fused to sortase A or to the LPXTG motif. This recognizable domain may be detected on the surface of a cell, or intracellularly after permeabilization, by addition of a reporter barcoded antibody or detecting molecule with specificity to the polyglycine or the polyglycine-conjugated signal molecule (e.g., a fluorophore, receptor domain, or other biomolecule). A reporter barcoded capture agent may also be added at this point, as previously described.

The detection of released cytokine is measured by co-detection of the reporter barcode oligonucleotides within cell-associated partitions (e.g., gel beads in emulsions), allowing the detected cytokine to be traced back to a single cell. In some examples, detection of the first reporter barcode oligonucleotide on the capture agent and the second reporter barcode oligonucleotide on the reporter agent, by molecular counts for a given cell, indicates that a cell has captured a secreted cytokine. In other examples, detection of the reporter barcode oligonucleotide attached to the polyglycine-recognizing or polyglycine conjugate-recognizing molecule for a given cell both indicates that the cell of interest physically contacted the monoclonal antibody, and secreted a given cytokine of interest.

In combination with various other multiomic measurements, including but not limited to spatial location (e.g., where an antibody was deposited on a spatial slide, microwell, or other indexed location), chromatin conformation, gene expression, surface or intracellular protein, those knowledgeable in the art can identify which immune cell populations exist within a sample, and which populations secreted cytokines in response to incubation with the monoclonal antibody of interest. Optionally, this is further assessed by comparison to the positive and negative control samples, to quantify the number of cells in the same populations that did or did not secrete antibodies after exposure to positive controls (e.g., stimulatory reagents) or negative controls (e.g., abundant circulating proteins in human serum).

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

**ASPECTS OF THE INVENTION:**

1. A method of single cell analysis, comprising:

   a) contacting a cell with a reporter agent comprising a reporter nucleic acid molecule to provide a labelled cell, wherein the reporter nucleic acid molecule comprises a reporter sequence corresponding to the reporter agent, wherein the labelled cell comprises a complex coupled to a surface of the cell, and wherein the complex comprises (i) a capture agent, (ii) a secreted analyte, and (iii) the reporter agent;
   b) partitioning the labelled cell in a partition, wherein the partition comprises a plurality of barcode nucleic acid molecules which comprise a plurality of partition barcode sequences; and
   c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

2. The method of aspect 1, wherein said secreted analyte is a secreted protein.
3. The method of aspect 1 or 2, wherein the capture agent is configured to couple to a cell surface molecule.
4. The method of aspect 3, wherein said cell surface molecule is a cell surface protein.
5. The method of any of aspects 1-4, wherein the capture agent is configured to couple to the secreted analyte.
6. The method of aspect 4, wherein said capture agent is configured to couple to both the cell surface protein and said secreted analyte.
7. The method of any of aspects 1-6, wherein said capture agent is a first protein binding agent.
8. The method of any of aspects 1-7, wherein the reporter agent is configured to couple to the secreted analyte.
9. The method of aspect 7 or 8, wherein said reporter agent is a second protein binding agent.
10. The method of any of aspects 1-9, wherein said reporter nucleic acid molecule further comprises a sequence configured to couple to the barcode nucleic acid molecule.

11. The method of any of aspects 1-10, wherein said labelled cell further comprises a second reporter agent comprising a second reporter nucleic acid molecule.

12. The method of aspect 11, wherein said second reporter agent is configured to couple to a second cell surface protein of the cell.

13. The method of aspect 11 or 12, wherein said second reporter nucleic acid molecule comprises a second reporter sequence corresponding to the second reporter agent.

14. The method of aspect any of aspects 1-13, wherein the partition further comprises a second barcode nucleic acid molecule of a plurality of second barcode nucleic acid molecules.

15. The method of aspect 14, wherein said second reporter nucleic acid molecule comprises a second sequence configured to couple to the second barcode nucleic acid molecule.

16. The method of aspect 14 or 15, wherein step (c) further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

17. The method of any of aspects 1-16, wherein said labelled cell further comprises a plurality of nucleic acid analytes, wherein a nucleic acid analyte of said plurality of nucleic acid analytes comprises a nucleic acid analyte sequence.

18. The method of any of aspects 1-17, wherein the partition further comprises a plurality of third barcode nucleic acid molecules.

19. The method of aspect 18, wherein a third barcode nucleic acid molecule of said plurality of third barcode nucleic acid molecules comprises a third sequence configured to couple to the nucleic acid analyte sequence.

20. The method of aspect 18 or 19, wherein step (c) further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

21. The method of any of aspects 1-20, wherein said partition comprises a support that comprises the plurality of barcode nucleic acid molecules.

22. The method of aspect 21, wherein said support is a bead.

23. The method of aspect 22, wherein said bead is a gel bead.

24. The method of any of aspects 21-23, wherein said plurality of barcode nucleic acid molecules are releasably attached to the support.

25. The method of any of aspects 1-24, wherein the partition is from a plurality of partitions.

26. The method of aspect 25, wherein the partition is a droplet or a microwell.

27. A method of single cell analysis, comprising:

a) contacting a cell bead with a reporter agent comprising a reporter nucleic acid molecule to provide a labelled cell bead, wherein the cell bead comprises a cell in a matrix, wherein the reporter nucleic acid molecule comprises a reporter sequence corresponding to the reporter agent, wherein the labelled cell bead comprises a complex in or on the cell bead, and wherein the complex comprises (i) a capture agent, (ii) a secreted analyte, and (iii) the reporter agent;
b) partitioning the labelled cell bead in a partition, wherein the partition comprises a plurality of barcode nucleic acid molecules which comprise a plurality of partition barcode sequences; and
c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

28. The method of aspect 27, wherein said secreted analyte is a secreted protein.

29. The method of aspect 27 or 28, wherein the capture agent is configured to couple to the matrix of the cell bead.

30. The method of aspect 29, wherein said matrix is a degradable polymer matrix.

31. The method of any of aspects 27-30, wherein the capture agent is configured to couple to the secreted analyte.

32. The method of aspect 30, wherein said capture agent is configured to couple to both the matrix and said secreted analyte.

33. The method of any of aspects 27-32, wherein said capture agent is a first protein binding agent.

34. The method of any of aspects 27-33, wherein the reporter agent is configured to couple to the secreted analyte.

35. The method of aspect 33 or 34, wherein said reporter agent is a second protein binding agent.

36. The method of any of aspects 27-35, wherein said reporter nucleic acid molecule further comprises a sequence configured to couple to the barcode nucleic acid molecule.

37. The method of any of aspects 27-36, wherein said cell or said labelled cell bead further comprises a second reporter agent comprising a second reporter nucleic acid molecule.

38. The method of aspect 37, wherein said second reporter agent is configured to couple to a cell surface protein of the cell.

39. The method of aspect 37 or 38, wherein said second reporter nucleic acid molecule comprises a second reporter sequence corresponding to the second reporter agent.

40. The method of aspect any of aspects 27-39, wherein the partition further comprises a second barcode nucleic acid molecule of a plurality of second barcode nucleic acid molecules.

41. The method of aspect 40, wherein said second reporter nucleic acid molecule comprises a second sequence configured to couple to the second barcode nucleic acid molecule.

42. The method of aspect 40 or 41, wherein step (c) further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

43. The method of any of aspects 27-42, wherein said cell further comprises a plurality of nucleic acid analytes, wherein a nucleic acid analyte of said plurality of nucleic acid analytes comprises a nucleic acid analyte sequence.

44. The method of any of aspects 27-43, wherein the partition further comprises a plurality of third barcode nucleic acid molecules.

45. The method of aspect 44, wherein a third barcode nucleic acid molecule of said plurality of third barcode nucleic acid molecules comprises a third sequence configured to couple to the nucleic acid analyte sequence.

46. The method of aspect 44 or 45, wherein step (c) further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

47. The method of any of aspects 27-46, wherein said partition comprises a support that comprises the plurality of barcode nucleic acid molecules.

48. The method of aspect 47, wherein said support is a bead.

49. The method of aspect 48, wherein said bead is a gel bead.

50. The method of any of aspects 47-49, wherein said plurality of barcode nucleic acid molecules are releasably attached to the support.

51. The method of any of aspects 27-50, wherein the partition is from a plurality of partitions.

52. The method of aspect 51, wherein the partition is a droplet or a microwell.

**Claims**

1. A method of single cell analysis, comprising:

   a) contacting a cell with a reporter agent comprising a reporter nucleic acid molecule to provide a labelled cell, wherein the reporter nucleic acid molecule comprises a reporter sequence corresponding to the reporter agent, wherein the labelled cell comprises a complex coupled to a surface of the cell, and wherein the complex comprises (i) a capture agent, (ii) a secreted analyte, and (iii) the reporter agent;
   b) partitioning the labelled cell in a partition, wherein the partition comprises a plurality of barcode nucleic acid molecules which comprise a plurality of partition barcode sequences; and
   c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

2. The method of claim 1, wherein:

   (a) said secreted analyte is a secreted protein; and/or
   (b) the capture agent is configured to couple to:

      (i) a cell surface molecule;
      (ii) the secreted analyte; or
      (ii) both a cell surface molecule and the secreted analyte,

   optionally wherein the cell surface molecule is a cell surface protein.

3. The method of claim 1 or 2, wherein:

   (a) said capture agent is a first protein binding agent; and/or
   (b) the reporter agent is configured to couple to the secreted analyte, optionally wherein the reporter agent is a second protein binding agent; and/or

(c) said reporter nucleic acid molecule further comprises a sequence configured to couple to the barcode nucleic acid molecule.

4. The method of any of claims 1-3, wherein said labelled cell further comprises a second reporter agent comprising a second reporter nucleic acid molecule, optionally wherein:

(a) said second reporter agent is configured to couple to a second cell surface protein of the cell; and/or
(b) said second reporter nucleic acid molecule comprises a second reporter sequence corresponding to the second reporter agent.

5. The method of claim any of claims 1-4, wherein the partition further comprises a second barcode nucleic acid molecule of a plurality of second barcode nucleic acid molecules, optionally wherein:

(a) said second reporter nucleic acid molecule comprises a second sequence configured to couple to the second barcode nucleic acid molecule; and/or
(b) step (c) further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

6. The method of any of claims 1-5, wherein said labelled cell further comprises a plurality of nucleic acid analytes, wherein a nucleic acid analyte of said plurality of nucleic acid analytes comprises a nucleic acid analyte sequence.

7. The method of claim 6, wherein the partition further comprises a plurality of third barcode nucleic acid molecules, optionally wherein:

(a) a third barcode nucleic acid molecule of said plurality of third barcode nucleic acid molecules comprises a third sequence configured to couple to the nucleic acid analyte sequence; and/or
(b) step (c) further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

8. The method of any of claims 1-7, wherein said partition comprises a support that comprises the plurality of barcode nucleic acid molecules, optionally wherein

(a) said support is a bead, optionally wherein said bead is a gel bead; and/or
(b) said plurality of barcode nucleic acid molecules are releasably attached to the support.

9. A method of single cell analysis, comprising:

a) contacting a cell bead with a reporter agent comprising a reporter nucleic acid molecule to provide a labelled cell bead, wherein the cell bead comprises a cell in a matrix, wherein the reporter nucleic acid molecule comprises a reporter sequence corresponding to the reporter agent, wherein the labelled cell bead comprises a complex in or on the cell bead, and wherein the complex comprises (i) a capture agent, (ii) a secreted analyte, and (iii) the reporter agent;
b) partitioning the labelled cell bead in a partition, wherein the partition comprises a plurality of barcode nucleic acid molecules which comprise a plurality of partition barcode sequences; and
c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

10. The method of claim 9, wherein:

(a) said secreted analyte is a secreted protein; and/or
(b) the capture agent is configured to couple to:

(i) the matrix of the cell bead;
(ii) the secreted analyte; or
(iii) the matrix of the cell bead and the secreted analyte,

optionally wherein the matrix is a degradable polymer matrix.

11. The method of claim 9 or 10, wherein:

(a) said capture agent is a first protein binding agent; and/or
(b) the reporter agent is configured to couple to the secreted analyte, optionally wherein said reporter agent is a second protein binding agent;

and/or

(c) said reporter nucleic acid molecule further comprises a sequence configured to couple to the barcode nucleic acid molecule; and/or

(d) said cell or said labelled cell bead further comprises a second reporter agent comprising a second reporter nucleic acid molecule, optionally wherein:

(i) said second reporter agent is configured to couple to a cell surface protein of the cell; and/or

(ii) said second reporter nucleic acid molecule comprises a second reporter sequence corresponding to the second reporter agent.

12. The method of claim any of claims 9-11, wherein the partition further comprises a second barcode nucleic acid molecule of a plurality of second barcode nucleic acid molecules, optionally wherein:

(a) said second reporter nucleic acid molecule comprises a second sequence configured to couple to the second barcode nucleic acid molecule; and/or

(b) step (c) further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof.

13. The method of any of claims 9-12, wherein said cell further comprises a plurality of nucleic acid analytes, wherein a nucleic acid analyte of said plurality of nucleic acid analytes comprises a nucleic acid analyte sequence.

14. The method of claim 13, wherein the partition further comprises a plurality of third barcode nucleic acid molecules, optionally wherein:

(a) a third barcode nucleic acid molecule of said plurality of third barcode nucleic acid molecules comprises a third sequence configured to couple to the nucleic acid analyte sequence; and/or

(b) step (c) further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof.

15. The method of any of claims 9-14, wherein said partition comprises a support that comprises the plurality of barcode nucleic acid molecules, optionally wherein

(a) said support is a bead, optionally wherein said bead is a gel bead; and/or

(b) said plurality of barcode nucleic acid molecules are releasably attached to the support.

EP 4 194 855 A1

**100**

**104**

**112**        **116**        **120**

**102**

**114**

**110**        **118**

**106**

*FIG. 1*

**FIG. 2**

**FIG. 3**

EP 4 194 855 A1

**FIG. 4**

**FIG. 5**

600

608

604

610

602

612

606

616

*FIG. 6*

*FIG. 7A*

*FIG. 7B*

FIG. 8

FIG. 9

FIG. 10

*FIG. 11*

FIG. 12A

1202

1206

FIG. 12B

**FIG. 13**

1402 — T cells or other types of cells are stimulated with specific antigens or other stimuli to allow secretion of cytokines or other analytes

1404 — T cells or other types of cells are incubated with a first binding agent comprising a polypeptide 1204 to bind to cytokines or other secreted analytes

1406 — A barcoded and cytokine specific second binding agent comprising a polypeptide 1302 (comprising a first barcode 1304) is attached to secreted cytokines or other analytes are coupled to (e.g., "captured" by) polypeptide 1204 (of the first binding agent)

1408 — Cells are encapsulated into droplets which subsequently gel into a hydrogel matrix encapsulating the cell, forming a cell bead

1410 — Cell beads are incubated with specific antigens or other stimuli to allow secretion of cytokines or other analytes; secreted cytokines or other analytes are coupled to (e.g., "captured" by) a first binding agent comprising a polypeptide 1508 linked to the backbone of the polymer forming the hydrogel matrix

1411a — A barcoded and cytokine specific second binding agent comprising a polypeptide 1512 (comprising a first barcode 1513) is attached to secreted cytokines or other analytes are coupled to (e.g., "captured" by) polypeptide 1508 (of the first binding agent) linked to the polymer backbone (e.g., of cell bead 1504)

1412 — Optionally, cells or cell beads are partitioned into droplets (e.g., emulsion droplets); cell beads are dissolved prior to cell lysis ; partitions may comprise beads comprising a second barcode sequence 1702

1414 — Cells are lysed inside the partitions (e.g., droplets or wells) to release intracellular constituent and barcodes 1304 attached to polypeptides 1302 and/or 1512, and optionally barcodes attached to 1204

1416 — Reverse transcription of the released mRNAs; attaching second (e.g., partition-specific) barcodes 1702 (may be attached to gel beads) to cDNA generated from mRNA and to the released first (analyte-specific) barcodes 1304; amplifying nucleic acid molecules

1418 — Pooling partitions (e.g., droplets or wells) into a bulk solution and further processing to convert into sequencing library

*FIG. 14A*

EP 4 194 855 A1

Cells are encapsulated into droplets which subsequently gel into a hydrogel matrix encapsulating the cell, forming a cell bead — 1408

Cell beads are incubated with specific antigens or other stimuli to allow secretion of cytokines or other analytes; secreted cytokines or other analytes are coupled to (e.g., "captured" by) a first binding agent comprising a polypeptide 1508 linked to the backbone of the polymer forming the hydrogel matrix — 1410

After capturing of the secreted cytokines or other analytes, the cell beads are partially digested by enzymes so that a plurality of antibodies labeled with barcodes can have access to the captured secreted cytokines or other analytes & stain partially digested cell beads with said plurality of antibodies — 1411b

Optionally, cells or cell beads are partitioned into droplets (e.g., emulsion droplets); cell beads are dissolved prior to cell lysis ; partitions may comprise beads comprising a second barcode sequence 1702 — 1412

Cells are lysed inside the partitions (e.g., droplets or wells) to release intracellular constituent and barcodes 1304 attached to polypeptides 1302 and/or 1512, and optionally barcodes attached to 1204 — 1414

Reverse transcription of the released mRNAs; attaching second (e.g., partition-specific) barcodes 1702 (may be attached to gel beads) to cDNA generated from mRNA and to the released first (analyte-specific) barcodes 1304; amplifying nucleic acid molecules — 1416

Pooling partitions (e.g., droplets or wells) into a bulk solution and further processing to convert into sequencing library — 1418

*FIG. 14B*

FIG. 14C

*FIG. 15A*

*FIG. 15B*

EP 4 194 855 A1

FIG. 15C

1513

1512

1503

1509

1510

1511

1504

1508

**FIG. 15D**

**FIG. 16**

**FIG. 17A**

FIG. 17B

Cell Surface
proteins

1802

Antigen
Specificity

1803

Secreted
Proteins

1804

mRNA

1805

Paired αβ TCR
Sequence

EP 4 194 855 A1

*FIG. 18*

*FIG. 19*

FIG. 20

EP 4 194 855 A1

**FIG. 21A**

**FIG. 21B**

FIG. 21C

**FIG. 22**

Europäisches
Patentamt

European
Patent Office

Office européen
des brevets

# EUROPEAN SEARCH REPORT

Application Number

EP 22 20 9155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/075693 A1 (10X GENOMICS INC [US]) 26 April 2018 (2018-04-26) * paragraph [0192] - paragraph [0195]; figures 5, 6 * * paragraph [0281] - paragraph [0283] * | 1-15 | INV. G01N33/543 C12Q1/6806 |
| A | PAYAM SHAHI ET AL: "Abseq: Ultrahigh-throughput single cell protein profiling with droplet microfluidic barcoding", SCIENTIFIC REPORTS, vol. 7, 14 March 2017 (2017-03-14), pages 1-12, XP055586462, DOI: 10.1038/srep44447 * abstract; figure 1 * | 1-15 | |
| A | WO 2019/118355 A1 (10X GENOMICS INC [US]) 20 June 2019 (2019-06-20) * paragraph [0084] - paragraph [0117]; claims 1-51; figures 9, 10 * | 1-15 | |

TECHNICAL FIELDS SEARCHED (IPC)

G01N
C12Q

The present search report has been drawn up for all claims

| Place of search | Date of completion of the search | Examiner |
|---|---|---|
| Munich | 24 March 2023 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 9155

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2023

| Patent document cited in search report | | Publication date | Patent family member(s) | | Publication date |
|---|---|---|---|---|---|
| WO 2018075693 | A1 | 26-04-2018 | CN | 109983126 A | 05-07-2019 |
| | | | SG | 10202012440V A | 28-01-2021 |
| | | | SG | 11201903519U A | 30-05-2019 |
| | | | WO | 2018075693 A1 | 26-04-2018 |
| WO 2019118355 | A1 | 20-06-2019 | CN | 111699388 A | 22-09-2020 |
| | | | EP | 3724658 A1 | 21-10-2020 |
| | | | US | 2020378961 A1 | 03-12-2020 |
| | | | WO | 2019118355 A1 | 20-06-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62914296
- WO 2019157529 A
- US 10011872 B
- US 20180105808 A
- US 20180216162 A
- US 10428326 B
- US 20190100632 A
- US 10590244 B
- US 2020017785 W
- US 2020020486 W
- US 20190367997 A
- US 20190064173 A
- US 2015025197 W
- US 20140155295
- US 20100105112
- US 20140378345
- US 10550429 B
- US 20190177800 A
- US 20190367969 A
- US 20190323088 A
- US 6265552 B
- WO 2018075693 A
- US 20150376609 A
- US 20150292988 A
- US 20140378345 A
- US 20190233878 A

**Non-patent literature cited in the description**

- **HUGHES L D et al.** *PLoS One,* 04 February 2014, vol. 9 (2), e87649
- **FANG et al.** Fluoride-Cleavable Biotinylation Phosphoramidite for 5'-end-Labelling and Affinity Purification of Synthetic Oligonucleotides. *Nucleic Acids Res.,* 15 January 2003, vol. 31 (2), 708-715
- *Angew. Chem. Int. Ed.,* 2001, vol. 40 (11), 2004-2021
- *Pharm Res,* 2008, vol. 25 (10), 2216-2230
- *Chem. Commun.,* 2011, vol. 47, 6257-6259
- *Nature,* 2015, vol. 519 (7544), 486-90
- **MADL ; HEILSHORN.** Bioorthogonal Strategies for Engineering Extracellular Matrices. *Adv. Funct. Mater.,* 2018, vol. 28, 1706046